(19) Europäisches Patentamt
European Patent Office
Office européen des brevets

(11) EP 3 475 445 B1

(12) **EUROPEAN PATENT SPECIFICATION**

(45) Date of publication and mention of the grant of the patent:
**09.02.2022 Bulletin 2022/06**

(21) Application number: **17731824.3**

(22) Date of filing: **02.06.2017**

(51) International Patent Classification (IPC):
***A61K 38/19*** *(2006.01)* ***A61P 25/16*** *(2006.01)*

(52) Cooperative Patent Classification (CPC):
(C-Sets available)
**C12Q 1/6897; A61K 31/19; A61K 31/192; A61K 31/52; A61K 31/713; A61P 25/16** (Cont.)

(86) International application number:
**PCT/EP2017/063554**

(87) International publication number:
**WO 2017/220315 (28.12.2017 Gazette 2017/52)**

(54) **MEANS AND METHODS FOR TREATING PARKINSON'S DISEASE**

VORRICHTUNG UND VERFAHREN ZUR BEHANDLUNG VON MORBUS PARKINSON

MOYENS ET MÉTHODES POUR TRAITER LA MALADIE DE PARKINSON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.06.2016 LU 93116**

(43) Date of publication of application:
**01.05.2019 Bulletin 2019/18**

(73) Proprietor: **Université du Luxembourg**
**4365 Esch-sur-Alzette (LU)**

(72) Inventors:
- **KRUEGER, Rejko**
  **4367 Belvaux (LU)**
- **BOUSSAAD, Ibrahim**
  **4367 Belvaux (LU)**
- **OBERMAIER, Carolin**
  **4367 Belvaux (LU)**

(74) Representative: **Schiweck Weinzierl Koch Patentanwälte Partnerschaft mbB**
**Ganghoferstraße 68 B**
**80339 München (DE)**

(56) References cited:
- **ONO K ET AL: "A chemical chaperone, sodium 4-phenylbutyric acid, attenuates the pathogenic potency in human alpha-synuclein A30P+A53T transgenic mice", PARKINSONISM AND RELATED DISORDERS, ELSEVIER SCIENCE, OXFORD, GB, vol. 15, no. 9, 5 November 2009 (2009-11-05), pages 649-654, XP026709202, ISSN: 1353-8020, DOI: 10.1016/J.PARKRELDIS.2009.03.002 [retrieved on 2009-04-03]**
- **NICHOLAS T. HERTZ ET AL: "A Neo-Substrate that Amplifies Catalytic Activity of Parkinson's-Disease-Related Kinase PINK1", CELL, vol. 154, no. 4, 1 August 2013 (2013-08-01), pages 737-747, XP055298490, US ISSN: 0092-8674, DOI: 10.1016/j.cell.2013.07.030**
- **ROBERT HERING ET AL: "Novel homozygous p.E64D mutation in DJ1 in early onset Parkinson disease (PARK7)", HUMAN MUTATION, vol. 24, no. 4, 30 October 2004 (2004-10-30), pages 321-329, XP055298523, US ISSN: 1059-7794, DOI: 10.1002/humu.20089**
- **LA COGNATA VALENTINA ET AL: "Splicing: is there an alternative contribution to Parkinson's disease?", NEUROGENETICS, OXFORD UNIVERSITY PRESS, OXFORD, GB, vol. 16, no. 4, 16 May 2015 (2015-05-16), pages 245-263, XP035537835, ISSN: 1364-6745, DOI: 10.1007/S10048-015-0449-X [retrieved on 2015-05-16]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MAYUMI YOSHIDA ET AL: "Rectifier of aberrant mRNA splicing recovers tRNA modification in familial dysautonomia", PROCEEDINGS OF THE NATIONAL ACADEMY OF SCIENCES, vol. 112, no. 9, 9 February 2015 (2015-02-09), pages 2764-2769, XP055298716, US ISSN: 0027-8424, DOI: 10.1073/pnas.1415525112**


(52) Cooperative Patent Classification (CPC): (Cont.)

C-Sets
**C12Q 1/6897, C12Q 2539/105**

SEQUENCE LISTING

<110> Universite du Luxembourg

<120> MEANS AND METHODS FOR TREATING PARKINSON’S DISEASE

<130> LUX15729PCT

<150> LU 93116
<151> 2016-06-22

<160> 13

<170> PatentIn version 3.5

<210> 1
<211> 570
<212> DNA
<213> Artificial Sequence

<220>
<223> cDNA of human DJ-1 (wt)

<400> 1

```
atggcttcca aaagagctct ggtcatcctg gctaaaggag cagaggaaat ggagacggtc        60
atccctgtag atgtcatgag gcgagctggg attaaggtca ccgttgcagg cctggctgga       120
aaagacccag tacagtgtag ccgtgatgtg gtcatttgtc ctgatgccag ccttgaagat       180
gcaaaaaaag agggaccata tgatgtggtg gttctaccag gaggtaatct gggcgcacag       240
aatttatctg agtctgctgc tgtgaaggag atactgaagg agcaggaaaa ccggaagggc       300
ctgatagccg ccatctgtgc aggtcctact gctctgttgg ctcatgaaat aggttttgga       360
agtaaagtta caacacaccc tcttgctaaa gacaaaatga tgaatggagg tcattacacc       420
tactctgaga atcgtgtgga aaaagacggc ctgattctta caagccgggg gcctgggacc       480
agcttcgagt ttgcgcttgc aattgttgaa gccctgaatg gcaaggaggt ggcggctcaa       540
gtgaaggctc cacttgttct taaagactag                                        570
```

<210> 2
<211> 570
<212> DNA
<213> Artificial Sequence

<220>
<223> cDNA of human DJ-1 c.192G>C

<400> 2

```
atggcttcca aaagagctct ggtcatcctg gctaaaggag cagaggaaat ggagacggtc        60
atccctgtag atgtcatgag gcgagctggg attaaggtca ccgttgcagg cctggctgga       120
aaagacccag tacagtgtag ccgtgatgtg gtcatttgtc ctgatgccag ccttgaagat       180
gcaaaaaaag acggaccata tgatgtggtg gttctaccag gaggtaatct gggcgcacag       240
aatttatctg agtctgctgc tgtgaaggag atactgaagg agcaggaaaa ccggaagggc       300
ctgatagccg ccatctgtgc aggtcctact gctctgttgg ctcatgaaat aggttttgga       360
agtaaagtta caacacaccc tcttgctaaa gacaaaatga tgaatggagg tcattacacc       420
tactctgaga atcgtgtgga aaaagacggc ctgattctta caagccgggg gcctgggacc       480
agcttcgagt ttgcgcttgc aattgttgaa gccctgaatg gcaaggaggt ggcggctcaa       540
gtgaaggctc cacttgttct taaagactag                                        570
```

<210> 3
<211> 102
<212> DNA
<213> human

<400> 3

```
attaaggtca ccgttgcagg cctggctgga aaagacccag tacagtgtag ccgtgatgtg      60

gtcatttgtc ctgatgccag ccttgaagat gcaaaaaaag ag                        102
```

<210> 4
<211> 102
<212> DNA
<213> human

<400> 4

```
attaaggtca ccgttgcagg cctggctgga aaagacccag tacagtgtag ccgtgatgtg      60

gtcatttgtc ctgatgccag ccttgaagat gcaaaaaaag ac                        102
```

<210> 5
<211> 189
<212> PRT
<213> human

<400> 5

```
Met Ala Ser Lys Arg Ala Leu Val Ile Leu Ala Lys Gly Ala Glu Glu
1               5                   10                  15

Met Glu Thr Val Ile Pro Val Asp Val Met Arg Arg Ala Gly Ile Lys
            20                  25                  30

Val Thr Val Ala Gly Leu Ala Gly Lys Asp Pro Val Gln Cys Ser Arg
        35                  40                  45

Asp Val Val Ile Cys Pro Asp Ala Ser Leu Glu Asp Ala Lys Lys Glu
    50                  55                  60

Gly Pro Tyr Asp Val Val Val Leu Pro Gly Gly Asn Leu Gly Ala Gln
65                  70                  75                  80

Asn Leu Ser Glu Ser Ala Ala Val Lys Glu Ile Leu Lys Glu Gln Glu
                85                  90                  95
```

Asn Arg Lys Gly Leu Ile Ala Ala Ile Cys Ala Gly Pro Thr Ala Leu
100 105 110

Leu Ala His Glu Ile Gly Phe Gly Ser Lys Val Thr Thr His Pro Leu
115 120 125

Ala Lys Asp Lys Met Met Asn Gly Gly His Tyr Thr Tyr Ser Glu Asn
130 135 140

Arg Val Glu Lys Asp Gly Leu Ile Leu Thr Ser Arg Gly Pro Gly Thr
145 150 155 160

Ser Phe Glu Phe Ala Leu Ala Ile Val Glu Ala Leu Asn Gly Lys Glu
165 170 175

Val Ala Ala Gln Val Lys Ala Pro Leu Val Leu Lys Asp
180 185

<210> 6
<211> 189
<212> PRT
<213> human

<400> 6

Met Ala Ser Lys Arg Ala Leu Val Ile Leu Ala Lys Gly Ala Glu Glu
1 5 10 15

Met Glu Thr Val Ile Pro Val Asp Val Met Arg Arg Ala Gly Ile Lys
20 25 30

Val Thr Val Ala Gly Leu Ala Gly Lys Asp Pro Val Gln Cys Ser Arg
35 40 45

Asp Val Val Ile Cys Pro Asp Ala Ser Leu Glu Asp Ala Lys Lys Asp
50 55 60

Gly Pro Tyr Asp Val Val Val Leu Pro Gly Gly Asn Leu Gly Ala Gln
65 70 75 80

Asn Leu Ser Glu Ser Ala Ala Val Lys Glu Ile Leu Lys Glu Gln Glu
85 90 95

Asn Arg Lys Gly Leu Ile Ala Ala Ile Cys Ala Gly Pro Thr Ala Leu
100 105 110

Leu Ala His Glu Ile Gly Phe Gly Ser Lys Val Thr Thr His Pro Leu
115 120 125

```
Ala Lys Asp Lys Met Met Asn Gly Gly His Tyr Thr Tyr Ser Glu Asn
    130                 135                 140

Arg Val Glu Lys Asp Gly Leu Ile Leu Thr Ser Arg Gly Pro Gly Thr
145                 150                 155                 160

Ser Phe Glu Phe Ala Leu Ala Ile Val Glu Ala Leu Asn Gly Lys Glu
                165                 170                 175

Val Ala Ala Gln Val Lys Ala Pro Leu Val Leu Lys Asp
            180                 185
```

<210> 7
<211> 191
<212> DNA
<213> Artificial sequence

<220>
<223> mutated U1 snRNA

<400> 7

```
atacttacgt ggcaggggag ataccatgat cacgaaggtg gttttcccag ggcgaggctt       60

atccattgca ctccggatgt gctgacccct gcgatttccc caaatgtggg aaactcgact      120

gcataatttg tggtagtggg ggactgcgtt cgcgctttcc cctgactttc tggagtttca      180

aaagtagact g                                                           191
```

<210> 8
<211> 11
<212> DNA
<213> human

<400> 8
auacuuaccu g 11

<210> 9
<211> 11
<212> DNA
<213> human

<400> 9
gagguuugua a 11

<210> 10
<211> 11
<212> DNA
<213> human

<400> 10
gacguuugua a 11

<210> 11

<211> 11
<212> DNA
<213> Artificial Sequence

<220>
<223> fragment of mutated U1 snRNA

<400> 11
auacuuacgu g 11

<210> 12
<211> 960
<212> DNA
<213> Artificial Sequence

<220>
<223> DJ-1 mRNA

<400> 12

```
ugagucugcg caguguggggg cugagggagg ccggacggcg cgcgugcgug cuggcgugcg 60
uucauuuuca gccuggugug gggugagugg uacccaacgg gccggggcgc cgcguccgca 120
ggaagaggcg cggggugcag gcuuguaaac auauaacaua aaaauggcuu ccaaaagagc 180
ucuggucauc cuggcuaaag gagcagagga aauggagacg gucaucccug uagaugucau 240
gaggcgagcu gggauuaagg ucaccguugc aggccuggcu ggaaaagacc caguacagug 300
uagccgugau gugguucauuu guccugaugc cagccuugaa gaugcaaaaa aagagggacc 360
auaugaugug gugguucuac caggagguaa ucugggcgca cagaauuuau cugagucugc 420
ugcugugaag gagauacuga aggagcagga aaaccggaag ggccugauag ccgccaucug 480
ugcagguccu acugcucugu uggcucauga aauagguuuu ggaaguaaag uuacaacaca 540
cccucuugcu aaagacaaaa ugaugaaugg aggucauuac accuacucug agaaucgugu 600
ggaaaaagac ggccugauuc uuacaagccg ggggccuggg accagcuucg aguuugcgcu 660
ugcaauuguu gaagcccuga auggcaagga gguggcggcu caagugaagg cuccacuugu 720
ucuuaaagac uagagcagcg aacugcgacg aucacuuaga gaaacaggcc guuaggaauc 780
cauucucacu guguucgcuc uaaacaaaac aguggguaggu uaauguguuc agaagucgcu 840
guccuuacua cuuuugcgga aguauggaag ucacaacuac acagagauuu cucagccuac 900
aaauuguguc uauacauuuc uaagccuugu uugcagaaua aacagggcau uuagcaaacu 960
```

<210> 13
<211> 858
<212> DNA
<213> Artificial Sequence

<220>
<223> DJ-1 mRNA without exon 3

<400> 13

```
ugagucugcg caguguggggg cugagggagg ccggacggcg cgcgugcgug cuggcgugcg      60
uucauuuuca gccuggugug gggugagugg uacccaacgg gccggggcgc cgcguccgca     120
ggaagaggcg cggggugcag gcuuguaaac auauaacaua aaaauggcuu ccaaaagagc     180
ucuggucauc cuggcuaaag gagcagagga aauggagacg gucaucccug uagaugucau     240
gaggcgagcu gggggaccau augauguggu gguucuacca ggagguaauc ugggcgcaca     300
gaauuuaucu gagucugcug cugugaagga gauacugaag gagcaggaaa accggaaggg     360
ccugauagcc gccaucugug cagguccuac ugcucuguug gcucaugaaa uagguuuugg     420
aaguaaaguu acaacacacc cucuugcuaa agacaaaaug augaauggag gucauuacac     480
cuacucugag aaucgugugg aaaaagacgg ccugauucuu acaagccggg ggccugggac     540
cagcuucgag uuugcgcuug caauuguuga agcccugaau ggcaaggagg uggcggcuca     600
agugaaggcu ccacuuguuc uuaaagacua gagcagcgaa cugcgacgau cacuuagaga     660
aacaggccgu uaggaaucca uucucacugu guucgcucua aacaaaacag ugguagguua     720
auguguucag aagucgcugu ccuuacuacu uuugcggaag uauggaaguc acaacuacac     780
agagauuucu cagccuacaa auugugucua uacauuucua agccuuguuu gcagaauaaa     840
cagggcauuu agcaaacu                                                   858
```

## Description

The invention relates to methods for screening a compound for Parkinson's disease treatment and to compounds for Parkinson's disease treatment.

Parkinson's disease (PD) is the second most common neurodegenerative brain disease. The characteristic motor symptoms including rigidity, imbalance and uncontrollable shaking are all familiar to the general public. To date, there is no cure for PD and motor symptoms can only be alleviated to increase the quality of life of patients. Overall, 2% of the world's population over 65 suffers from this debilitating disease and the incidence of PD is set to double in the next 20 years due to the demo-graphic development of the.

The protein DJ-1, which is encoded by PARK7 (or "DJ-1 gene") is involved in a variety of biological processes including transcriptional regulation, chaperone-like functions, oxidative stress response and mitochondrial protection. DJ-1 has a small 20kDa domain, which in solutions forms a dimer to become active. DJ-1 has multiple functions and is cytoprotective. The DJ-1 gene was first described as an oncogene in 1997 and shortly thereafter DJ-1 was found to regulate male fertility. In 2003, Bonifati and colleagues described four PD patients in a Dutch and three PD patients in an Italian family with mutations in DJ-1.

The gene encoding DJ-1 is composed of seven exons with exon 1 not coding. DJ-1 consists of 189 amino acids and belongs to the Thj1/Pfp1 superfamily. It is highly conserved among different species, homologues are found in all aerobic species from prokaryotes to eukaryotes and the amino acid residues are highly conserved, including the presumably most important residues for PD, namely C106, located in a nucleophile elbow, shown as ball and stick. The cysteine in position 106 of the peptide sequence is highly oxidisable and responds to oxidative stress/ROS by forming a sulfinic acid. DJ-1 with oxidised C106 is thought to be less stable and therefore becomes inactive. Interestingly, when C106 was replaced with other amino acids, DJ-1 lost its neuro protective activity. Only oxidised DJ-1 gets transported to the mitochondria where it acts mitoprotective.

The precise biochemical functions of DJ-1 in PD, however, are unclear. One reason might be that no post mortem brain samples from PD patients with DJ-1 mutations reached autopsy to study the pathology in these cases.

Another reason is the broad spectrum of publications describing different and partially quite vague functions of this small protein. DJ-1 is ubiquitously expressed. In the human brain, it is found in neurons and astrocytes. In sporadic PD patients, a strong expression is seen in reactive astrocytes, but it is not accumulating in the pathognomonic Lewy bodies (Bandopadhyay et al., 2004, Neumann et al., 2004, Rizzu et al., 2004).

Mutations in DJ-1 cause approximately 1 % of all PD cases world-wide. However, there is a variation seen in the frequency of DJ-1 mutations between different ethnic groups. Caucasians: 0.83 % in familial cases and 0.99 % in sporadic cases and 0.54 % copy number variations, Asians: 3.03 % in familial cases, Arabs: 0.74 % in sporadic cases and Ashkenazi Jews: 1.96 % in sporadic cases. The mutations result in an early-onset with slow progression (except for the compound heterozygous c.317_322del:p. 106_108del mutation) of the disease. For many of the mutations, the effect is still unknown including the previously described c.G192C mutation (corresponding to the p.E64D mutation) (Hering et al., 2004, Human Mutation 24:321-329).

The p.E64D mutation was first found in a male Turkish patient. He developed PD symptoms at the age of 34 (Hering et al., 2004). Hering et al have also studied the p.E64D mutation of DJ-1. For this purpose, HEK293 cells were transfected with cDNA encoding a DJ1 mutant comprising the p.E64D mutation. They observed that the recombinantly expressed DJ-1 E64D mutant displayed only a mild reduction in the protein level compared to the wild-type DJ-1. Hering et al further reported that the overall structure of the monomer and dimer of the p.E64D mutant are essentially the same as those of the wild-type DJ-1. Thus, they suggested that the p.E64D may affect binding of DJ-1 to another protein, thus causing deleterious effects by disrupting this interaction.

The results of Hering et al. 2004 were also confirmed by Malgieri and Eliezer, 2007, Protein Science, 17:855-868, who expressed p.E64D mutant of DJ-1 in *E. coli* using cDNA. In their study, Malgieri and Eliezer further reported that no significant change in thermodynamic stability occurs due to the p.E64D mutation. They also suggest that this mutation most likely alters interactions of DJ-1 with other cellular partners that are crucial for proper execution of its function. Ono et al., Parkinsonism and Related Disorders 15 (2009) 649-654 discloses that a chemical chaperone, sodium 4-phenylbutyric acid, attenuates the pathogenic potency in human $\alpha$-synuclein A30P + A53T transgenic mice. Hertz et al., 2013, Cell 154, 737-747 discloses a neo-substrate that amplifies catalytic activity of Parkinson's disease-related kinase PINK1. La Cognata et al., Neurogenetics (2015) 16:245-263 provides an overview on the impact of alternative splicing in Parkinson's disease.

The inventors of the present invention, however, have surprisingly found out that the c.G192C in the genome leads to a loss of DJ-1 proteins in the affected cells. This is because the inventors have recognized the importance of studying patient-derived primary cells instead of recombinantly expressed DJ-1 proteins. The inventors of the present invention have further conducted experiments using qPCR and an *in vitro* splicing assay, which showed that a splicing defect caused complete skipping of the mutation-carrying exon 3 in the pre-mRNA. This result could only be obtained because the inventors of the present invention have recognized the importance of studying the mutated gene in its native

environment, instead of studying the gene expression using cDNA as for example conducted by Hering et al., 2004 or Malgieri and Eliezer, 2007.

After deciphering the pathogenic mechanism of the c.G192C, the inventors of the present invention were able to establish a method for screening a compound for Parkinson's disease treatment.

The invention thus envisions a method for screening a compound for Parkinson's disease treatment, comprising contacting a cell having a DJ-1 gene containing a c.192G>C mutation with a compound of interest; and testing whether said compound of interest prevents skipping of exon 3 of said DJ-1 gene, thereby identifying said compound as candidate for Parkinson's disease treatment. It is understood that the method is preferably an *in vitro* method.

The term "DJ-1 gene" also known as "PARK7" gene, as used herein refers to a nucleic acid sequence encoding the DJ-1 protein. It is understood that this nucleic acid sequence is preferably a genomic nucleic acid sequence, i.e. comprising introns and exons. It is also understood that the term "DJ-1" gene preferably relates to a human DJ-1 gene. It is envisioned by the invention that the term "DJ-1" gene preferably relates to a "native DJ-1" gene, i.e. a naturally occurring genomic sequence of the DJ-1 gene, which preferably has no known association with a pathologic phenotype of the carrier of said native DJ-1 gene, especially no association with an increased probability of developing Parkinson's disease. In humans, the DJ-1 gene is located at chromosome band 1p36.23. It is further envisioned that the term "DJ-1 gene" preferably relates to the human gene having the cytogenetic location: 1p36.23 having the genomic coordinates (GRCh38): 1:7,961,653-7,985,281 (UCSC Genome Browser on Human Dec. 2013 (GRCh38/hg38) Assembly) as described in OHIM entry 602533 in the version of 15 June 2015.

The term "exon 3" of the "DJ-1" gene, when relating to a human "DJ-1" gene, preferably relates to the sequence set forth in SEQ ID NO: 3. It may also relate to a homolog of SEQ ID NO: 3.

The term "homolog" of a given gene (sequence) or protein (sequence) generally relates to a gene (sequence) or protein (sequence) having at least about 70 %, preferably at least about 75 %, preferably at least about 80%, preferably at least about 85%, preferably at least about 90%, preferably at least about 91%, preferably at least about 92 %, preferably at least about 93%, preferably at least about 94%, preferably at least about 95%, preferably at least about 96%, preferably at least about 97%, preferably at least about 98%, preferably at least about %, preferably at least about 99 % sequence identity to said given gene (sequence) or protein (sequence).

"Percent (%) sequence identity" with respect to a nucleic acid or an amino acid sequence disclosed herein is defined as the percentage of nucleic acid or amino acid residues in a candidate sequence that are pair-wise identical with the nucleic acid or amino acid residues in a reference sequence, after aligning the sequences and introducing gaps, if necessary, to achieve the maximum percent sequence identity. Alignment for purposes of determining percent amino acid sequence identity can be achieved in various ways that are within the skill in the art, for instance, using publically available computer software such as BLAST, ALIGN, or Megalign (DNASTAR) software. Those skilled in the art can determine appropriate parameters for measuring alignment, including any algorithms needed to achieve maximum alignment over the full length of the sequences being compared.

The terms "mutated", "mutant" and "mutation" in reference to a nucleic acid or a polypeptide refers to the exchange, deletion, or insertion of one or more nucleotides or amino acids, respectively, compared to a naturally occurring nucleic acid or polypeptide, i.e. to a reference sequence that can be taken to define a wild-type.

It is understood in this regard that the term "position", when used in accordance with the present invention, means the position of a nucleic acid or an amino acid within a nucleic acid or amino acid sequence depicted herein. This position may be indicated relative to a resembling native sequence, e.g. a sequence of a naturally occurring IgG domain or chain. The term "corresponding" as used herein also includes that a position is not necessarily, or not only, determined by the number of the preceding nucleotides/amino acids. Thus, the position of a given amino acid in accordance with the present invention which may be substituted may vary due to deletion or addition of amino acids elsewhere in the antibody chain.

Thus, under a "corresponding position" in accordance with the present invention it is to be understood that nucleic acids or amino acids may differ in the indicated number but may still have similar neighbouring nucleic acids or amino acids. Said nucleic acids or amino acids which may be exchanged, deleted or added are also encompassed by the term "corresponding position". In order to determine whether a nucleic acid or an amino acid residue in a given nucleic acid or amino acid sequence corresponds to a certain position in the nucleic acid or amino acid sequence of a naturally occurring gene or protein, the skilled person can use means and methods well-known in the art, e.g., alignments, either manually or by using computer programs such as BLAST2.0, which stands for Basic Local Alignment Search Tool or ClustalW or any other suitable program which is suitable to generate sequence alignments.

An "exon" refers to a defined section of nucleic acid that encodes for a protein, or a nucleic acid sequence that is represented in the mature form of an RNA molecule after either portions of a pre-processed (or precursor) RNA have been removed by splicing. The mature RNA molecule can be a messenger RNA (mRNA) or a functional form of a non-coding RNA, such as rRNA or tRNA. The human DJ-1 gene has 8 exons.

An "intron" refers to a nucleic acid region (within a gene) that is not translated into a protein. An intron is a non-coding section that is transcribed into a precursor mRNA (pre-mRNA), and subsequently removed by splicing during

formation of the mature RNA.

"Exon skipping" refers generally to the process by which an entire exon, or a portion thereof, is removed from a given pre-processed RNA, and is thereby excluded from being present in the mature RNA, such as the mature mRNA that is translated into a protein. Hence, the portion of the protein that is otherwise encoded by the skipped exon is not present in the expressed form of the protein.

As used herein, the terms "treat", "treatment" or "therapy" refer to alleviation, attenuation (e.g. of progression), or prevention of a pathological state, such as a disease.

Further envisaged herein is a compound for treating Parkinson's disease. Preferably, the compound is for the treatment of early onset Parkinson's disease. The compound may be for treatment of idiopathic Parkinson Disease. The Parkinson Disease may be caused by or is associated with aberrant splicing. The aberrant splicing may be caused by or is associated with a mutation in e.g. PARK7, PINK1, GBA, or PARK2. The Parkinson disease may be caused by or is associated with a U1-dependent splicing defect. The compound is also preferably for treating PARK7-associated Parkinson's disease, in particular for treating Parkinson's disease associated with a c.G192C mutation in the PARK7 gene.

It is envisioned that the cell used in the methods of the present invention comprises at least one allele of the DJ-1 gene, preferably two. It is further envisioned that the cell has a c.192G>C mutation in at least one allele of the DJ-1 gene. In this context it is noted that "c.192G>C" and "c.G192C" are used synonymously and interchangeably throughout the application and both terms relate to a G->C substitution at position 192 of a reference nucleic acid sequence. In is further envisioned that the cell preferably has two alleles of the DJ-1 gene and that both alleles of the DJ-1 gene have a c. 192G>C mutation.

As used herein, the term "allele(s)" means any of one or more alternative forms of a gene at a particular locus. In a diploid cell of an organism, alleles of a given gene are located at a specific location or locus (loci plural) on a chromosome. One allele is present on each chromosome of the pair of homologous chromosomes.

It is further envisioned that the c.192G>C mutation in a DJ-1 gene may lead to a p.E64D amino acid exchange in the DJ-1 protein.

A cell according to the invention may preferably be an isolated cell. The cell may be any cell, but is preferably a mammalian cell, such as a human cell, a mouse cell, a rat cell, a dog cell, a rabbit cell, a swine cell, a cattle cell, a monkey cell, an ape cell. Most preferably, the cell is a human cell.

It is envisioned that the cell used in the methods of the invention is a human cell or a human cell line. This human cell line preferably contains a DJ-1 gene having a c.192G>C mutation, preferably a c.192G>C mutation in both alleles of the DJ-1 gene. Said human cell line may be a primary fibroblast cell, an immortalized fibroblast cell, an induced pluripotent stem cell (iPSC), a small-molecule-derived neuronal precursor cell (smNPC), or a midbrain-specific dopaminergic neuro (mDA), each of which may preferably be obtained from a patient having at least one c.192G>C mutation in a DJ-1 gene. It is understood that the step of obtaining the cell line from the subject may preferably be excluded from the methods of the invention.

It is further envisioned that the DJ-1 gene containing a c.192G>C mutation that is comprised in the cell used in the methods of the invention may be fused to a reporter gene. As used herein, the term "reporter gene" refers to any gene whose expression can be measured.

A reporter gene may preferably not have any untranslated regions (UTRs). A reporter gene may preferably be a contiguous open reading frame. A reporter gene can have a previously determined reference range of detectable expression.

A reporter gene can express a screenable marker. Exemplary screenable markers include: green fluorescent protein (GFP) or any other fluorescent protein, a β-glucuronidase or uidA gene (GUS) which encodes an enzyme for which various chromogenic substrates are known; a gene which encodes an enzyme for which various chromogenic substrates are known (e.g., PADAC, a chromogenic cephalosporin); a luciferase gene; a tyrosinase gene, which encodes an enzyme capable of oxidizing tyrosine to DOPA and dopaquinone which in turn condenses to melanin; and α-galactosidase, which can be used in colormetric assays. A preferred screenable marker is GFP.

Included within the terms "screenable marker genes" are also genes that encode a secretable marker whose secretion can be detected as a method of identifying or selecting for transformed cells. Examples include markers that encode a secretable antigen that can be identified by antibody interaction, or even secretable enzymes, which can be detected utilizing their inherent biochemical properties. Secretable proteins fall into a number of classes, including small, diffusible proteins which are detectable, (e.g., by ELISA), or small active enzymes which are detectable in extracellular solution (e.g., α-amylase, β-lactamase, phosphinothricin transferase). Other possible selectable or screenable marker genes, or both, are apparent to those of skill in the art.

It is further envisioned by the present invention that the step of testing whether said compound of interest prevents skipping of exon 3 of said DJ-1 gene can be conducted using any method that is known to the skilled person and that is suitable for detection of such a exon skipping. Suitable methods include, but are not limited to, western blot, qPCR, mass spectrometry, detection of reporter gene activity, a microscopy-based assay, or a FACS-based assay.

The invention further envisions a compound which prevents skipping of exon 3 of the DJ-1 gene for use in a

method of treatment of Parkinson's disease wherein (a) the treatment comprises prevention of skipping of exon 3 of the DJ-1 gene in a patient; or (b) the treatment is of a patient having a c.192G>C mutation in the DJ-1 gene in at least one allele; or (c) the Parkinson's disease is associated with a c.G192C mutation in the PARK7 gene. Such compound is preferably obtainable by a method for screening a compound for Parkinson's disease treatment described herein.

According to the present invention, such a compound may be a mutated U1 snRNA allowing the U1 complex to bind to the mutant pre-mRNA. Such a mutated U1 snRNA thus may allow correct splicing of the c.192G>C mutant pre-mRNA. Such a mutated U1 snRNA may preferably have the nucleotide sequence shown in SEQ ID NO: 7.

As used herein, "snRNA" (or "small nuclear ribonucleic acid"), is a class of small RNA molecules that are found within the splicing speckles and Cajal bodies of the cell nucleus in eukaryotic cells. The length of an average snRNA is approximately 150 nucleotides. They are transcribed by either RNA polymerase II or RNA polymerase III, and studies have shown that their primary function is in the processing of pre-messenger RNA (hnRNA) in the nucleus. A snRNA may further be associated with a set of specific proteins, and the complexes are referred to as small nuclear ribonucleoproteins (snRNP) that may form the splicesosome.

Another compound that may be for use in a method of treatment of Parkinson's disease according to the invention is an optionally substituted C1-C10 alkyl carboxylic acid. Such a compound may be butyric acid. Such a compound may also comprise at least one aryl and/or at least one heteroaryl substituent.

As used herein, "alkyl" refers to a straight-chain, or branched alkyl group having 1 to 10 carbon atoms. Exemplary alkyl groups include methyl, ethyl, propyl, isopropyl, butyl, isobutyl, sec-butyl, tert-butyl, pentyl, isoamyl, neopentyl, 1-ethylpropyl, 3-methylpentyl, 2,2-dimethylbutyl, 2,3-dimethylbutyl, hexyl, octyl, etc. The alkyl moiety of alkyl-containing groups, such as alkoxy, alkoxycarbonyl, and alkylaminocarbonyl groups, has the same meaning as alkyl defined above. Alkyl groups may be optionally substituted.

As used herein, "aryl" refers to an aromatic group preferably having in the range of 6 up to 14 carbon atoms, preferably 6 carbon atoms. A "substituted aryl" refers to aryl groups further bearing one or more substituents.

As herein, "heteroaryl" refers to arylic groups containing one or more heteroatoms (e.g., N, O, S, or the like) as part of the ring structure, and preferably having in the range of 3 up to 14 carbon atoms.

A preferred compound that may be for use in a method of treatment of Parkinson's disease according to the invention is a compound having the structural formula (I):

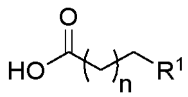

(I),

wherein $R^1$ is optionally substituted aryl or optionally substituted heteroaryl; and n is an integer of from 0 to 8. $R^1$ may preferably be an unsubstituted aryl, preferably a unsubstituted phenyl. Moreover, n may be 0, 1, 2, 3, 4, 5, 6, 7, or 8, preferably 2. A preferred compound may be 4-phenylbutyric acid.

A further compound that may be for use in a method of treatment of Parkinson's disease according to the invention is a compound having the structural formula (II):

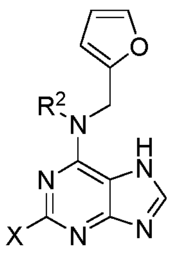

(II),

wherein $R^2$ is hydrogen or optionally substituted alkyl; and X is hydrogen or halogen. $R^2$ is preferably hydrogen or an unsubstituted alkyl, preferably hydrogen or methyl. X is preferably hydrogen or chlorine. Preferred compounds are those in which $R^2$ is hydrogen and X is hydrogen; in which $R^2$ is hydrogen and X is a halogen, preferably chlorine; in which $R^2$ is an unsubstituted alkyl, preferably methyl, and X is a halogen, preferably chlorine, such as a compound where $R^2$ is methyl and X is chlorine. A preferred compound is N6-furfuryladenine (also referred to as "kinetin"). Another preferred compound is 2-chloro-N-[(furan-2-yl)methyl]-7H-purin-6-amine (also referred to as "rectas"). Another preferred compound is 2-chloro-N-[(furan-2-yl)methyl]-N-methyl-7H-purin-6-amine (also referred to as "MolPort-032-631-702" or "032.631.702").

The present disclosure further envisions a method of treating Parkinson's disease comprising administering to

a subject a compound useful for the treatment of Parkinson's disease as described herein. It is understood that the compound may preferably administered in a therapeutically effective amount. It is further understood that the subject is preferably in need of the administration, e.g. that the subject suffers from Parkinson's disease or has a c.192G>C mutation in the DJ-1 gene in at least one allele, preferably both alleles. The subject is preferably human.

A compound used for treatment of Parkinson's disease as described herein may typically be administered in the form of a pharmaceutical composition. The present invention thus further provides a pharmaceutical composition comprising, as an active agent, a compound used for treatment of Parkinson's disease as disclosed herein, and, optionally, one or more pharmaceutically excipient(s), wherein (a) the treatment comprises prevention of skipping of exon 3 of the DJ-1 gene in a patient; or (b) the treatment is of a patient having a c.192G>C mutation in the DJ-1 gene in at least one allele; or (c) the Parkinson's disease is associated with a c.G192C mutation in the PARK7 gene. Accordingly, the use of a compound used for treatment of Parkinson's disease as disclosed herein for the manufacture of a pharmaceutical composition or medicament, preferably for the treatment of Parkinson's disease is also envisaged herein.

A pharmaceutical composition for use in a method of treatment of Parkinson's disease may comprise one or more compounds which prevents skipping of exon 3 of the DJ-1 gene. Such one or more compounds may be compounds which prevents skipping of exon 3 of the DJ-1 gene may be compounds that are described herein. The pharmaceutical composition may comprise at least two compounds which prevents skipping of exon 3 of the DJ-1 gene that are described herein. The pharmaceutical composition may comprise an optionally substituted C1-C10 alkyl carboxylic acid and a compound having the structural formula (II). The optionally substituted C1-C10 alkyl carboxylic acid may be butyric acid or a salt thereof, or a substituted C1-C10 alkyl carboxylic acid or a salt thereof that comprises at least one aryl and/or at least one heteroaryl substituent, or a compound having the structural formula (I) or a salt thereof. The optionally substituted C1-C10 alkyl carboxylic acid is preferably 4-phenyl butyric acid or a salt thereof. The compound of having the structural formula (II) may be N6-furfuryladenine, 2-chloro-N-[(furan-2-yl)methyl]-7H-purin-6-amine, or 2-chloro-N-[(furan-2-yl)methyl]-N-methyl-7H-purin-6-amine. The compound of having the structural formula (II) is preferably 2-chloro-N-[(furan-2-yl)methyl]-7H-purin-6-amine. The pharmaceutical composition preferably comprises 4-phenyl butyric acid or a salt thereof, such as the sodium salt thereof, and 2-chloro-N-[(furan-2-yl)methyl]-7H-purin-6-amine.

The term "pharmaceutical composition" particularly refers to a composition suitable for administering to a human. However, compositions suitable for administration to non-human animals are generally also encompassed by the term.

The pharmaceutical composition and its components (i.e. active agents and optional excipients) are preferably pharmaceutically acceptable, i.e. capable of eliciting the desired therapeutic effect without causing any undesirable local or systemic effects in the recipient. Pharmaceutically acceptable compositions of the invention may for instance be sterile or non-sterile. Specifically, the term "pharmaceutically acceptable" may mean approved by a regulatory agency or other generally recognized pharmacopoeia for use in animals, and more particularly in humans.

A compound used for treatment of Parkinson's disease as described herein is preferably present in the pharmaceutical composition in a therapeutically effective amount. By "therapeutically effective amount" is meant an amount of the active agent that elicits the desired therapeutic effect. Therapeutic efficacy and toxicity can be determined by standard procedures, e.g. in cell culture or in test animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, ED50/LD50. Pharmaceutical compositions that exhibit large therapeutic indices are preferred.

The term "excipient" includes fillers, binders, disintegrants, coatings, sorbents, antiadherents, glidants, preservatives, antioxidants, flavoring, coloring, sweeting agents, solvents, co-solvents, buffering agents, chelating agents, viscosity imparting agents, surface active agents, diluents, humectants, carriers, diluents, preservatives, emulsifiers, stabilizers and tonicity modifiers. It is within the knowledge of the skilled person to select suitable excipients for preparing the desired pharmaceutical composition of the invention. Exemplary carriers for use in the pharmaceutical composition of the invention include saline, buffered saline, dextrose, and water. Typically, choice of suitable excipients will inter alia depend on the specific active agent used, the disease to be treated, and the desired formulation of the pharmaceutical composition.

The present invention further provides pharmaceutical compositions comprising one or more of the active agents specified above and one or more additional active agents that are suitable for treatment of the disease to be treated. Examples of active ingredients suitable for combinations include levodopa (or L-DPOA), a dopamine agonists such as bromocriptine, pergolide, pramipexole, ropinirole, piribedil, cabergoline, apomorphine, and lisuride, a MAO-B inhibitor (monoamine oxidase inhibitor) such as selegiline or rasagilines, amantadine, anticholinergics, clozapine, cholinesterase inhibitors, modafinil, donepezil, quetiapine, ziprasidone, aripiprazole, or paliperidone.

The exact dosage of the compound used for treatment of Parkinson's disease as described herein (or the pharmaceutical composition comprising the same) will be ascertainable by one skilled in the art using known techniques. Suitable dosages provide sufficient amounts of the compound used for treatment of Parkinson's disease as described herein and are preferably therapeutically effective.

As is known in the art, adjustments for purpose of the treatment (e.g. remission maintenance vs. acute flare of

disease), route, time and frequency of administration, time and frequency of administration formulation, age, body weight, general health, sex, diet, severity of the disease state, drug combination(s), reaction sensitivities, and tolerance/response to therapy may be required. Suitable dosage ranges for compound used for treatment of Parkinson's disease as described herein or its derivatives can be determined using data obtained from cell culture assays and animal studies and may include the $ED_{50}$. It is recognized that treatment may require a single administration of a therapeutically effective dose or multiple administrations of a therapeutically effective dose of the compound used for treatment of Parkinson's disease as described herein. E.g., the compound used for treatment of Parkinson's disease as described herein (or a pharmaceutical composition comprising the same) might be administered daily, every 3 to 4 days, every week, or once every two weeks, or once within a month depending on formulation, half-life and clearance rate of the particular compound or composition.

A variety of routes are applicable for administration of the pharmaceutical composition described herein. Typically, administration may be accomplished enterally or parenterally. Methods of enteral delivery include oral application, for example as a tablet or capsule or int the form of powder or granulate. Methods of parenteral delivery include topical, intra-arterial, intramuscular, subcutaneous, intramedullary, intrathecal, intraventricular, intravenous, intraperitoneal, intrauterine, intravaginal, sublingual or intranasal administration.

The pharmaceutical compositions of the invention can be formulated in various forms, e.g. in solid, liquid, gaseous or lyophilized form and may be, for instance, in the form of a tablet, a pill, a capsule, a powder, a granule, a solution, an ointment, a cream, transdermal patches, a gel, an aerosol, suspensions, emulsions, syrups, liquids, elixirs, extracts, tincture or fluid extracts or in a form which is particularly suitable for the desired method of administration. Processes known *per se* for producing medicaments are indicated in 22nd edition of Remington's Pharmaceutical Sciences (Ed. Maack Publishing Co, Easton, Pa., 2012) and may include, for instance conventional mixing, dissolving, granulating, dragee-making, levigating, emulsifying, encapsulating, entrapping or lyophilizing processes. After pharmaceutical compositions of the invention have been prepared, they can be placed in an appropriate container and labeled for treatment of an indicated condition. Such labeling would for instance include amount, frequency and method of administration.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

Nothing herein is to be construed as an admission that the invention is not entitled to antedate such disclosure by virtue of prior invention.

## BRIEF DESCRIPTION OF THE SEQUENCE LISTING

SEQ ID NO 1: cDNA sequence of human wild type (wt) PARK7 (DJ-1)

SEQ ID NO 2: cDNA sequence of human c.192G>C PARK7 (DJ-1) mutant

SEQ ID NO 3: exon 3 of human wild type (wt) PARK7 (DJ-1)

SEQ ID NO 4: exon 3 of human c. 192G>C PARK7 (DJ-1) mutant

SEQ ID NO 5: amino acid sequence of human DJ-1 wild type (wt) protein

SEQ ID NO 6: amino acid sequence human DJ-1 E64D mutant

SEQ ID NO 7: mutated U1 snRNA

SEQ ID NO 8: fragment of U1 snRNA

SEQ ID NO 9: fragment of DJ-1 RNA (wt)

SEQ ID NO 10: fragment of DJ-1 RNA (c. 192G>C)

SEQ ID NO 11: fragment of mutated U1 snRNA

SEQ ID NO 12: wt DJ-1 mRNA

SEQ ID NO: 13: Δex3 DJ-1 mRNA

**BRIEF DESCRIPTION OF THE DRAWINGS**

**Figure 1:** cDNA sequence of human wild type (wt) PARK7 (DJ-1) (SEQ ID NO: 1) and human c.192G>C PARK7 (DJ-1) mutant (SEQ ID NO: 2). Exon 2 is denoted in standard letters, exon 3 is denoted in italic letters, exon 4 is denoted in underlined letters, exon 5 is denoted in bold letters, exon 6 is denoted in italic and underlined letters, exon 7 is denoted in bold italic letters. Exon 3 in the human c. 192G>C PARK7 (DJ-1) mutant (SEQ ID NO: 2) is only present if the mRNA has been correctly spliced, e.g. after pharmacological rescue.

**Figure 2:** amino acid sequence of human DJ-1 wild type (wt) protein (SEQ ID NO: 5) and of human DJ-1 ED64D mutant (SEQ ID NO: 6).

**Figure 3:** mutated U1 snRNA. The mutated nucleic acid (c->g substitution) is underlined. U1 termination sequence is shown in italic.

**Figure 4: A) Western blot and B) densitometry of Western blot of cell lysates prepared from human control fibroblasts and c.192G>C *DJ-1* mutation carriers.** Samples are separated in a 4 % acrylamide SDS-PAGE gel. Result shows a variation of DJ-1 level in controls and heterozygous c. 192G>C *DJ-1* mutation carriers and results show the undetectability of DJ-1 protein in homozygous c.192G>C *DJ-1* mutation carriers. N=1.

**Figure 5: A) Western blot and B) densitometry of Western blot of DJ-1 protein level in immortalised fibroblasts.** Cell lysates prepared from immortalised fibroblasts of a healthy control and indicated c.192G>C *DJ-1* mutation carriers were prepared and separated in a 4 % acrylamide SDS-PAGE gel and detected with a DJ-1 specific antibody. Result shows an approximately 65 % reduced level of DJ-1 protein in the heterozygous c.192G>C *DJ-1* mutation carrier and DJ-1 protein is not detectable homozygous c.192G>C *DJ-1* mutation carriers. Ratios were normalised to healthy control female A. N=1.

**Figure 6: *DJ-1* amplification of cDNA of indicated patient derived fibroblasts of c.192G>C *DJ-1* mutation carriers and healthy control.** Fibroblasts were pelleted, RNA was extracted from the cell pellet and cDNA was transcribed. *DJ-1* was amplified by PCR and subsequently run on an agarose gel. DNA gel analysis showed reduced mRNA length in both homozygous c.192G>C *DJ-1* mutation carriers. Bands corresponding to both reduced and normal length mRNA were visible in the heterozygous c. 192G>C *DJ-1* mutation carrier.

**Figure 7: Schematic overview of Minigene assay.** wt *DJ-7* exon 3 or mutant DJ-*1* exon 3 carrying the c.192G>C *DJ-1* mutation respectively including flanking intronic sequences were cloned in a vector (pSPL3) (Invitrogen GmbH, Karlsruhe, Germany) between two given exons (A and B). After transfection of the final plasmids splicing of wt or mutant exon 3 was analysed by rtPCR and subsequent sequencing of cDNA. It was analysed whether the c.192G>C

*DJ-1* mutation leads to physiological splicing or missplicing with skipping of exon 3.

**Figure 8: rtPCR result of minigene assay of wt and c.192G>C *DJ-7* mutation carrying exon.** Minigene transient transfection assay showing the effect of wt exon 3 and mutant DJ-1 exon 3 carrying the c.192G>C mutation. Two prominent products are seen on agarose gel, with the shorter band representing a PCR product lacking *DJ-1* exon 3 (lane 3 and 4) and the longer band a product including *DJ-1* exon 3 (lane 2 and 5). No template control (NTC). Size standard: 100 bp ladder (Invitrogen GmbH, Karlsruhe, Germany).

**Figure 9: qPCR of *DJ-1* RNA expression level with and without exon 3 in immortalised fibroblasts.** Cell pellets were collected, RNA was isolated and transcribed into cDNA. cDNA was amplified by qPCR using one primer pair that amplifies *DJ-1* cDNA with and without exon 3 (A) and one primer pair amplifying *DJ-1* cDNA only in the presence of exon 3 (B). **A**) DJ-1 RNA expression is about 40 % reduced in c.192G>C *DJ-1* mutation carriers. **B**) Correctly spliced full length *DJ-1* mRNA is around 30 % reduced in heterozygous c.192G>C *DJ-1* mutation carrier and about 80 % in homozygous c.192G>C *DJ-1* mutation carriers. Ratios were normalised to healthy control. Values show mean + SEM. ** $p \leq 0.01$, **** $p \leq 0.0001$ by one-way ANOVA followed by Tukey's multiple comparisons test. N=3.

**Figure 10: qPCR of *DJ-1* RNA expression level with and without exon 3 in iPSCs.** Cell pellets of indicated iPSC clones were collected, RNA was isolated and transcribed into cDNA. cDNA was amplified by qPCR using one primer pair that amplifies *DJ-1* cDNA with and without exon 3 (A) and one primer pair amplifying *DJ-1* cDNA only in the presence of exon 3 (B). **A**) *DJ-1* RNA expression is reduced in two indicated homozygous c.192G>C *DJ-1* mutation carrying clones from affected and premotor diseased individuals. **B**) Correctly spliced full length *DJ-1* gene expression is reduced to 50 % in one heterozygous c.192G>C *DJ-1* mutation carrying clone in comparison to healthy control and strongly reduced in all homozygous c.192G>C *DJ-1* mutation carriers. Ratios were normalised to healthy control 2. N=1.

**Figure 11: Scheme of splicing of c.192G>C *DJ-1* mutation carrying pre-mRNA.** Upper lane shows *DJ-1* exon 3 carrying the c.192G>C mutation, which is the last base of exon 3 (red) and therefore lies in the splice donor site. During the splicing process of the pre-mRNA, exon 3 does not get recognised as exon as the c.192G>C *DJ-1* mutation changes the splice donor site which, no longer gets recognised as such. Due to that, the splice acceptor does not get recognised either. Finally, this leads to skipping of exon 3.

**Figure 12: Binding of U1 snRNP to wt *DJ-1* exon 3 and c.192G>C mutant *DJ-1* exon 3.** U1 snRNP binds to the pre-mRNA by base pairing to initiate the formation of the spliceosome. **A**) Binding of U1 snRNP to wt *DJ-1* exon 3. Binding takes place in the presence of 4 mismatches. **B**) Not binding of U1 snRNP to c.192G>C mutant *DJ-1* exon 3. The c.192G>C *DJ-1* mutation leads to the fifth mismatch between U1 snRNA and the pre-mRNA therefore U1 snRNP does not bind to c.192G>C mutant *DJ-1* exon 3.

**Figure 13: Binding of C>G mutant U1 snRNP to c.192G>C mutant *DJ-1* exon 3.** By mutagenesis, a C>G base exchange (indicated in cyan) was introduced in the U1 snRNA so that it matches the c.192G>C *DJ-1* mutation and hence binds to c.192G>C mutant *DJ-1* exon 3.

**Figure 14: rtPCR result of minigene assay of wt and c.192G>C *DJ-7* mutation carrying exon cotransfected with wt U1 snRNA or c.192G>C U1 snRNA.** Minigene transient transfection assay showing the effect of wt exon 3 and mutant *DJ-1* exon 3 carrying the c.192G>C mutation cotransfected with wt U1 snRNA or c.192G>C U1 snRNA. Wt exon 3 in all three indicated and tested conditions leads to the inclusion of exon 3, resulting in a long band in the rtPCR (lane 2, 3 and 4). rtPCR results of c.192G>C mutation carrying exon 3 minigene without cotransfection and after cotransfection with wt U1 snRNA lead to a short band, representing skipping of exon 3 (lane 5 and 6). Two prominent products, the short and the long band, are seen on agarose gel, when c.192G>C exon 3 minigene was cotransfected with c.192G>C U1 snRNA (lane 7). No template control (NTC). Size standard: 100 bp ladder (Invitrogen GmbH, Karlsruhe, Germany).

**Figure 15: Transient transfection of wt and c.192G>C U1 snRNA in immortalised fibroblasts of the index patient.** 6 $\mu$g of empty vector, wt U1 snRNA or c.192G>C U1 snRNA respectively were electroporated into immortalised fibroblasts of the index patient carrying the c.192G>C *DJ-1* mutation using the Amaxa nucleofector I (Lonza Group Ltd, Basel, CH). 24 h after electroporation cell pellets were collected, RNA was isolated and transcribed into cDNA. cDNA was amplified by qPCR using one primer pair that amplifies *DJ-1* cDNA with and without exon 3 (A) and one primer pair amplifying *DJ-1* cDNA only in the presence of exon 3 (B). A) A significantly higher RNA expression was measured after electroporation of wt U1 snRNA. Expression was significantly higher in comparison to RNA

expression after electroporation with empty vector and c.192G>C U1 snRNA. B) An increase in RNA expression of *DJ-1* RNA including exon 3 was measured after electroporation with wt U1 snRNA in comparison to electroporation with empty vector. The increase was even higher after electroporation with c.192G>C U1 snRNA. Ratios were normalised to empty vector treatment. Values show mean + SEM. * $p \leq 0.05$, ** $p \leq 0.01$ by one-way ANOVA followed by Tukey's multiple comparisons test. N=3. C) DJ-1 was amplified by rtPCR from cDNA from homozygous c.192G>C *DJ-1* mutation carrier after transient electroporation with empty vector control (lane 2), wt U1 snRNA (lane 3) or c.192G>C U1 snRNA (lane 4). Lane 1 shows marker and lane 5 NTC. Rescue of splicing of DJ-1 exon 3 indicated by longer band can only be seen after electroporation with c.192G>C U1 snRNA.

**Figure 16: Expression of recombinant DJ-1 in patient-derived smNPC.** A. Transduction strategy with lentiviral vectors expressing either ΔEx3- or full length DJ-1. B. Enrichment of stably transduced cells. C. DJ-1 protein levels in transduced cells (n=3).

**Figure 17: Genetic rescue of DJ-1 by expression of mutant U1 snRNA.** A. Transduction strategy with lentiviral vectors expressing either ΔEx3- or full length DJ-1 and enrichment of stably transduced cells. B. DJ-1 protein levels of stably transduced cells. C. Level of Dj-1 full length mRNA in transduced patients smNPC line M1 normalized to beta Actin.

**Figure 18: Genetic rescue of DJ-1 by expression of mutant U1 snRNA and full length DJ-1.** A. DJ-1 protein levels in smNPC. B. DJ-1 protein levels neurons DIV 14. C. Level of full length DJ-1 mRNA in patient's smNPC line M1 normalized to beta Actin (left), DAG1 (middle) and HMBS (right).

**Figure 19: Chemical rescue of DJ-1 by expression in patient-derived smNPC.** Cells were treated for 48h with 4-Phenybutric acid or Sodium Butyrate at indicated concentrations and DJ-1 protein level was subsequently analyzed by western blot analysis. Dj-1 level is normalized to beta Actin and DJ-1 level of healthy control line K2 is set to 1.

**Figure 20: Chemical rescue of DJ-1 by expression in patient-derived smNPC.** Cells were treated for 7d with Rectas or its analogue 032.631.702 at indicated concentrations and DJ-1 protein level was subsequently analyzed by western blot analysis. Dj-1 level is normalized to beta Actin and DJ-1 level of healthy control line K2 is set to 1.

**Figure 21: The c.192G>C DJ-1 mutation leads to pronounced loss of DJ-1 protein in mutation carriers.** (a) Pedigree structure of the family of c.192G>C mutation carriers. Black symbols denote homozygous carriers of the c.192G>C DJ-1 mutation. Symbols with a black dot denote heterozygous c.192G>C DJ-1 mutation carriers. White symbols denote healthy individuals and grey symbols containing a question mark denote untested individuals. Pedigree has been modified to prevent identification of individuals. (b-e) Immunoblots and densitometry of DJ-1 levels in fibroblasts (b), iPSCs (c), smNPCs (d) and mDA neurons (e) comparing controls (black bars), heterozygous (grey bars) and homozygous (white bars) c.192G>C mutation carriers. (f) Representative immunoblot of DJ-1 levels after blocking of proteasomal degradation in patient-derived smNPCs with indicated increasing concentrations of MG132 compared to untreated, DMSO treated and healthy control cells. β-actin was used as loading control and detection of increasing polyubiquitination as treatment control. n = 4 Western blots (g) Representative immunoblot of DJ-1 levels after blocking of degradation via autophagy-lysosomal pathway in patient-derived smNPCs with indicated increasing concentrations of bafilomycin A1 compared to untreated, DMSO treated and healthy control cells. β-actin was used as loading control and detection of increasing LC3II levels as treatment control. n = 4 Western blots, (H) Number of peptides with a sequence unique for DJ-1 (solid bars, right Y-axis) detected by mass spectrometry and signal intensity of this measurements calculated as label free quantification (LFQ) (striped bars, left Y-axis) in protein lysates of control and patient derived smNPCs.

**Figure 22: Loss of DJ-1 protein in homozygous c.192G>C mutation carrier.** (a) Comparison of DJ-1 levels in fibroblasts by Western blot. (left) GAPDH and DJ-1 immunoblot of lysates from healthy control fibroblasts (left panels) and from fibroblasts of heterozygous and homozygous c.192G>C mutation carriers (right panels). (right) Quantification of the Western blot. (b) (top) Immunoblot of indicated smNPC lines comparing control lines with patient derived lines using three different anti-DJ-1 monoclonal antibodies (mAB) from Cell Signaling (mAB D29E5 XP, left panels), Abcam (mAB EP2816Y, middle panels) and Invitrogen (mAB E2.1, right panels). (bottom) Regions corresponding to the peptides that have been used to generate the three mAB are highlighted in the DJ-1 amino acid sequence with exon 3 highlighted in red letters.

**Figure 23: Characterization, transduction and differentiation of smNPC.** (a) Transduction efficiency with lentiviral vectors encoding GFP and either U1 wt snRNA or G>C U1 snRNA was detected by flow cytometry after

enrichment by FACS sorting. (b) Neuronal differentiation of smNPC. (left) Representative plots of flow cytometric analysis of DIV 30 neurons. Cells were stained with antibodies against CD200 and CD49f and gates were set to detect percentage of CD200+/CD49f- neurons. (right) Average percentage of neurons after in vitro differentiation from indicated smNPC lines. Bars show mean + s.e.m. n = 6 (c) Transduction efficiency with lentiviral vectors encoding GFP and either wt or Δex3 DJ-1 was detected by flow cytometry after enrichment by FACS sorting.

**Figure 24: Mechanism of loss of protein.** (a) Quantification of DJ-1 immunoblots of indicated smNPC clones transduced with wt DJ-1 (dark grey bars) or Δex3 DJ-1 (light grey bars) lentiviral constructs in comparison to untransduced (black bars) clones. DJ-1 levels were normalized to β-actin. Values are normalized to control C2 and show mean + s.e.m. *** $p \leq 0.001$, **** $p \leq 0.0001$ by one-way ANOVA followed by Dunnett' s multiple comparisons test. $n \geq 5$ (b) One-Step RT-qPCR results of the indicated smNPC clones. Levels of full-length DJ-1 mRNA (left graph) and Δex3 DJ-1 mRNA (right graph) were detected by duplex One-step RT-qPCR using TaqMan probes for either DJ-1 variant and β-actin mRNA as internal control. DJ-1 mRNA levels were normalized to expression levels of the β-actin gene ACTB. Values show mean + s.e.m. **** $p \leq 0.0001$ by one-way ANOVA followed by Dunnett' s multiple comparisons test (left graph) and Turkey' s multiple comparison test (right graph). $n \geq 3$ (c) Quantification of DJ-1 immunoblots of neurons differentiated in vitro from indicated smNPC clones transduced with wt DJ-1 (dark grey bars) and Δex3 DJ-1 (light grey bars) lentiviral constructs in comparison to untransduced clones (black bars). DJ-1 levels were normalized to β-actin. Values are normalized to control C2 and show mean + s.e.m. ** $p \leq 0.01$ by one-way ANOVA followed by Dunnett' s multiple comparisons test. $n \geq 3$ (d-e) Secondary structure predictions for wt DJ-1 mRNA (d, SEQ ID NO: 12) and Δ ex3 DJ-1 mRNA (e, SEQ ID NO: 13) were generated using the RNAfold software (f) (left) Northern blot of cytosolic (first panel) and nuclear (2nd panel) fraction of indicated smNPC lines. mRNA was visualized using a DJ-1 specific probe. (right) Western blot of the same cytosolic and nuclear fractions of smNPC C3 (3rd panel) and hom2-4 (4th panel) stained with anti-p84 and anti-Tubulin antibodies to confirm purity of the fractions. (g) Expression of recombinant DJ-1 in prokaryotic system by transformation of BL21 bacteria with wt DJ-1 and Δex3 DJ-1. Representative figure of DJ-1 cDNA amplification from bacterial RNA without and upon induction of plasmid expression by Isopropyl β- D -1-thiogalactopyranoside (IPTG) (upper panel) and immunoblot of DJ-1 protein from bacterial lysates without and upon IPTG induction (lower panel).

**Figure 25: Loading controls of Figure 24.** (a) Formaldehyde gel of RNA from cytosolic and nuclear fractions of indicated smNPC served as loading control for the Northern blot from Fig. 4e. RNA was visualized by ethidium bromide. (b) Ponceau red staining of the nitrocellulose membrane used in Fig. 4f was used to confirm equal loading of protein amounts of all samples.

**Figure 26: Genetic and pharmacologic rescue of loss of DJ-1-associated cellular phenotypes.** (a) (left) Representative images of TMRM (red) and DAPI (blue) stained immortalized fibroblasts from control C-im (left image) and patient-derived line hom2-im (right image). (right) MMP represented as mean fluorescence intensity (MFI) of TMRE signal normalized to control C-im. Images from 36 individual cells of each line were analyzed by an investigator blinded to the experimental design. Values show mean + s.e.m. * $p \leq 0.05$, ** $p \leq 0.01$ by two-way ANOVA followed by Dunnett's multiple comparisons test. (b) MMP quantification as shown in (a) of indicated lines after transfection with empty or wt DJ-1 expression vector. Values show mean + s.e.m. n = 3. **** $p \leq 0.0001$ by one-way ANOVA followed by Tukey's multiple comparisons test. (c) Levels of correctly spliced full-length DJ-1 mRNA in in vitro differentiated neurons from indicated clones after 4 d treatment with solvent controls or indicated concentrations of sodium phenylbutyrate and Rectas (grey bars) compared to untreated neurons (black bars) determined by duplex TaqMan RT-qPCR. Full-length DJ-1 mRNA levels were normalized to expression levels of the β -actin gene ACTB. Values are normalized to control C2 and show mean + s.e.m. * $p \leq 0.05$ by one-way ANOVA followed by Tukey's multiple comparisons test. $n \geq 5$ (d) (top) Representative immunoblot of DJ-1 and β-actin of in vitro derived neurons from indicated clones treated for 14 d with solvent controls or indicated concentrations of sodium phenylbutyrate and Rectas (grey bars) compared to untreated neurons (black bars). (bottom) Quantification of DJ-1 levels normalized to β -actin. Values are normalized to control C2 and show mean + s.e.m. * $p \leq 0.05$ by one-way ANOVA followed by Tukey's multiple comparisons test. $n \geq 3$ (e) Densitometry of DJ-1 immunoblots of indicated immortalized fibroblast lines after 4 d of treatment with solvent controls or indicated concentrations of sodium phenylbutyrate and Rectas (grey bars) compared to untreated lines (black bars). DJ-1 levels were normalized to β-actin. Values are normalized to control c-im and show mean + s.e.m. * $p \leq 0.05$ by one-way ANOVA followed by Dunnett's multiple comparisons test. n = 5 (f) Flowcytometric analysis of MMP by TMRE staining in indicated immortalized fibroblast lines after 4 d treatment with solvent controls or indicated concentrations of sodium phenylbutyrate and Rectas (grey bars) compared to untreated lines (black bars). Values are normalized to control C-im and show mean + s.e.m. * $p \leq 0.05$, ** $p \leq 0.01$ by one-way ANOVA followed by Dunnett's multiple comparisons test. n = 7.

**Figure 27:** Level of relative full length DJ-1 protein in patient-derived neurons after 4 or 8 d of treatment with solvent controls or indicated concentrations of sodium phenylbutyrate or Rectas (grey bars). DJ-1 levels were normalized to β-actin.

## EXAMPLES

The following examples illustrate the invention. These examples should not be construed as to limit the scope of this invention. The examples are included for purposes of illustration and the present invention is limited only by the claims.

### Example 1: Establishment of an iPSC model from patient derived fibroblasts

Reprogramming of both homozygous c.192G>C *DJ-1* mutation carriers and the heterozygous c.192G>C *DJ-1* mutation carrier was performed using retroviral vectors expressing the classical four 'Yamanaka factors'. A minimum of 20 clones per individual was picked, passaged and stocks were frozen down for subsequent characterization of the clones. Characterization included tests about pluripotency and stem cell characteristics of the clones. Therefore, qPCR was performed to ensure silencing of the four exogenous viral factors and endogenous expression of pluripotency markers, ICC was used to show the expression of stem cell markers. Furthermore, the picked clones were tested for major chromosomal aberrations and mutations via G-banding and a genotyping microarray analysis. Additionally, the mutation carrying exon 3 of *DJ-1* was sequenced in all clones to verify the correct genotype. Likewise, the ability of the clones to differentiate in cell types of all three germ layers was tested by ICC after differentiating the clones. At least two different clones per one individual were correctly characterized and a sufficient number of stocks was frozen down.

### Example 2: DJ-1 protein level varies between different human control fibroblasts and c.192G>C *DJ-1* mutation carriers do not express DJ-1 protein

Loss of DJ-1 function is a rare cause of familial PD. To calculate the steady state DJ-1 protein levels in different healthy individuals in comparison to the c.192G>C *DJ-1* mutation carriers, samples from the respective individuals were analyzed on a low percentage SDS-PAGE gel (4 % acrylamide). We took cell lysates from three different female human control individuals and from two different male human control individuals. Lysates from fibroblasts of family members of the c.192G>C *DJ-1* family were taken as well. Lysates were taken from individual IV.9 who is a female heterozygous c.192G>C *DJ-1* mutation carrier, from individual III. 7, who is a male heterozygous c.192G>C *DJ-1* mutation carrier and from individual III. 11 and III. 13 who are both homozygous c.192G>C *DJ-1* mutation carriers; III. 11 being female and premotor diseased and III. 13 being the male index patient with motor symptoms.

DJ-1 proteins were examined by immunoblotting using a DJ-1 specific antibody (Figure 4). Whereas DJ-1 protein levels vary between control fibroblasts and heterozygous c.192G>C *DJ-1* mutation carriers, DJ-1 protein is not detectable in both homozygous c.192G>C *DJ-1* mutation carriers.

Primary fibroblasts from patients are a useful tool but at the same time they only grow very slow and particularly from older people they become senescent after a certain times of passaging. To not lose the ability to work with fibroblasts from the c.192G>C *DJ-1* family we immortalized fibroblasts using a lentiviral vector expressing SV40. This opened the opportunity to work with cells from patients that grow faster and can be used at higher passages.

To test if the immortalization had an effect on DJ-1 protein level and to see if the c.192G>C *DJ-1* mutation still results in undetectable DJ-1 protein in immortal cells we repeated the Western blot for DJ-1 in this cell type which has a higher metabolic rate. Indeed DJ-1 protein could not be detected in both homozygous c.192G>C *DJ-1* mutation carriers. The DJ-1 protein level of the heterozygous c.192G>C *DJ-1* mutation carrier was reduced to about 20 % of the amount seen in the healthy control (Figure 5).

### Example 3: The c.192G>C *DJ-1* mutation causes skipping of *DJ-1* exon 3

The findings on DJ-1 protein level in c.192G>C *DJ-1* mutation carriers raised interest in the underlying effect of the mutation. In a first experiment, RNA from patient derived fibroblasts was isolated, transcribed into cDNA and amplified *DJ-1* by PCR. Agarose gel revealed a reduced length of c.192G>C *DJ-1* carrying cDNA and a normal size band as well as the short band in the heterozygous c.192G>C carrying cDNA. cDNA from a healthy control served as positive control for correct length (Figure 6).

Subsequently, these results led us to sequence the amplified cDNA to find out which sequence segment was missing in the cDNA of c.192G>C *DJ-1* mutation carriers. Sequencing result shows that the end of *DJ-1* exon 2 is directly followed by the beginning of exon 4. Complete sequence of *DJ-1* exon 3 was missing or skipped.

After obtaining these results, we wanted to validate the finding of skipping of *DJ-1* exon 3 in c.192G>C *DJ-1*

mutation carrying cells. Therefore, minigene assay to assess the effect of *DJ-1* exon 3 on splicing was performed.

Minigene assay is used to analyze splicing of an exon of interest by cloning it into a vector (pSPL3) between two given exons (Exon A and Exon B) and subsequent analysis of the cDNA by rtPCR and sequencing (Figure 7). In brief, wt DJ-1 exon 3 or mutant DJ-1 exon 3 carrying the c.192G>C DJ-1 mutation respectively including flanking intronic sequences were cloned in a vector (pSPL3) (Invitrogen GmbH, Karlsruhe, Germany) between two given exons (A and B). After transfection of the final plasmids splicing of wt or mutant exon 3 was analyzed by rtPCR and subsequent sequencing of cDNA. It was analyzed whether the c.192G>C DJ-1 mutation leads to physiological splicing or missplicing with skipping of exon 3.

In the minigene assay we tested wt exon 3 of the control and mutant exon 3 (carrying the c.192G>C *DJ-1* mutation) from the index patient. At the same time minigene constructs were tested where the wt exon 3 was mutated to carry the c. 192G>C mutation and the corrected exon from the index patient now carrying the wt allele. Agarose gel electrophoresis (Figure 8) revealed that cultures transfected with pSPL3 constructs with the mutant c.192 C-allele yielded rtPCR products that were clearly smaller than products from cultures transfected with the wildtype c.192 G-allele, indicating abnormal splicing/skipping of the mutant minigene construct. Subsequent sequencing showed that rtPCR products derived from the transfection with the mutant c.192 C-allele lacked exon 3 of the DJ1 gene.

The c.192G>C *DJ-1* mutation in exon 3 which does not lead to a premature stop codon and which does not cause a frameshift in the RNA caused the skipping of exon 3 in the minigene assay. In addition to the correction of the mutation in the patient exon 3 and the mutating of exon 3 to carry the c.192G>C *DJ-1* mutation from a healthy control in the minigene, sequencing of the entire *DJ-1* locus of the patient's DNA was performed. This was done in order to have a second confirmation that the c.192G>C *DJ-1* mutation suffices to cause skipping of exon 3 in c.192G>C *DJ-1* mutation carriers and other sequence variants can be excluded.

The minigene assay showed, in an artificial system, that the c.192G>C *DJ-1* mutation which lies at the end of exon 3 leads to skipping of the respective exon. Skipping of this exon does not lead to a frameshift or a premature stop codon.

Next, we wanted to verify our minigene assay results in the different cell types available from the c.192G>C *DJ-1* mutation carriers. Therefore, cells were grown under standard conditions, harvested, RNA was extracted and transcribed into cDNA. Quantitative qPCR was performed using cDNA and two different pairs of primers for amplification. One primer pair to detect correctly spliced DJ-1 as well as cDNA lacking exon 3 and another primer pair where one primer is complementary with exon 2 and exon 3, leading to only correctly spliced (with exon 3) *DJ-1* cDNA detected by qPCR. qPCR was performed with cDNA from immortalized fibroblasts (Figure 9), iPSCs, mDA neurons and smNPCs.

qPCR results generated with primers that detect normal and misspliced *DJ-1* cDNA show a reduction which is not significant of *DJ-1* RNA expression in all c. 192G>C *DJ-1* mutation carriers (Figure 9A). Reduction is even higher when looking at normal spliced RNA where we see a significant reduction of *DJ-1* RNA expression between the healthy control and the heterozygous c.192G>C *DJ-1* mutation carrier. Even lower levels in both indicated homozygous c.192G>C *DJ-1* mutation carriers were seen. RNA expression of *DJ-1* is significantly reduced in both homozygous c.192G>C *DJ-1* mutation carriers when compared to healthy control or heterozygous carrier of the same mutation (Figure 9B).

When looking at the qPCR results in iPSCs derived from these fibroblasts one sees a reduction in RNA expression of *DJ-1* with and without skipping of exon 3 of the c.192G>C *DJ-1* mutation carriers. With two clones (homozygous premotor diseased c4 and homozygous affected c4) being particularly low (Figure 10A). The results generated with primers that only detect *DJ-1* cDNA when exon 3 is present all cDNAs from homozygous carriers are practically not detectable. The two indicated heterozygous clones vary with one showing the same RNA expression level as the healthy control and the other one showing a 50 % decrease in correctly spliced *DJ-1* RNA (*Figure 10B*).

**Example 4: Genetically modified U1 rescues skipping of exon 3**

Further experiments aimed at analyzing why the c.192G>C base exchange leads to missplicing of *DJ-1* pre-mRNA and skipping of exon 3 during the splicing process. Hypothesized mechanism of splicing of pre-mRNA carrying the c.192G>C *DJ-1* mutation is shown (Figure 11). The c.192G>C *DJ-1* mutation lies at the end of exon 3 and hence in the splice donor recognition site. If the splice donor sequence does not get recognized as such due to the mutation, the splice acceptor site will also not be recognized. During the splicing process, U1 snRNP and U2 snRNP will recognize the splice donor sequence of exon 2 and the splice acceptor sequence of exon 4 respectively, surrounding exon 3, but not exon 3 itself. This will finally lead to skipping of *DJ-1* exon 3 and a shortened *DJ-1* mRNA (Figure 11).

As the c.192G>C *DJ-1* mutation lies in the splice donor site normally it would get recognized by the U1 snRNP which is initiating the formation of the spliceosome. U1 snRNP recognizes the 5' splice site through base pairing at the 5' end. Of note, a hundred percent match is not necessary. In case of *DJ-1* exon 3 in the wt situation there are already four mismatches between U1 snRNA and the splice donor site (Figure 12A). The presence of the c.192G>C *DJ-1* mutation leads to five mismatches between U1 and the splice donor site (Figure 11B). The aim of this part of the project was to test whether this additional mismatch causes U1 snRNP to not recognize the splice donor site hence leading to

skipping of exon 3 (Figure 11).

To test this hypothesis, one base of the U1 snRNA was modified in the sense that it matches the c.192G>C mutation in *DJ-1* exon 3, namely C>G were exchanged as indicated in cyan (Figure 13).

In a splicing reaction where C>G mutant U1 snRNP is present and recognizes the splice donor sequence of c.192G>C mutant *DJ-1* exon 3 skipping of this exon does not take place.

In order to test the hypothesis, the previously performed minigene experiment was repeated and a plasmid expressing c.192G>C U1 snRNA was co-transfected.

The minigene construct expressing wt exon 3 resulted in the inclusion of the exon 3 when the minigene assay was performed with the minigene construct alone, when wt U1 snRNP was co-transfected and when c.192G>C U1 snRNA was co-transfected, this is shown by the long prominent bands on the agarose gel picture in (Figure 14) lane 2 - 4. When the experiment was performed with a minigene construct with the c.192G>C mutation carrying *DJ-1* exon 3 alone or co-transfected with wt U1 snRNA, a short prominent band can be seen on lane 5 and 6 of the agarose gel as rtPCR result. This means that in these cases mutant exon 3 was skipped. When this minigene construct was co-transfected together with c.192G>C U1 snRNA in the minigene assay, however, two products were amplified by rtPCR, displayed by the short band, indicating skipping of exon 3, and the long band indicating the presence of exon 3. This is shown in lane 7 on the agarose gel picture in (Figure 14).

As c.192G>C U1 snRNA was able to rescue skipping of c.192G>C *DJ-1* mutation carrying exon 3 in the minigene assay in HEK cells, the next step was to test the effect of c.192G>C U1 snRNA in patient-derived cells.

Immortalised fibroblasts of the index patient carrying the c.192G>C *DJ-1* mutation were electroporated with empty vector, wt U1 snRNA or c.192G>C U1 snRNA, respectively. Electroporation with wt U1 snRNA caused a significant increase in *DJ-1* RNA expression of normal RNA and RNA lacking *DJ-1* exon 3 in comparison to electroporation with empty vector or c.192G>C U1 snRNA. c.192G>C U1 snRNA also caused higher RNA levels in comparison to empty vector (Figure 15A). When only looking at correctly spliced *DJ-1* RNA (including exon 3), more RNA expression was seen after electroporation with wt U1 snRNA and even more expression after electroporation with c.192G>C U1 snRNA, compared to electroporation with empty vector (Figure 15B).

**Example 5: Rescue of DJ-1 Protein in patient based cellular models**

The c.192G>C mutation causes mis-splicing of the DJ-1 pre mRNA upon which Exon 3 is spliced out. Although the resulting shorter ΔEx3-mRNA is present in the patient derived small molecule-derived neuronal precursor cells (smNPC), the protein levels of DJ-1 are reduced to an almost undetectable level. The ΔEx3-mRNA is, although stably expressed in the cells, not translated into protein. Even overexpression of a recombinant ΔEx3-mRNA that doesn't undergo splicing does not translate into expression of a truncated ΔEx3-DJ-1 protein. However, overexpression of recombinant full length DJ-1 restores DJ-1 full length mRNA and protein levels in patient derived.

Both constructs (ΔEx3 and full length DJ-1) were delivered to cells by stable transduction using lentiviral vectors. The vectors co-express GFP and population of successfully transduced cells were enriched by FACS sorting for GFP+ cells to an purity of over 80%. (Figure 16).

As the DJ-1 ΔEx3-mRNA does not get translated into protein we aimed to rescue the pathological skipping of exon 3 genetically in the patient-derived cell model. By stably transducing the cells with a specifically designed mutated U1 snRNA (U1mut) that will allow the U1 complex to bind again to the mutant pre-mRNA, the amount of correctly spliced DJ-1 RNA was predicted to increase in the cell model. A lentiviral vector encoding wild type U1 snRNA (U1wt) was used as control to account for effects on protein levels that occur due to general increase of U1 levels in the cells. These lentiviral vectors were co-expressing GFP for later enrichment of transduced cells.

The enriched population of U1 transduced cells indeed showed a rescue of DJ-1 protein only when transduced with the mutant U1 snRNA (Figure 17) The overexpression of wild type U1 was not sufficient to restore DJ-1 protein levels.

Both rescue strategies, expression of DJ-1 full length and U1mut, increase level of full length RNA and protein levels not only in smNPC but also in DIV14 neurons differentiated from smNPC (Figure 18).

The genetic rescue shows that the DJ-1 protein can be successfully restored in patient cells after identification of the underlying mechanism by correcting the splicing defect.

This opens the opportunity for a second strategy to rescue DJ-1 protein level, the use of small chemical compounds. The patient-derived smNPC were used to screen for active compounds. Here, our experiments identified 4-Phenylbutric acid as active in rescuing DJ-1 protein expression in concentrations of 150μM and 300μM as shown by Western blot (Figure 19). Similar tendency of rescue effect was observed in preliminary experiments in which smNPC were treated for 7d with Rectas or with a Rectas analogue called 032.631.702 (Figure 20).

**Example 6: Impaired translation of c.192G>C mutant mRNA causing loss of DJ-1 protein**

Since exon 3 skipping is in frame, a shorter DJ-1 peptide would be expected, that is identical to the wt protein

except for the deleted 34 amino acids encoded by exon 3. Strikingly, no truncated protein can be detected (Fig. 21, Fig. 22 a-b). To exclude the possibility that expression of DJ-1 protein in our patient-derived cell model is impaired by defects of the translation machinery that are unrelated to the mutation itself, we overexpressed wt DJ-1 by lentiviral transduction of patient-derived smNPC. Moreover, we transduced cells with a Δex3 DJ-1 cDNA vector whose transcript requires no splicing and is expected to translate into a truncated protein (Fig. 23c). Overexpression of wt DJ-1 restored protein levels in clones hom2-1 and hom2-4 (Fig. 24a). However, the overexpression of the Δex3 vector failed to increase DJ-1 protein levels (Fig. 24a). In line with this observation, the overexpression of wt DJ-1 resulted in a significant increase of full-length mRNA (Fig. 24b, left graph). Interestingly, although levels of Δex3 mRNA were significantly increased in transduced clones compared to non-transduced clones (Fig. 24b, right graph), truncated protein was still not detected (Fig. 24a). Neurons derived from smNPC transduced with wt DJ-1 also revealed significantly increased DJ-1 levels. DJ-1 levels in neurons derived from Δex3 DJ-1 transduced smNPC were not rescued (Fig. 24c).

Next, we investigated the predicted centroid secondary structure of mRNA, where 47.1% of the bases were predicted to be unpaired for wt DJ-1, while only 40.3% were unpaired for the Δex3 variant. This difference in the number of unpaired bases in the native mRNA structure is also reflected by a slightly higher predicted minimum free energy (-271.1 kcal/mol) as compared to the Δex3 variant (-273.1 kcal/mol). These energy predictions can only provide indicative estimates, but structure visualizations (Fig. 24d and e) pointed to systematic differences between the variants across several bases in one specific local branch, between the bases at position 209 and 486 in the native structure and bases 214 and 381 in the mutant. In this secondary structure branch, the majority of bases in the native structure are unpaired, whereas the corresponding branch in the mutant is dominated by base pairs forming extended stem-loop structures. However, the predicted differences of mRNA structure do not result in inhibition of mRNA transport from the nucleus to the cytoplasm where translation takes place. Northern blot analyses of cytosolic and nuclear fractions of smNPC showed no difference in DJ-1 mRNA levels between control lines and patient-derived cells (Fig. 24f, Fig. 25a). Next we explored, whether impaired Δex3 DJ-1 translation is only related to interference with the eukaryotic translation machinery. When expressed in a prokaryotic organism, Δex3 DJ-1 cDNA also failed to be translated (Fig. 24g). E.coli transformed with either Δex3 DJ-1 or wt DJ-1 cDNA constructs expressed DJ-1 mRNA (Fig. 24g, upper panel), however, only wt DJ-1 mRNA was translated into protein (Fig. 24g, lower panel, Fig. 25b). Together, our results show that the lack of DJ-1 protein in homozygous c.192G>C carriers is not caused by mRNA instability or mis-localization, but rather interference with the translation machinery.

## Example 7: Combination of compounds

We hypothesized a synergistic effect of RECTAS when combined with PB to rescue DJ-1 in c.192G>C carriers. Therefore, DIV 30 neurons differentiated from the two patient-derived smNPC clones were treated for 4 days with 1 mM PB and indicated concentrations of RECTAS. mRNA levels of full-length DJ-1 were measured by qPCR. Compared to the untreated and the solvent control treated neurons correctly spliced full-length DJ-1 mRNA was significantly increased after treatment with 1 mM PB and 50 μM RECTAS. Four days treatment with 1 mM PB and RECTAS concentrations of 10 μM and 25 μM increased the amount of correctly spliced mRNA in a dose-dependent manner (Fig. 26c). Therefore, we chose the concentration of 25 μM and the most effective concentration of 50 μM for longer treatments of 14 days to rescue DJ-1 protein. DJ-1 protein levels increased up to 9.841% and 17.77% upon treatment with 1 mM PB and 25 μM or 50μM RECTAS, respectively, while solvent control treatment showed no increase (Fig. 26d). This rescued DJ-1 protein in patient-derived cells carrying the p.E64D mutation revealed no toxic effect in previous studies. Therefore, we hypothesized that the rescue of E64D DJ-1 in patient-derived cells would also rescue cellular phenotypes. Indeed, after 4 days treatment of hom2-im fibroblasts with PB 1 mM + RECTAS 10 μM or 25 μM we observed a dose dependent increase of DJ-1 protein levels that reached statistical significance at the higher concentration (Fig. 26e). This increase was of physiological relevance as it led to a significant increase of MMP. While untreated and solvent control treated hom2-im fibroblasts showed decreased MMP compared to healthy control C-im it was significantly increased in fibroblasts treated with 1 mM PB + 10 or 25 μM RECTAS (Fig. 26f).

We also tested the effect of RECTAS and PB as a single treatment for rescuing DJ-1 in c.192G>C carriers. The experiments were carried out with patient-derived neurons as described above. As shown in Fig. 27, Rectas alone moderately enhances the portion of correctly processed mRNA, while such an increase can only be seen for PB at a concentration of 1 mM after 8 days of treatment. However, since PB generally enhances expression of DJ-1 mRNA rather than effecting correct splicing, an increase of DJ-1 protein level following PB single treatment, as shown in Fig. 19 may be explained by the overall increase of both correctly and incorrectly processed mRNA.

## Example 8: Treatment of Parkinson's disease associated with aberrant splicing

Here we describe a novel mechanistic concept for the pathogenic role of the PD-associated c.192G>C mutation in *PARK7*. In contrast to a missense mutation that was predicted to cause instability of E64D mutant DJ-1 protein, we

identified a drastic reduction of DJ-1 protein levels in patient-based cellular models due to U1-dependent mis-splicing of pre-mRNA. The demonstration of the disease-causing Δex3 DJ-1 mRNA splice variant as cause of the drastically reduced expression of DJ-1 protein reported here underscores the relevance of access to patient material for functional studies in order to validate predictions and define the underlying molecular pathology as basis for precision medicine approaches.

The observed discrepancy between the overall amount of mutant pre-mRNA and the drastically reduced levels of DJ-1 protein in homozygous c.192G>C carriers indicated the involvement of pathological mRNA processing and/or translation and argued against nonsense-mediated decay (NMD) as underlying mechanism. In our case, the c.192G>C mutation in the DJ-1 gene leads to an in-frame deletion of exon 3 without a PTC and therefore mutant mRNA was predicted to encode a smaller protein lacking the 34 amino acids encoded by exon 3. However, neither mass spectroscopy nor Western blot with different antibodies directed against different epitopes of DJ-1 revealed a truncated protein encoded by Δex3 DJ-1 pre-mRNA (figure 21, 22 Fig. 21b-h, Fig. 22b). Therefore, we speculate that significant changes of the secondary structure related to Δex3 DJ-1 mRNA confer increased stability due to extended stem-loop structures and thus interfere with ribosomal function (Fig. 24d). Indeed similar changes in mRNA secondary structure created by minor shifts of hair pins by 9 base pairs were shown to reduce the translation of the encoded protein more than 50-fold (Babendure, J. R. Control of mammalian translation by mRNA structure near caps. RNA 12, 851-861 (2006)). Moreover, certain stable RNA structures like extended stem loops can stall the ribosome and lead to translational arrest (Doma, M. K. & Parker, R. Endonucleolytic cleavage of eukaryotic mRNAs with stalls in translation elongation. Nature 440, 561-564 (2006)).

In order to prove a U1-mediated pathogenic splicing mechanism underlying loss of DJ-1 function in PD, we performed genetic rescue experiments. This may open avenues for future gene transfer strategies to the central nervous system, as options for viral vectors delivered to the brain are becoming safer and more effective.

Here, we successfully applied an advanced literature mining approach for prioritization of candidate drugs to revert molecular and cellular phenotypes associated with mis-splicing of DJ-1 in homozygous carriers of the c.192G>C mutation. The novel combination of a RECTAS with PB significantly increased mRNA and protein levels of DJ-1 in patient-based cells across different tissues (Fig. 26d and 26e). Interestingly, no complete rescue of protein levels to control level was required for restoring mitochondrial function. This is in line with the high variability of physiological DJ-1 levels observed in cells from healthy individuals, that overlapped with levels of DJ-1 in asymptomatic heterozygous carriers of the c.192G>C mutation (Fig. 22a).

Our findings on the combination of RECTAS and PB as causative treatment for DJ-1 deficiency have an immediate impact for homozygous carriers of the c.192G>C mutation, and may qualify for treatment at the prodromal stage of PD for addressing neuroprotective strategies in PD. As a first drug candidate for correction of aberrant splicing, recently kinetin underwent clinical assessments for pharmacokinetics, safety and effectiveness *in vivo* (Axelrod, F. B. et al. Kinetin improves IKBKAP mRNA splicing in patients with familial dysautonomia. Pediatr. Res. 70, 480-3 (2011); Shetty, R. S. et al. Specific correction of a splice defect in brain by nutritional supplementation. Hum. Mol. Genet. 20, 4093-101 (2011)). Within a clinical trial kinetin was well-tolerated, safe, and resulted in increased levels of correctly spliced mRNA *in vivo.* This indicates that drugs targeting U1-mediated splicing defects have a high potential to become a therapeutic tool and supports future clinical trials.

The therapeutic potential of combined RECTAS and PB treatment for targeting defective splicing is further substantiated by additional mutations in monogenic PD. Homozygous mutations in *PARK7* affecting the 5' consensus splice site for U1 were described as a c.91-2AG mutation in an Iranian family and a c.317-322del mutation in a Turkish family. Moreover, a novel c. 1488+1 G>A mutation in the PINK1 gene was shown to affect a U1 binding site and cause an in-frame deletion of exon 7 (Samaranch, L. et al. PINK1-linked parkinsonism is associated with Lewy body pathology. Brain 133, 1128-42 (2010)). Interestingly, as for the c.192G>C mutation in *PARK7*, no NMD of the mutant PINK1 mRNA was observed in affected carriers.

These findings already indicate that the pathogenic relevance of exonic splicing mutations may be underestimated in PD, as it is known to represent a common cause for human diseases with approximately 15% of all mutations caused by aberrant spliceosome function, that translates into defective pre-mRNA processing (Nissim-Rafinia, M. & Kerem, B. Splicing regulation as a potential genetic modifier. Trends Genet. 18, 123-7 (2002)). To further assess the potential role of splicing-related mutations in PD, we performed an exploratory analysis of exonic mutations affecting U1-mediated splicing using publicly available databases including whole exome sequencing results from the PPMI study (Parkinson Progression Marker Initiative. The Parkinson Progression Marker Initiative (PPMI). Prog. Neurobiol. 95, 629-35 (2011).). We found a significantly higher burden of rare exonic variants affecting U1 snRNA binding sites in 380 PD patients compared to 162 control samples (n=109, P-value=0.015, OR=1.55, CI=1.05-2.29), with the majority of the burden defined by brain expressed genes (P-value=0.047, OR=1.50, CI=0.95-2.38). These results are in line with a recent study showing that approximately 10% of pathogenic missense variants predicted to alter protein code are essentially disrupting splicing (Soemedi, R. et al. Pathogenic variants that alter protein code often disrupt splicing. Nat. Genet. (2017). doi:10.1038/ng.3837). This supports our findings of an enrichment of missense and synonymous disease-associated variants in the exon boundary and underscores the therapeutic potential of compounds acting on pathological splicing.

Our study illustrates the promise for treatment concepts in precision medicine in PD that focus on genetic and molecular stratification. In order to account for the increasingly recognized heterogeneity in PD and other neurodegenerative disorders, new strategies have to be developed for the stratification of patients along shared pathogenic mechanisms.

Variant annotation and filtering:

In the current study we only focused on the intronic 5' splice single nucleotide variants (SNVs). Multi-allellic variants were decomposed by using variant-tests (Tan, A., Abecasis, G. R. & Kang, H. M. Unified representation of genetic variants. Bioinformatics 31, 2202-2204 (2015)) and left normalized by bcftools (Li, H. et al. The Sequence Alignment/Map format and SAMtools. Bioinformatics 25, 2078-2079 (2009)). Variants were annotated by using ANNOVAR (Wang, K., Li, M. & Hakonarson, H. ANNOVAR: functional annotation of genetic variants from high-throughput sequencing data. Nucleic Acids Res. 38, e164-e164 (2010)) version 2016December05 using RefSeq gene annotations and the dbNSFP v3.0 (Liu, X., Wu, C., Li, C. & Boerwinkle, E. dbNSFP v3.0: A One-Stop Database of Functional Predictions and Annotations for Human Nonsynonymous and Splice-Site SNVs. Hum. Mutat. 37, 235-241 (2016)) prediction scores. Only rare variants, as defined by variants with a MAF < 0.05 in the current dataset and also European population of public databases such as 1000 genomes (The 1000 Genomes Project Consortium. A global reference for human genetic variation. Nature 526, 68-74 (2015)), ExAC (release 0.3), and the Exome variant server (http://evs.gs.washington.edu/EVS/) were selected. In order to prioritize the 5' splice variants based on their deleteriousness, we used three different scores. The first score is generated by using the MaxEntScan method (Yeo, G. & Burge, C. B. Maximum entropy modeling of short sequence motifs with applications to RNA splicing signals. J. Comput. Biol. J. Comput. Mol. Cell Biol. 11, 377-394 (2004).) which is based on the maximum entropy principle. The other two scores were ensemble scores (dbscSNV_ADA and dbscSNV_RF) generated from multiple splice site prediction tools (Jian, X., Boerwinkle, E. & Liu, X. In silico prediction of splice-altering single nucleotide variants in the human genome. Nucleic Acids Res. 42, 13534-13544 (2014)) which are available as part of dbNSFP database (Liu, X., Wu, C., Li, C. & Boerwinkle, E. dbNSFP v3.0: A One-Stop Database of Functional Predictions and Annotations for Human Nonsynonymous and Splice-Site SNVs. Hum. Mutat. 37, 235-241 (2016)).

Generation of MaxEntScan score:

In the current study we only focused on the variants present in the consensus splice site positions i.e., +3 to -6 from the exon/intron boundary. To prioritize such variants using MaxEntScan method, for each SNV that lies in the consensus splice site region ($SNV_{splice}$ ) a wild type 9 mer (WT) was extracted from the reference genome (hg19). Then the variant was introduced within the WT by using the python module pyfaidx (Shirley, M. D., Ma, Z., Pedersen, B. S. & Wheelan, S. J. Efficient 'pythonic' access to FASTA files using pyfaidx. (PeerJ PrePrints, 2015).), hence creating a mutated consensus splice site (MUT) sequence for each variant. In the next steps the scores were calculated for both WT and MUT sequences by using the scripts provided in the MaxEntScan website (http://genes.mit.edu/burgelab/maxent/Xmaxentscan_scoreseq.html). The relative percentage change (maxentscan_change) was calculated by using the formula below and all the variant sites were annotated with the $WT_{score}$ and maxentscan_change.

$$\text{maxentscan_change} = \left(\frac{\text{WTscore} - \text{MUTscore}}{\text{WTscore}}\right) * 100$$

Deleterious splice site variants ($SNV_{splicedel}$) were defined as SNVs with the following criteria: $WT_{score}$ >5 and maxentscan_change >70 and dbscSNV_ADA score >0.9 and dbscSNV_RF score >0.9. If the ensemble scores are not available for any particular variant only MaxEntScan methods ($WT_{score}$ >5 and maxentscan change >70) was used.

Burden tests:

To test the genome-wide differential burden of $SNV_{splicedel}$ variants in cases compared to the controls CMCFisherExact test available part of rvtests (Zhan, X., Hu, Y., Li, B., Abecasis, G. R. & Liu, D. J. RVTESTS: an efficient and comprehensive tool for rare variant association analysis using sequence data. Bioinformatics 32, 1423-1426 (2016)) with default parameters was used and one sided P-values were reported. CMCFisherExact test collapses the variants in the given regions (gene or gene-set) and performs the fisher exact test. We used a list of brain expressed genes to see if there is an increased burden in the brain expressed genes (n= 14,177) compared to the non-brain expressed genes (n=6,428).

Results:

Burden analysis:

After the filtering based on ethnicity, cryptic relatedness and quality parameters the final dataset comprised of 380 PD and 162 control samples. A total of 128 $SNV_{splicedel}$ variants were included in the final analysis. We observed a genome-wide significant burden in cases compared to the controls (P-value= 0.021, OR=1.49, CI=1.02-2.18). The signal is coming mainly from the exonic $SNV_{splicedel}$ variants (n=109, P-value= 0.015, OR=1.55, CI= 1.05-2.29) rather than the intronic ones (n=19, P-value=0.24, OR=1.34, CI=0.68-2.65). In the exonic $SNV_{splicedel}$variants majority of the burden is caused by the brain expressed genes (P-value 0.047, OR=1.50, CI=0.95-2.38), compared to the genes that are not expressed in brain (P-value 0.12, OR=1.37, CI=0.84-2.23). The $SNV_{splicedel}$ variants are provided in the supplemental table 1, along with their respective case-control counts.

Research co-funded by the Fonds National de la Recherche Luxembourg (FNR) under grant number FNR/P13/6682797/Krüger

Other embodiments are within the following claims. In addition, where features or aspects of the invention are described in terms of Markush groups, those skilled in the art will recognize that the invention is also thereby described in terms of any individual member or subgroup of members of the Markush group.

**Claims**

**1.** An in vitro method for screening a compound for Parkinson’s disease treatment, comprising

(a) contacting a cell having a DJ-1 gene containing a c.192G>C mutation with a compound of interest;
(b) testing whether said compound of interest prevents skipping of exon 3 of said DJ-1 gene, thereby identifying said compound as candidate for Parkinson’s disease treatment.

**2.** The method of claim 1, wherein

(a) said cell has a c. 192G>C mutation in at least one allele of the DJ-1 gene;
(b) said cell has a c.192G>C mutation in both alleles of the DJ-1 gene; or
(c) said c.192G>C mutation leads to a p.E64D amino acid exchange in the DJ-1 protein.

**3.** The method of claim 1, wherein said cell is a mammalian cell, preferably a human cell line, wherein said cell preferably contains a DJ-1 gene having a c.192G>C mutation.

**4.** The method of any one of the preceding claims, wherein

(a) said cell is a primary fibroblast cell, an immortalized fibroblast cell, an induced pluripotent stem cell (iPSC), a small-molecule-derived neuronal precursor cell (smNPC), or a midbrain-specific dopaminergic neuron (mDA) from a patient having at least one c. 192G>C mutation in a DJ-1 gene;
(b) the DJ-1 gene containing a c.192G>C is fused to a reporter gene; or
(c) said testing in step (b) is accomplished by western blot, qPCR, mass spectrometry, detection of reporter gene activity, microscopy-based assay, or FACS-based assay

**5.** A compound which prevents skipping of exon 3 of the DJ-1 gene for use in a method of treatment of Parkinson's disease, wherein

(a) the treatment comprises prevention of skipping of exon 3 of the DJ-1 gene in a patient; or
(b) the treatment is of a patient having a c.192G>C mutation in the DJ-1 gene in at least one allele; or
(c) the Parkinson's disease is associated with a c.G192C mutation in the PARK7 gene.

**6.** The compound for the use of claim 5, which is obtainable by the method of any one of claims 1 to 4.

**7.** The compound for the use of claim 5, which is a mutated U1 snRNA allowing the U1 complex to bind to the mutant pre-mRNA, wherein said mutated U1 snRNA preferably has the nucleotide sequence shown in SEQ ID NO: 7.

**8.** The compound for the use of claim 5, which is an optionally substituted C1-C10 alkyl carboxylic acid.

**9.** The compound for the use of claim 8, which is butyric acid.

**10.** The compound for the use of claim 8, which comprises at least one aryl and/or at least one heteroaryl substituent, which is preferably a compound having the structural formula (I):

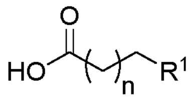

(I),

wherein $R^1$ is optionally substituted aryl or optionally substituted heteroaryl; and
n is an integer of from 0 to 8,
wherein the compound is preferably 4-phenyl butyric acid.

**11.** The compound for the use of claim 5, which is a compound having the structural formula (II):

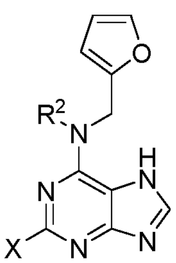

(II),

wherein $R^2$ is hydrogen or optionally substituted alkyl; and
X is hydrogen or halogen,
wherein the compound is preferably N6-furfuryladenine, 2-chloro-N-[(furan-2-yl)methyl]-7H-purin-6-amine, or 2-chloro-N-[(furan-2-yl)methyl]-N-methyl-7H-purin-6-amine.

**12.** A pharmaceutical composition comprising one or more compounds which prevents skipping of exon 3 of the DJ-1 gene for use in a method of treatment of Parkinson's disease, wherein

(a) the treatment comprises prevention of skipping of exon 3 of the DJ-1 gene in a patient; or
(b) the treatment is of a patient having a c.192G>C mutation in the DJ-1 gene in at least one allele; or
(c) the Parkinson's disease is associated with a c.G192C mutation in the PARK7 gene.

**13.** The pharmaceutical composition for the use of claim 12, wherein the one or more compound comprises a compound as defined in any one of claims 5 to 11.

**14.** The pharmaceutical composition for the use of claim 12 or 13, wherein

(a) the composition comprises at least two compounds as defined in any one of the claims 5 to 11;

(b) the composition comprises a compound as defined in any one of claims 8 to 10 and a compound as defined in claim 11; or
(c) the composition comprises 4-phenyl butyric acid and 2-chloro-N-[(furan-2-yl)methyl]-7H-purin-6-amine.

**15.** The method, compound or pharmaceutical composition of any one of the preceding claims, wherein the Parkinson's disease is early onset Parkinson's disease, idiopathic Parkinson's Disease, Parkinson's disease associated with aberrant splicing, Parkinson's disease associated with a U1-dependent splicing defect, PARK7-associated Parkinson's disease, or Parkinson's disease associated with a c.G192C mutation in the PARK7 gene.

**Patentansprüche**

**1.** *In vitro*-Verfahren zum Durchmustern einer Verbindung für die Behandlung von Parkinson-Krankheit, umfassend

a) Inkontaktbringen einer Zelle, die ein DJ-1-Gen hat, die eine c.192G>C-Mutation enthält mit einer zu testenden Verbindung;
(b) Testen ob die zu testende Verbindung das *Skipping* von Exon 3 auf dem DJ-3-Gen verhindert, wodurch die Verbindung als Kandidat für die Behandlung der Parkinson-Krankheit identifiziert wird.

**2.** Verfahren nach Anspruch 1, wobei

(a) die Zelle eine c.192G>C-Mutation in mindestens einem Allel des DJ-1-Gens hat;
(b) die Zelle eine c. 192G>C-Mutation in beiden Allelen des DJ-1-Gens hat; oder
(c) die c.192G>C-Mutation zu einem p.E64D-Aminosäureaustausch in dem DJ-1-Protein führt.

**3.** Verfahren nach Anspruch 1, wobei die Zelle eine Säugetierzelle, vorzugsweise eine menschliche Zellline ist, wobei die Zelle vorzugsweise ein DJ-1-Gen enthält, das eine c.192G>C-Mutation hat.

**4.** Verfahren nach einem der vorangehenden Ansprüche, wobei

(a) die Zelle eine primäre Fibroblastenzelle, eine immortalisierte Fibroblastenzelle, eine induzierte pluripotente Stammzelle (iPSC), eine durch kleine Moleküle erhaltene neuronale Vorläuferzelle (*small-molecule-derived neuronal precursor cell*, smNPC) oder ein mittelhirnspezifisches dopaminerges Neuron (mDA) von einem Patienten ist, der mindestens eine c.192G>C-Mutation in einem DJ-1-Gen hat;
(b) das DJ-1-Gen, welches ein c.192G>C enthält, mit einem Reportergen fusioniert ist; oder
(c) das Testen in Schritt (b) durch Western Blot, qPCR, Massenspektrometrie, Detektion von Reportergenaktivität, mikroskopbasiertem Test oder FACSbasiertem Test erfolgt.

**5.** Verbindung, die ein Skipping von Exon 3 auf dem DJ-1-Gen verhindert, zur Verwendung in einem Verfahren zur Behandlung von Parkinson-Krankheit, wobei

(a) die Behandlung das Verhindern des *Skppings* von Exon 3 des DJ-1-Gens in einem Patienten umfasst;
(b) die Behandlung, die eines Patienten ist, der eine c.192G>C-Mutation in dem DJ-1-Gen in mindestens einem Allel hat;
(c) die Parkinson-Krankheit mit einer c.G192C-Mutation in dem PARK7-Gen assoziiert ist.

**6.** Verbindung zur Verwendung nach Anspruch 5, die erhältlich ist durch das Verfahren nach einem der Ansprüche 1 bis 4.

**7.** Verbindung zur Verwendung nach Anspruch 5, die eine mutierte U1 snRNA ist, die das Binden des U1-Komplexes an die mutierte prä-mRNA ermöglicht, wobei die mutierte U1 snRNA vorzugsweise die Nukleotidsequenz hat, die in SEQ ID NO: 7 gezeigt ist.

**8.** Verbindung zur Verwendung nach Anspruch 5, die eine optional substituierte C1-C10-Alkylcarbonsäure ist.

**9.** Verbindung zur Verwendung nach Anspruch 8, die Buttersäure ist.

**10.** Verbindung zur Verwendung nach Anspruch 8, die mindestens einen Aryl- und/oder mindestens einen Heteroaryl-Substituenten umfasst, die vorzugsweise eine Verbindung ist, die die Strukturformel (I) hat:

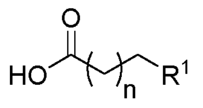

(I),

wobei $R^1$ ein optional substituiertes Aryl oder ein optional substituiertes Heteroaryl ist; und
n eine ganze Zahl von 0 bis 8 ist,
wobei die Verbindung vorzugsweise 4-Phenylbuttersäure ist.

**11.** Verbindung zur Verwendung nach Anspruch 5, die eine Verbindung ist, die die Strukturformel (II) hat:

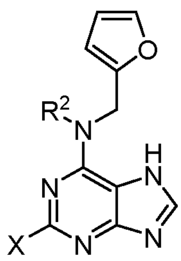

(II),

wobei $R^2$ Wasserstoff oder ein optional substituiertes Alkyl ist; und
X Wasserstoff oder ein Halogen ist,
wobei die Verbindung vorzugsweise N6-Furfuryladenin, 2-Chlor-N-[(furan-2-yl)methyl]-7H-purin-6-amin oder 2-Chlor-N-[(furan-2-yl)methyl]-N-methyl-7H-purin-6-amin ist.

**12.** Pharmazeutische Zusammensetzung umfassend eine oder mehrere Verbindung(en), die ein Skipping von Exon 3 auf dem DJ-1-Gen verhindert, zur Verwendung in einem Verfahren zur Behandlung von Parkinson-Krankheit, wobei

(a) die Behandlung das Verhindern des *Skppings* von Exon 3 des DJ-1-Gens in einem Patienten umfasst;
(b) die Behandlung, die eines Patienten ist, der eine c.192G>C-Mutation in dem DJ-1-Gen in mindestens einem Allel hat;
(c) die Parkinson-Krankheit mit einer c.G192C-Mutation in dem PARK7-Gen assoziiert ist.

**13.** Pharmazeutische Zusammensetzung zur Verwendung nach Anspruch 12, wobei die eine oder mehrere Verbindung(en) eine Verbindung umfasst, wie sie in einem der Ansprüche 5 bis 11 definiert ist.

**14.** Pharmazeutische Zusammensetzung zur Verwendung nach Anspruch 12 oder 13, wobei

(a) die Zusammensetzung mindestens zwei Verbindungen umfasst, wie sie in einem der Ansprüche 5 bis 11 definiert ist;
(b) die Zusammensetzung eine Verbindung umfasst, wie sie in einem der Ansprüche 8 bis 10 definiert ist, und eine Verbindung, wie sie in Anspruch 11 definiert ist; oder
(c) die Zusammensetzung 4-Phenylbuttersäure und 2-Chlor-N-[(furan-2-yl)methyl]-7H-purin-6-amin umfasst.

**15.** Verfahren, Verbindung oder pharmazeutische Zusammensetzung nach einem der vorangehenden Ansprüche, wobei die Parkinson-Krankheit eine früh einsetzende Parkinson-Krankheit, eine idiopathische Parkinson-Krankheit, eine mit aberrantem Spleißen assoziierte Parkinson-Krankheit, eine mit einem U1-abhängigen Spleißdefekt assoziierte Parkinson-Krankheit, eine PARK7-assoziierte Parkinson-Krankheit oder eine mit einer c.G192C-Mutation im PARK7-Gen assoziierte Parkinson-Krankheit ist.

## Revendications

**1.** Procédé in vitro pour le criblage d'un composé pour le traitement de la maladie de Parkinson, comprenant les opérations suivantes:

(a) mettre en contact une cellule ayant un gène DJ-1 contenant une mutation c.192G>C avec un composé d'intérêt ;
(b) tester si ledit composé d'intérêt empêche le saut de l'exon 3 dudit gène DJ-1, identifiant ainsi ledit composé comme candidat pour le traitement de la maladie de Parkinson.

**2.** Procédé selon la revendication 1, dans lequel

(a) ladite cellule présente une mutation c.192G>C dans au moins un allèle du gène DJ-1;
(b) ladite cellule présente une mutation c.192G>C dans les deux allèles du gène DJ-1; ou
(c) ladite mutation c.192G>C conduit à un échange d'acides aminés p.E64D dans la protéine DJ-1.

**3.** Procédé selon la revendication 1, dans lequel ladite cellule est une cellule de mammifère, de préférence une lignée cellulaire humaine, dans laquelle ladite cellule contient de préférence un gène DJ-1 ayant une mutation c.192G>C.

**4.** Procédé selon l'une des revendications précédentes, dans lequel

(a) ladite cellule est une cellule fibroblastique primaire, une cellule fibroblastique immortalisée, une cellule souche pluripotente induite (iPSC), une cellule pré-curseur neuronale dérivée d'une petite molécule (smNPC) ou un neurone dopa-minergique spécifique du cerveau moyen (mDA) provenant d'un patient ayant au moins une mutation c.192G>C dans un gène DJ-1;
(b) le gène DJ-1 contenant un c. 192G>C est fusionné à un gène rapporteur; ou
(c) lesdits essais de l'étape (b) sont réalisés par western blot, qPCR, spectrométrie de masse, détection de l'activité du gène rapporteur, essai au microscope ou essai FACS

**5.** Composé qui empêche le saut de l'exon 3 du gène DJ-1 pour utilisation dans un procédé de traitement de la maladie de Parkinson, dans lequel

(a) le traitement comprend la prévention du saut de l'exon 3 du gène DJ-1 chez un patient; ou
(b) le traitement est celui d'un patient présentant une mutation c. 192G>C du gène DJ-1 dans au moins un allèle; ou
(c) la maladie de Parkinson est associée à une mutation c.G192C dans le gène PARK7.

**6.** Composé pour utilisation selon la revendication 5, qui peut être obtenu par le procédé selon l'une des revendications 1 à 4.

**7.** Composé pour utilisation selon la revendication 5, qui est un snARN U1 muté permettant au complexe U1 de se lier au pré-ARNm mutant, dans lequel ledit snARN U1 muté a de préférence la séquence nucléotidique indiquée dans la SEQ ID NO: 7.

**8.** Composé pour utilisation selon la revendication 5, qui est un acide alkylcarboxylique en C1-C10 éventuellement substitué.

**9.** Composé pour utilisation selon la revendication 8, qui est l'acide butyrique.

**10.** Composé pour utilisation selon la revendication 8, qui comprend au moins un substituant aryle et/ou au moins un substituant hétéroaryle, qui est de préférence un composé présentant la formule développée (I):

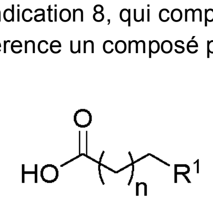

(I),

dans laquelle R1 est un aryle éventuellement substitué ou un hétéroaryle éventuellement substitué ; et
n est un nombre entier compris entre 0 et 8,
où le composé est de préférence l'acide 4-phénylbutyrique.

**11.** Composé pour utilisation selon la revendication 5, qui est un composé présentant la formule développée (II):

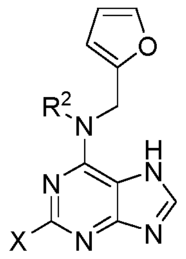 (II),

dans laquelle $R^2$ est un atome d'hydrogène ou un groupe alkyle éventuellement substitué; et
X est l'hydrogène ou un halogène ;
dans laquelle le composé est de préférence la N6-furfuryladénine, la 2-chloro-N-[(furan-2-yl)méthyl]-7H-purin-6-amine ou la 2-chloro-N-[(furan-2-yl)méthyl]-N-méthyl-7H-purin-6-amine.

**12.** Composition pharmaceutique comprenant un ou plusieurs composés qui empêchent le saut de l'exon 3 du gène DJ-1, pour utilisation dans un procédé traitement de la maladie de Parkinson, dans laquelle

(a) le traitement comprend la prévention du saut de l'exon 3 du gène DJ-1 chez un patient; ou
(b) le traitement est celui d'un patient présentant une mutation c. 192G>C du gène DJ-1 dans au moins un allèle; ou
(c) la maladie de Parkinson est associée à une mutation c.G192C dans le gène PARK7.

**13.** Composition pharmaceutique pour utilisation selon la revendication 12, dans laquelle le ou les composés comprennent un composé tel que défini dans l'une quelconque des revendications 5 à 11.

**14.** Composition pharmaceutique pour utilisation selon les revendications 12 ou 13, dans laquelle

(a) la composition comprend au moins deux composés tels que définis dans l'une quelconque des revendications 5 à 11;
(b) la composition comprend un composé tel que défini dans l'une quelconque des revendications 8 à 10 et un composé tel que défini dans la revendication 11; ou
(c) la composition comprend de l'acide 4-phénylbutyrique et de la 2-chloro-N-[(furan-2-yl)méthyl]-7H-purin-6-amine.

**15.** Procédé, composé ou composition pharmaceutique selon l'une quelconque des revendications précédentes, dans lesquels la maladie de Parkinson est la maladie de Parkinson à début précoce, la maladie de Parkinson idiopathique, la maladie de Parkinson associée à un épissage aberrant, la maladie de Parkinson associée à un défaut d'épissage dépendant de U1, la maladie de Parkinson associée à PARK7, ou la maladie de Parkinson associée à une mutation c.G192C dans le gène PARK7.

**Figure 1**

PARK7 (DJ-1)wt coding region (cDNA)
ATGGCTTCCAAAAGAGCTCTGGTCATCCTGGCTAAAGGAGCAGAGGAAATGGAGACGGTCATCCC
TGTAGATGTCATGAGGCGAGCTGGG*ATTAAGGTCACCGTTGCAGGCCTGGCTGGAAAAGACCCAG*
*TACAGTGTAGCCGTGATGTGGTCATTTGTCCTGATGCCAGCCTTGAAGATGCAAAAAAAGAG*GGA
CCATATGATGTGGTGGTTCTACCAGGAGGTAATCTGGGCGCACAGAATTTATCTGAG**TCTGCTGC**
**TGTGAAGGAGATACTGAAGGAGCAGGAAAACCGGAAGGGCCTGATAGCCGCCATCTGTGCAG***GTC*
*CTACTGCTCTGTTGGCTCATGAAATAGGTTTTGGAAGTAAAGTTACAACACACCCTCTTGCTAAA*
*GACAAAATGATGAATGGAG****GTCATTACACCTACTCTGAGAATCGTGTGGAAAAAGACGGCCTGAT***
***TCTTACAAGCCGGGGGCCTGGGACCAGCTTCGAGTTTGCGCTTGCAATTGTTGAAGCCCTGAATG***
***GCAAGGAGGTGGCGGCTCAAGTGAAGGCTCCACTTGTTCTTAAAGACTAG***
(SEQ ID NO: 1)

PARK7 (DJ-1)c.192G>C coding region (cDNA)
ATGGCTTCCAAAAGAGCTCTGGTCATCCTGGCTAAAGGAGCAGAGGAAATGGAGACGGTCATCCC
TGTAGATGTCATGAGGCGAGCTGGG*ATTAAGGTCACCGTTGCAGGCCTGGCTGGAAAAGACCCAG*
*TACAGTGTAGCCGTGATGTGGTCATTTGTCCTGATGCCAGCCTTGAAGATGCAAAAAAAGAC*GGA
CCATATGATGTGGTGGTTCTACCAGGAGGTAATCTGGGCGCACAGAATTTATCTGAG**TCTGCTGC**
**TGTGAAGGAGATACTGAAGGAGCAGGAAAACCGGAAGGGCCTGATAGCCGCCATCTGTGCAG***GTC*
*CTACTGCTCTGTTGGCTCATGAAATAGGTTTTGGAAGTAAAGTTACAACACACCCTCTTGCTAAA*
*GACAAAATGATGAATGGAG****GTCATTACACCTACTCTGAGAATCGTGTGGAAAAAGACGGCCTGAT***
***TCTTACAAGCCGGGGGCCTGGGACCAGCTTCGAGTTTGCGCTTGCAATTGTTGAAGCCCTGAATG***
***GCAAGGAGGTGGCGGCTCAAGTGAAGGCTCCACTTGTTCTTAAAGACTAG***
(SEQ ID NO: 2)

Exon2
*Exon3*
Exon4
**Exon5**
*Exon6*
***Exon7***

**Figure 2**

DJ-1 wt-Protein
MASKRALVILAKGAEEMETVIPVDVMRRAGIKVTVAGLAGKDPVQCSRDVVICPDASLEDAKKEG
PYDVVVLPGGNLGAQNLSESAAVKEILKEQENRKGLIAAICAGPTALLAHEIGFGSKVTTHPLAK
DKMMNGGHYTYSENRVEKDGLILTSRGPGTSFEFALAIVEALNGKEVAAQVKAPLVLKD*
(SEQ ID NO: 5)

DJ-1 E64D-Protein
MASKRALVILAKGAEEMETVIPVDVMRRAGIKVTVAGLAGKDPVQCSRDVVICPDASLEDAKKDG
PYDVVVLPGGNLGAQNLSESAAVKEILKEQENRKGLIAAICAGPTALLAHEIGFGSKVTTHPLAK
DKMMNGGHYTYSENRVEKDGLILTSRGPGTSFEFALAIVEALNGKEVAAQVKAPLVLKD*
(SEQ ID NO: 6)

**Figure 3**

Mutated U1 snRNA
Atacttacgtggcaggggagataccatgatcacgaaggtggttttcccagggcgaggcttatcca
ttgcactccggatgtgctgacccctgcgatttccccaaatgtgggaaactcgactgcataatttg
tggtagtgggggactgcgttcgcgctttcccctgactttctgga*gtttcaaaagtagactg*
(SEQ ID NO: 7)

Figure 4 A

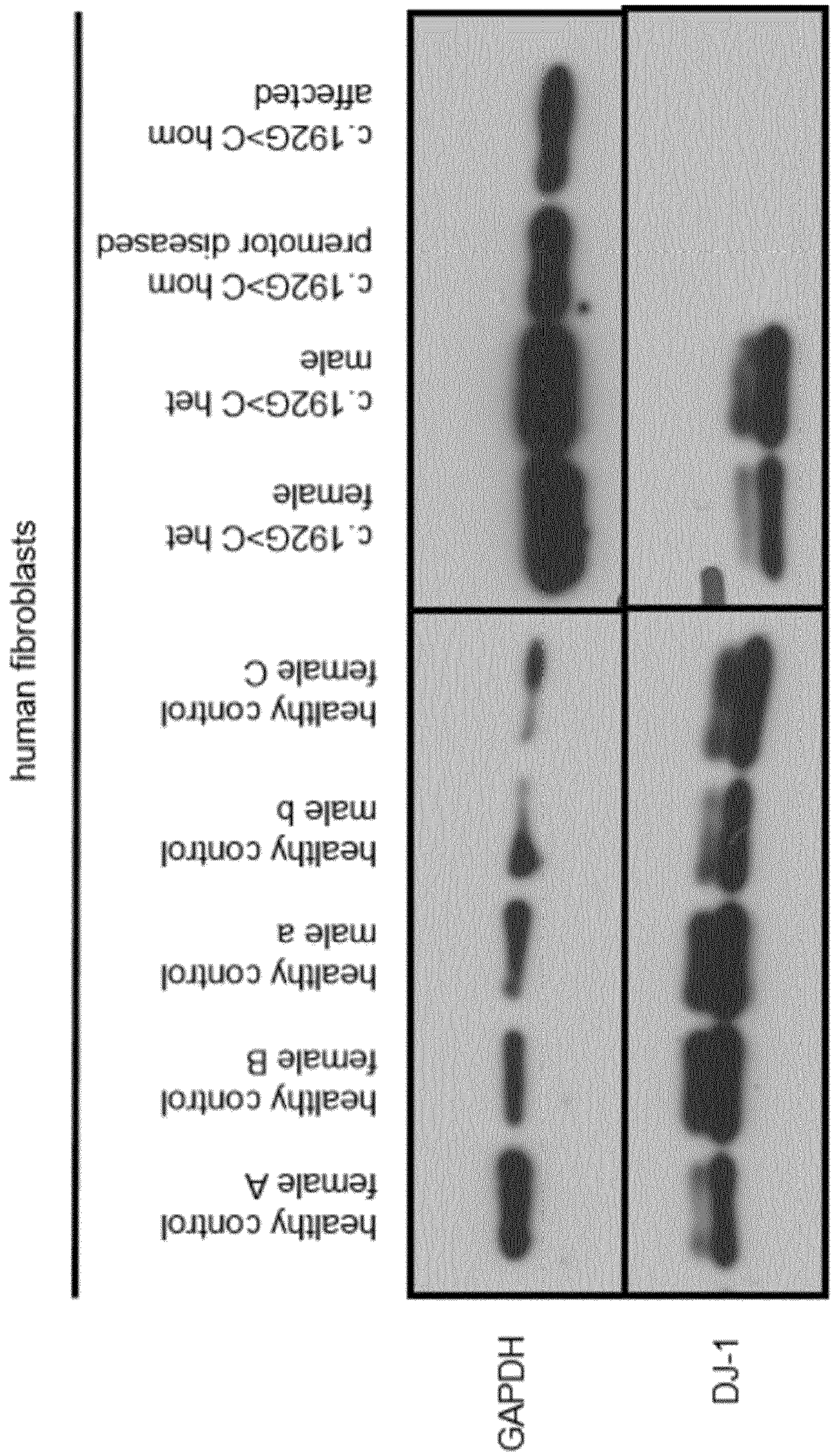
human fibroblasts
healthy control female A
healthy control female B
healthy control male a
healthy control male b
healthy control female C
c.192G>C het female
c.192G>C het male
c.192G>C hom premotor diseased
c.192G>C hom affected
GAPDH
DJ-1

**Figure 4 B**

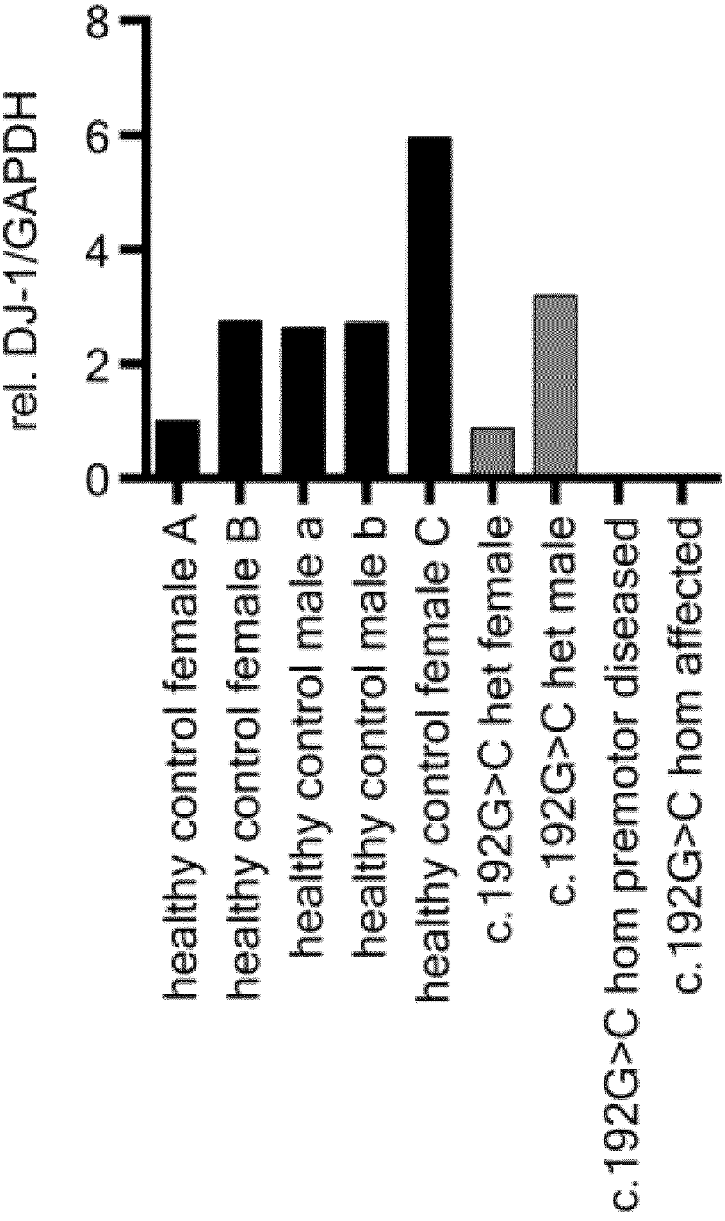
rel. DJ-1/GAPDH
0
2
4
6
8
healthy control female A
healthy control female B
healthy control male a
healthy control male b
healthy control female C
c.192G>C het female
c.192G>C het male
c.192G>C hom premotor diseased
c.192G>C hom affected

**Figure 5**

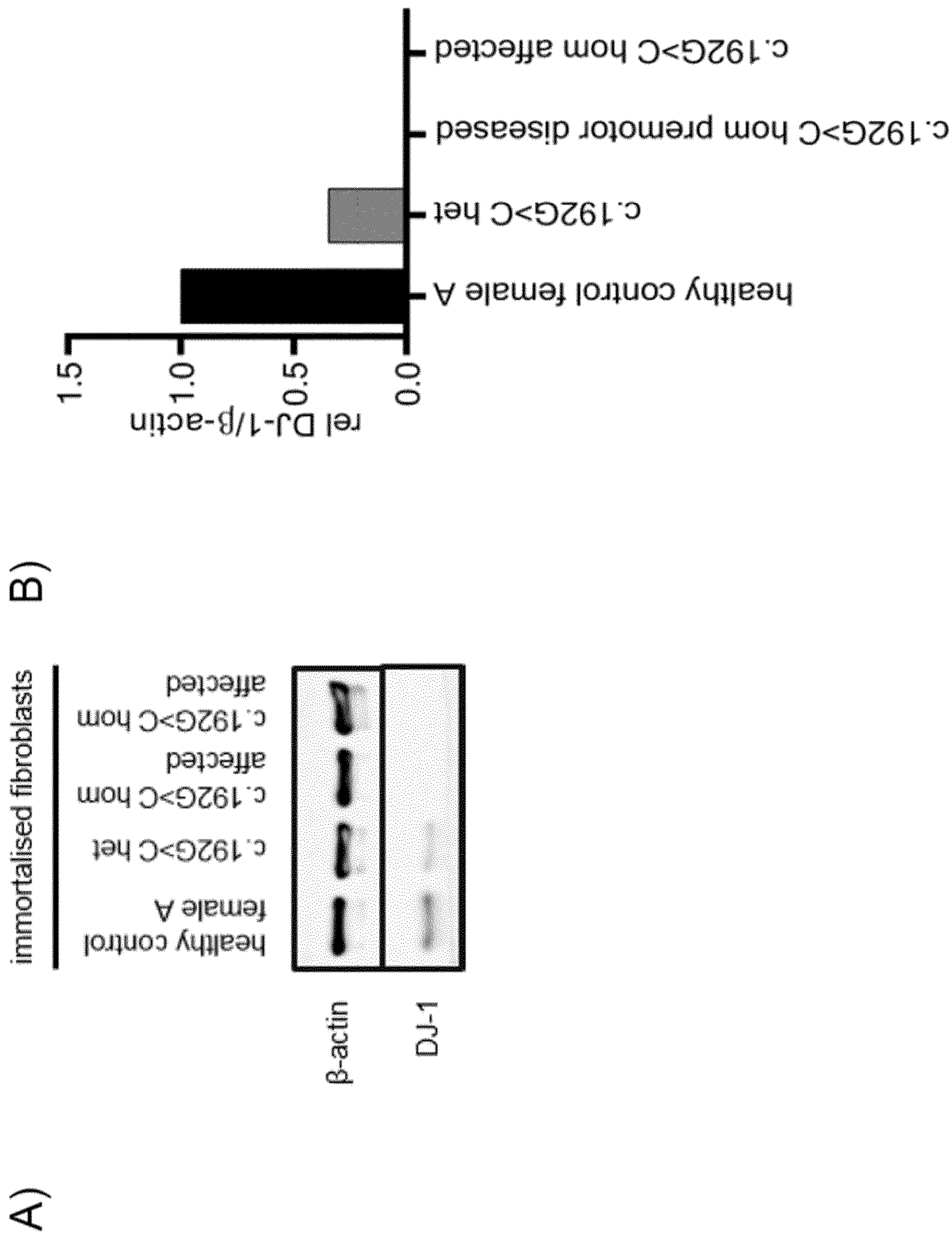
A)
immortalised fibroblasts
healthy control female A
c.192G>C het
c.192G>C hom affected
c.192G>C hom affected
β-actin
DJ-1
B)
rel DJ-1/β-actin
1.5
1.0
0.5
0.0
healthy control female A
c.192G>C het
c.192G>C hom premotor diseased
c.192G>C hom affected

**Figure 6**

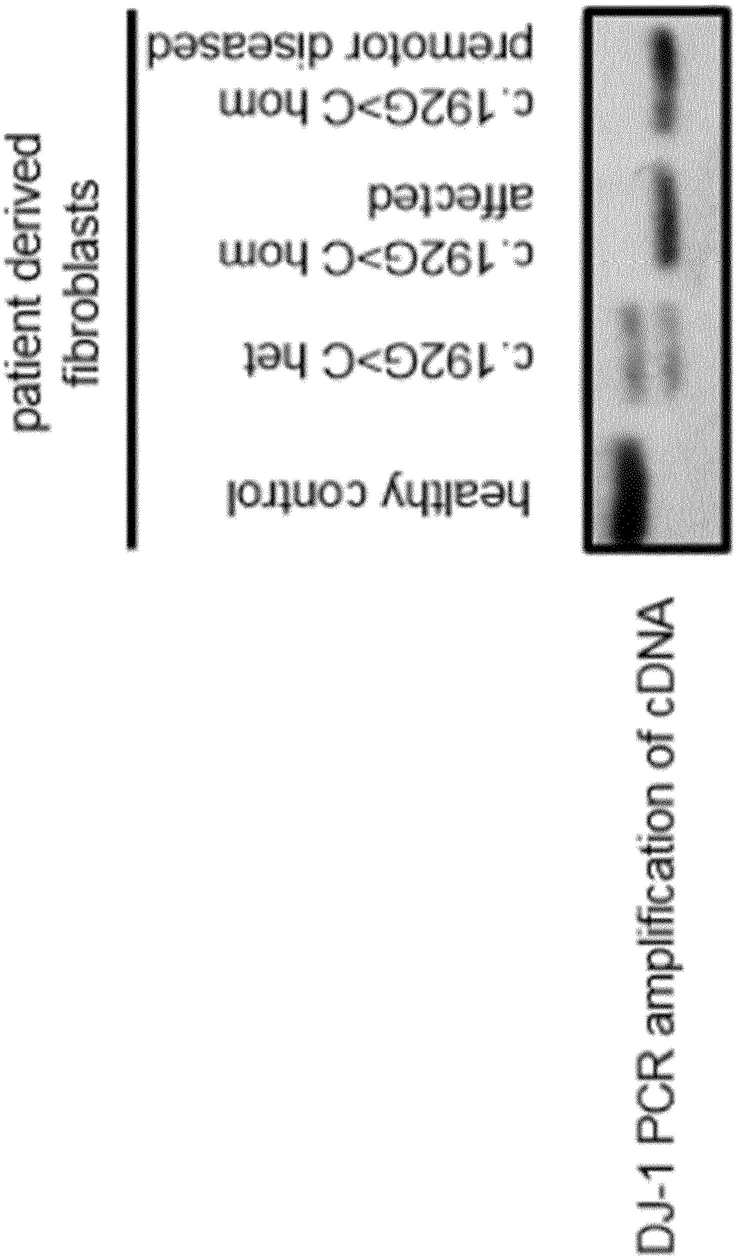
patient derived fibroblasts
healthy control
c.192G>C het
c.192G>C hom affected
c.192G>C hom premotor diseased
DJ-1 PCR amplification of cDNA

Figure 7

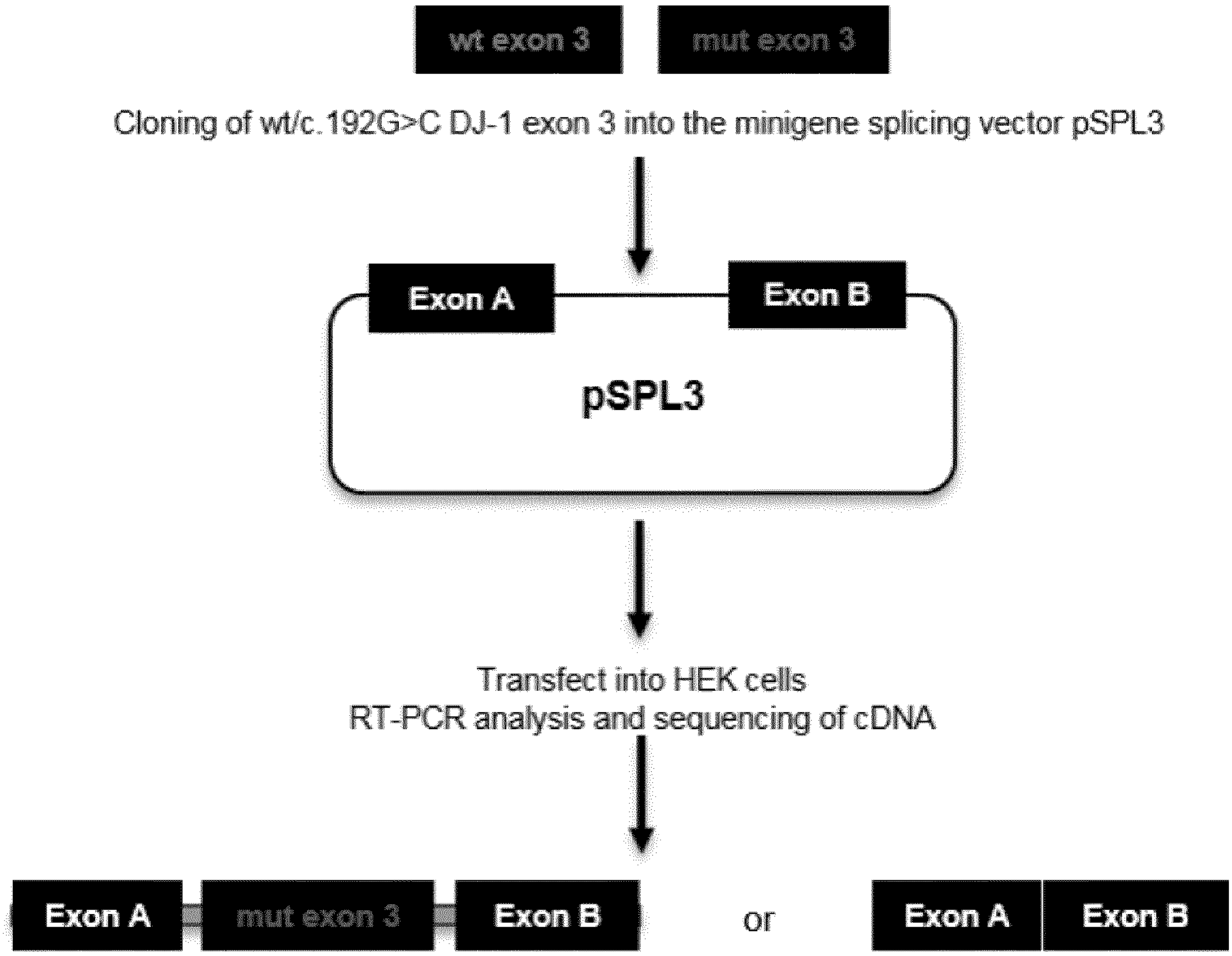
wt exon 3
mut exon 3
Cloning of wt/c.192G>C DJ-1 exon 3 into the minigene splicing vector pSPL3
Exon A
Exon B
pSPL3
Transfect into HEK cells
RT-PCR analysis and sequencing of cDNA
Exon A
mut exon 3
Exon B
or
Exon A
Exon B

Figure 8

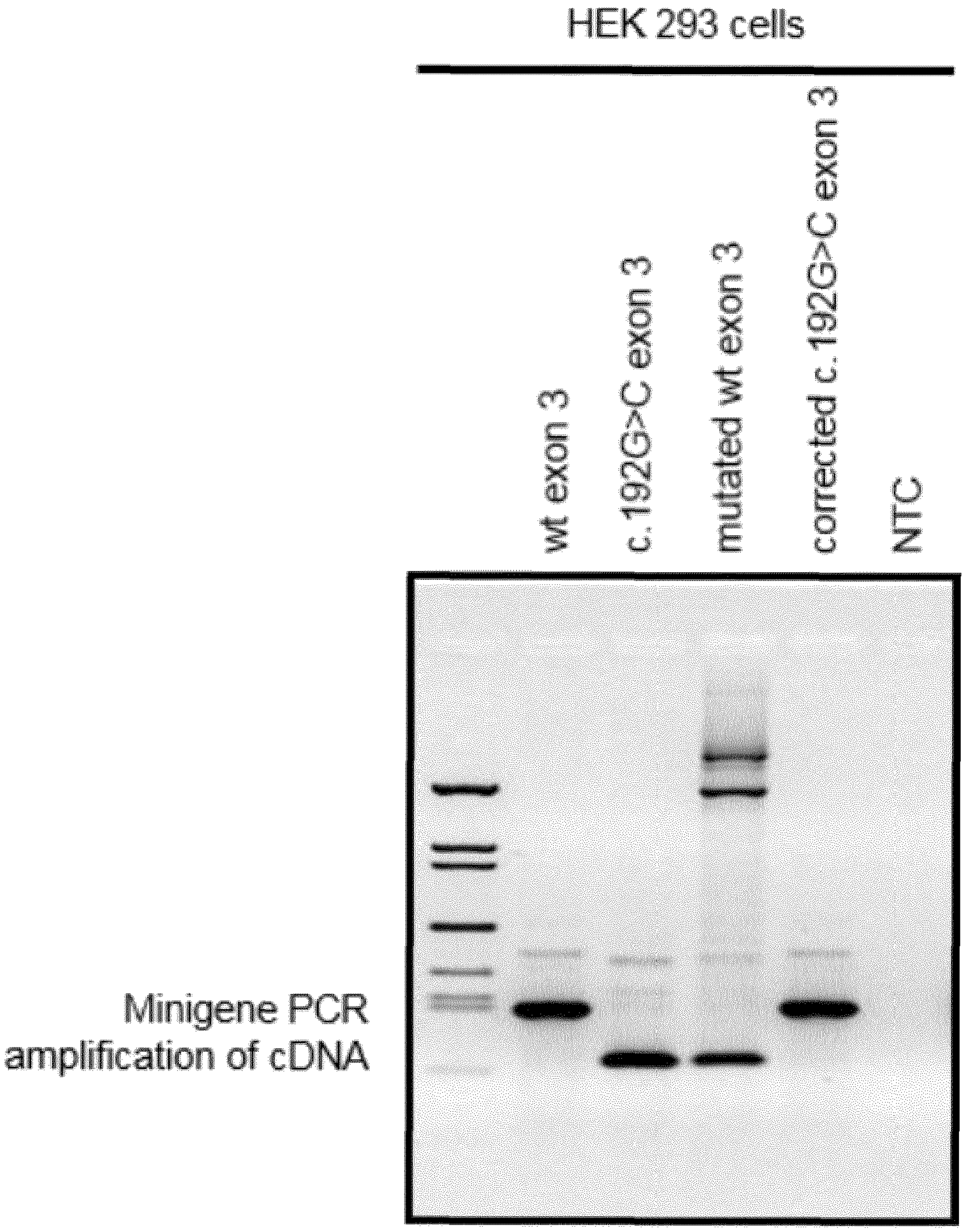
HEK 293 cells
wt exon 3
c.192G>C exon 3
mutated wt exon 3
corrected c.192G>C exon 3
NTC
Minigene PCR
amplification of cDNA

**Figure 9**

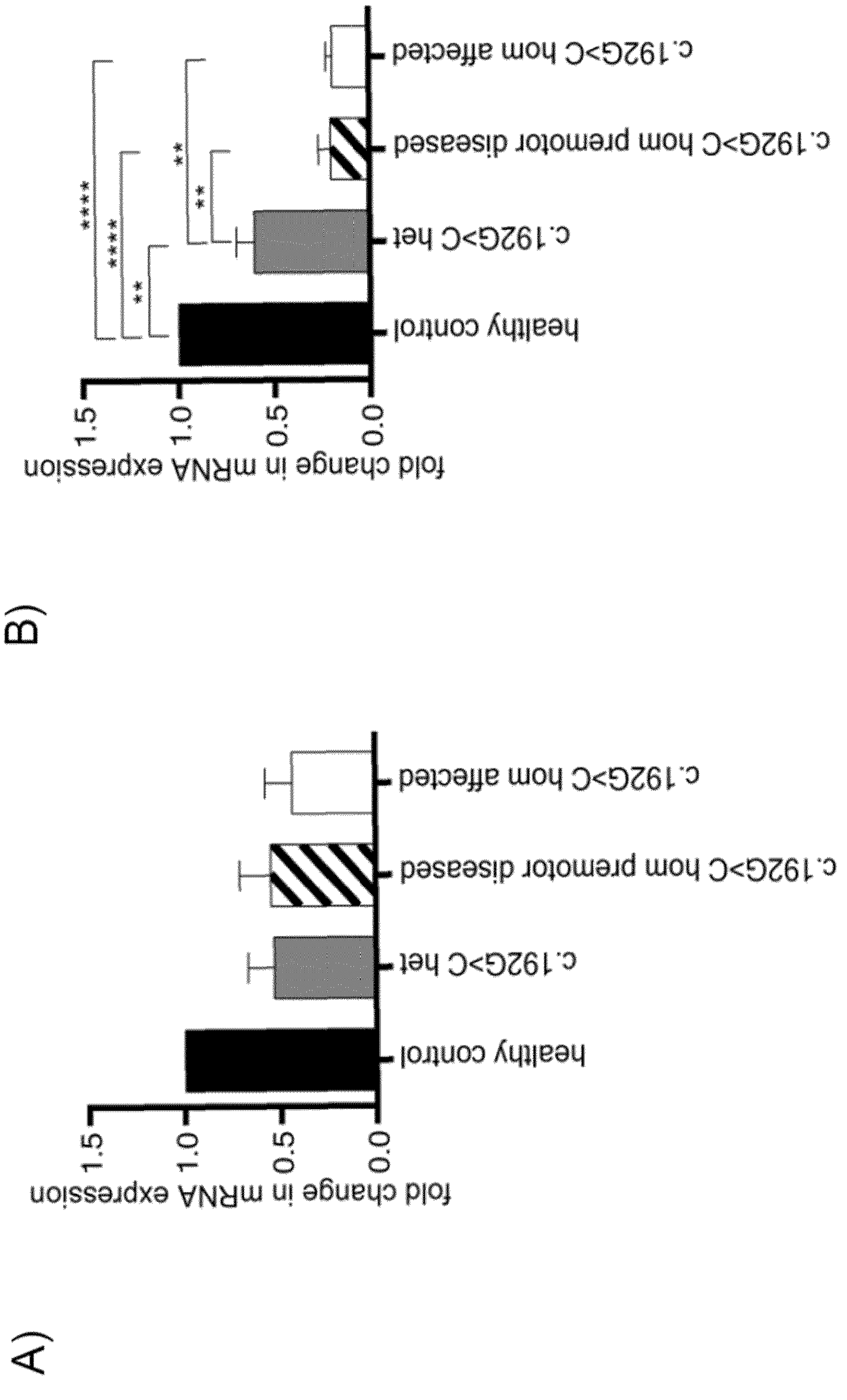
A)
B)
fold change in mRNA expression
1.5
1.0
0.5
0.0
healthy control
c.192G>C het
c.192G>C hom premotor diseased
c.192G>C hom affected
**
****
****
**
**

**Figure 10**

A)

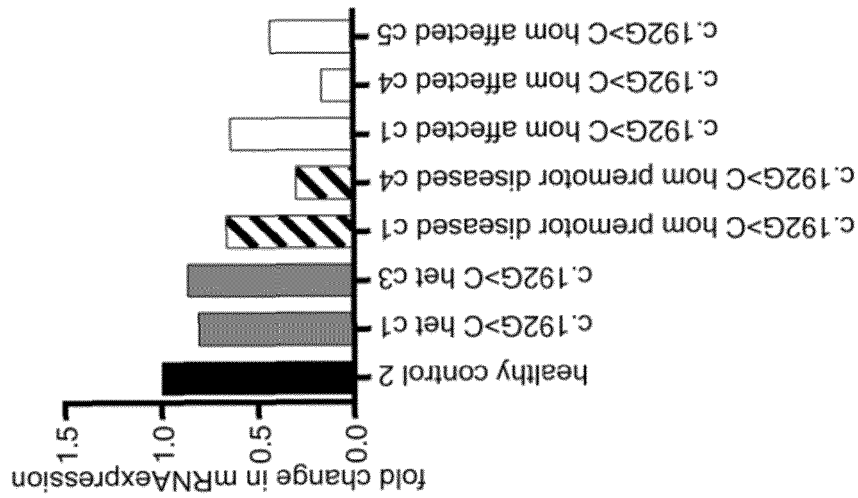
fold change in mRNAexpression
0.0
0.5
1.0
1.5
healthy control 2
c.192G>C het c1
c.192G>C het c3
c.192G>C hom premotor diseased c1
c.192G>C hom premotor diseased c4
c.192G>C hom affected c1
c.192G>C hom affected c4
c.192G>C hom affected c5

B)

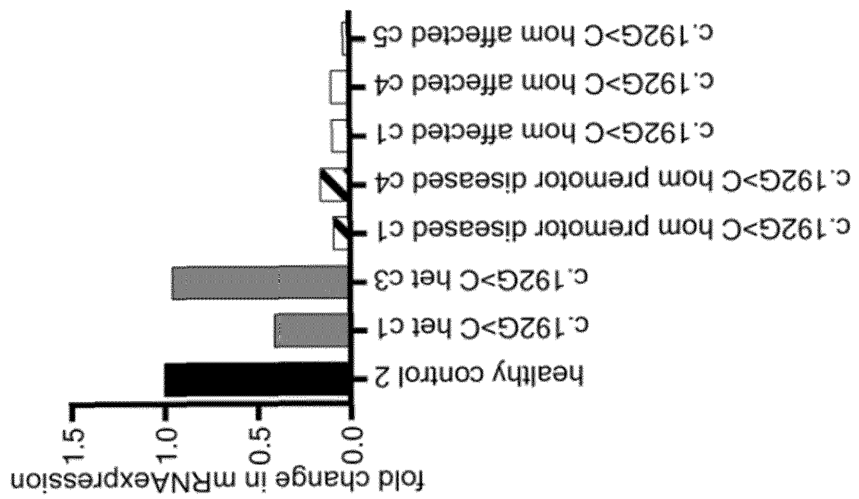
fold change in mRNAexpression
0.0
0.5
1.0
1.5
healthy control 2
c.192G>C het c1
c.192G>C het c3
c.192G>C hom premotor diseased c1
c.192G>C hom premotor diseased c4
c.192G>C hom affected c1
c.192G>C hom affected c4
c.192G>C hom affected c5

**Figure 11**

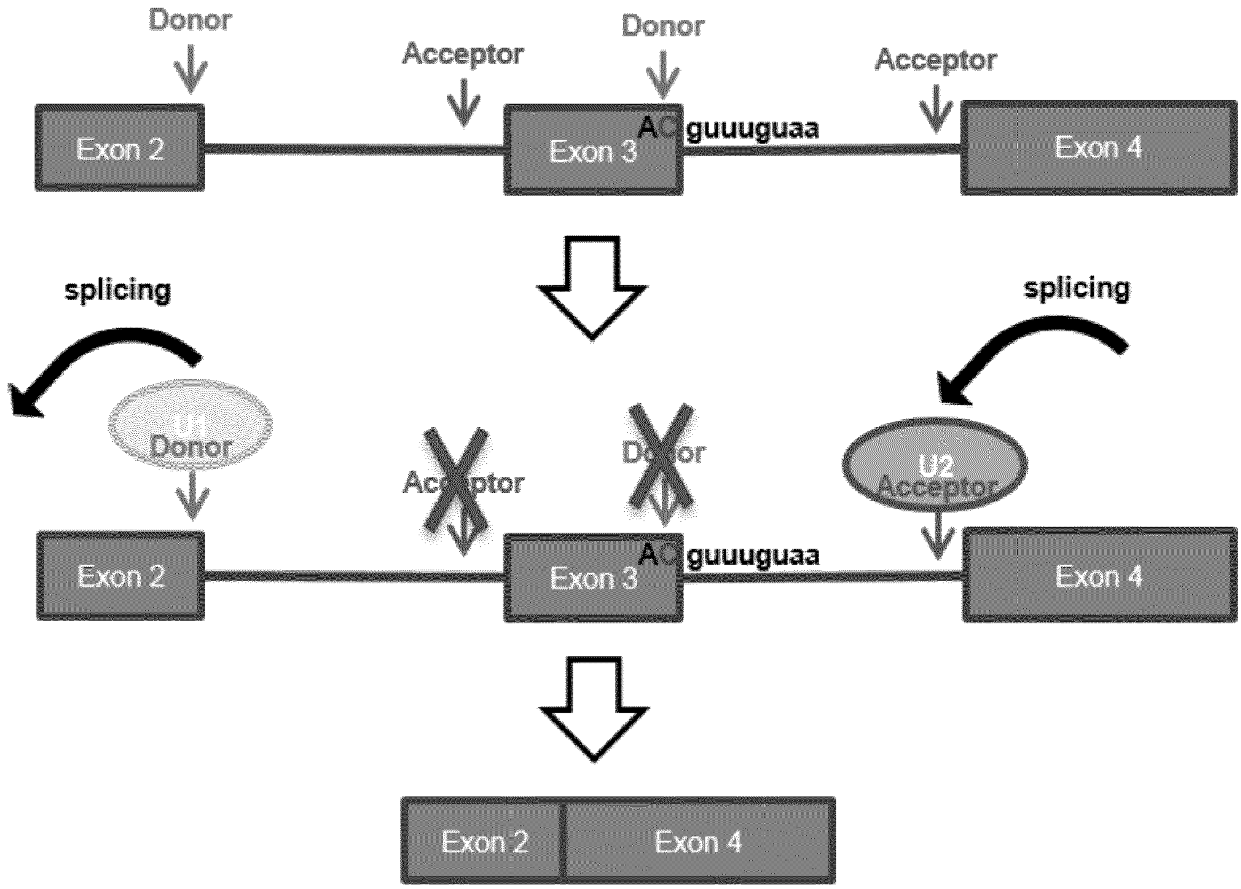
Donor
Acceptor
Donor
Acceptor
Exon 2
Exon 3
AC guuuguaa
Exon 4
splicing
splicing
U1
Donor
Acceptor
Donor
U2
Acceptor
Exon 2
Exon 3
AC guuuguaa
Exon 4
Exon 2
Exon 4

**Figure 12**

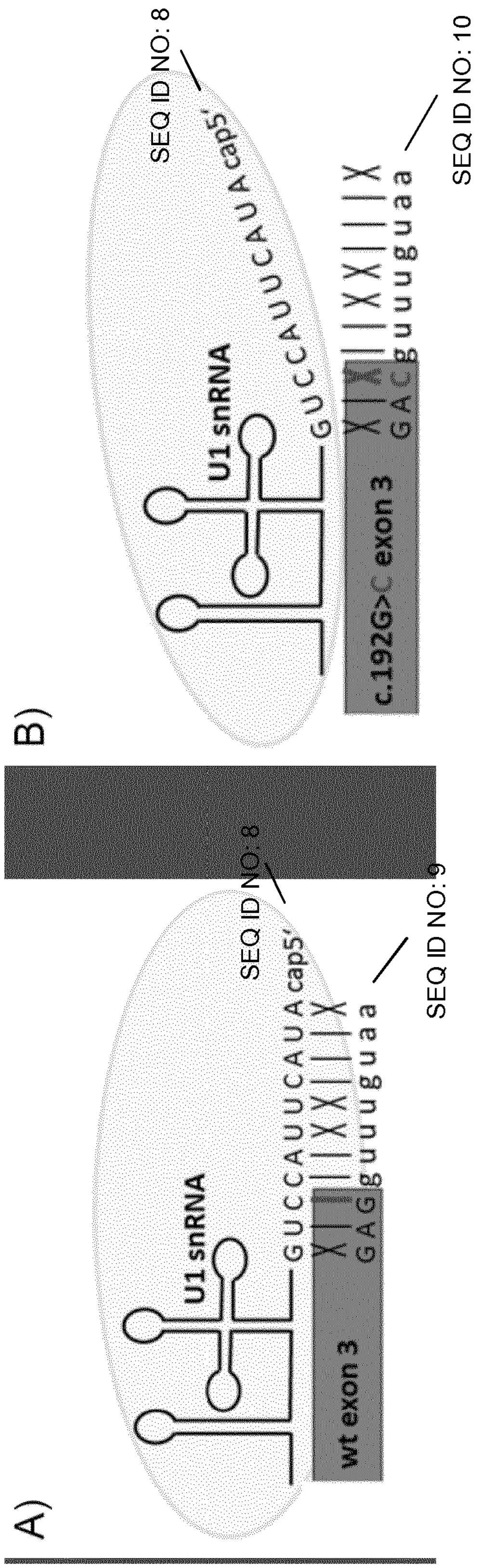
A)
U1 snRNA
GUCCAUUCAUA cap5'
SEQ ID NO: 8
wt exon 3
GAGguuuguaa
SEQ ID NO: 9
B)
U1 snRNA
GUCCAUUCAUA cap5'
SEQ ID NO: 8
c.192G>C exon 3
GACguuuguaa
SEQ ID NO: 10

Figure 13

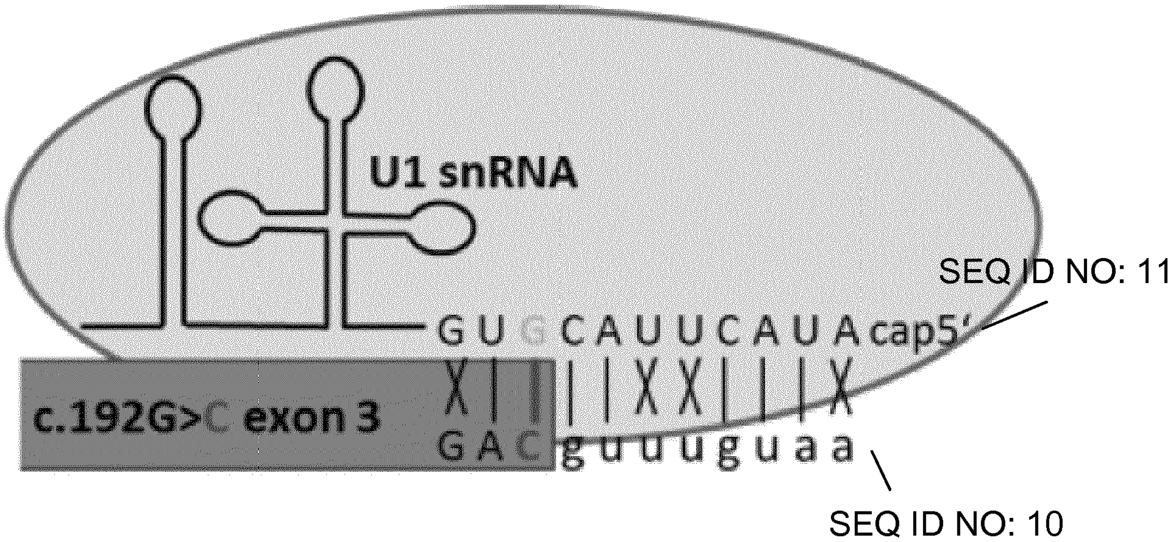
U1 snRNA
SEQ ID NO: 11
G U G C A U U C A U A cap5'
c.192G>C exon 3
G A C g u u u g u a a
SEQ ID NO: 10

Figure 14

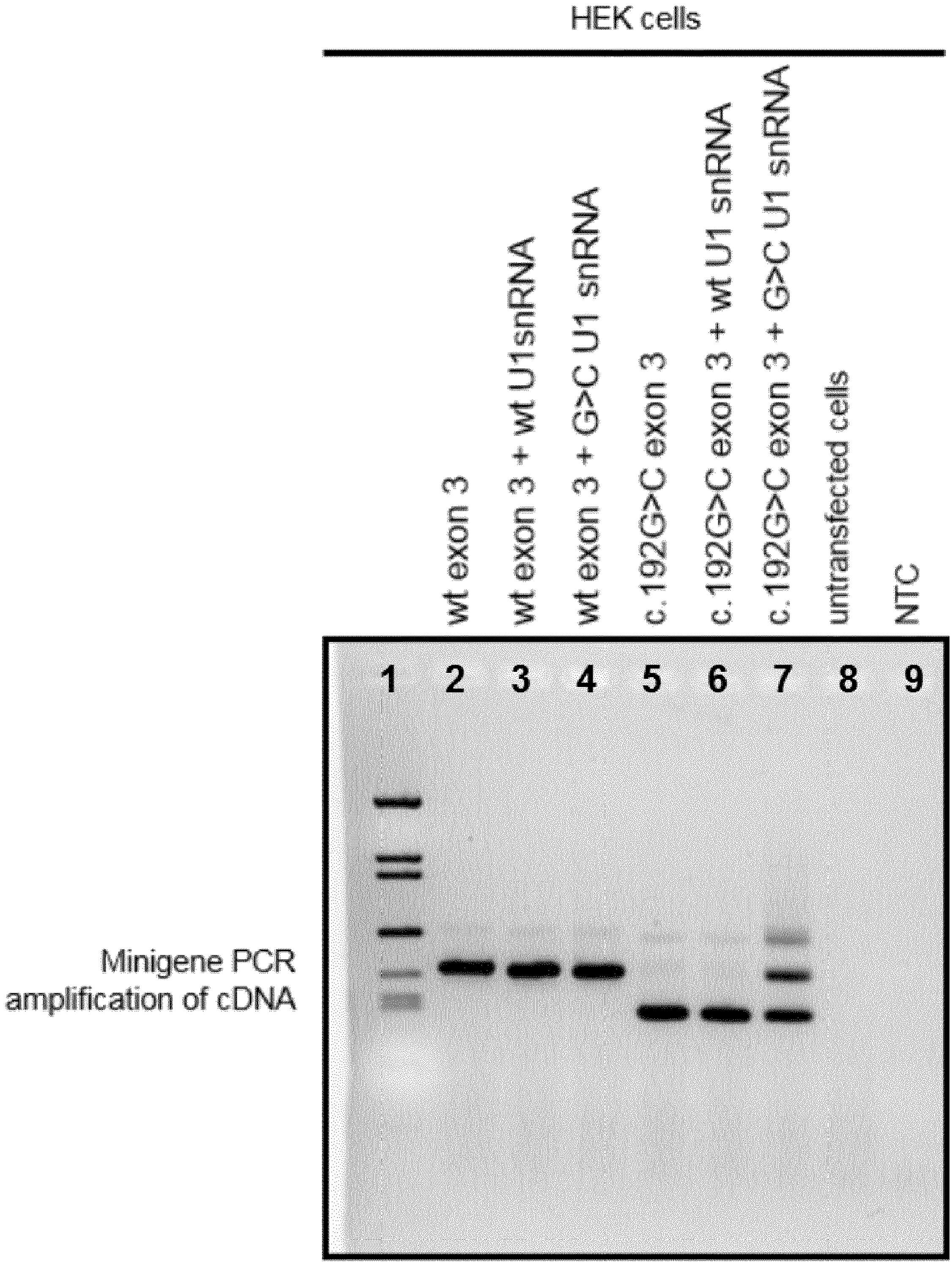
HEK cells
wt exon 3
wt exon 3 + wt U1snRNA
wt exon 3 + G>C U1 snRNA
c.192G>C exon 3
c.192G>C exon 3 + wt U1 snRNA
c.192G>C exon 3 + G>C U1 snRNA
untransfected cells
NTC
1 2 3 4 5 6 7 8 9
Minigene PCR amplification of cDNA

Figure 15

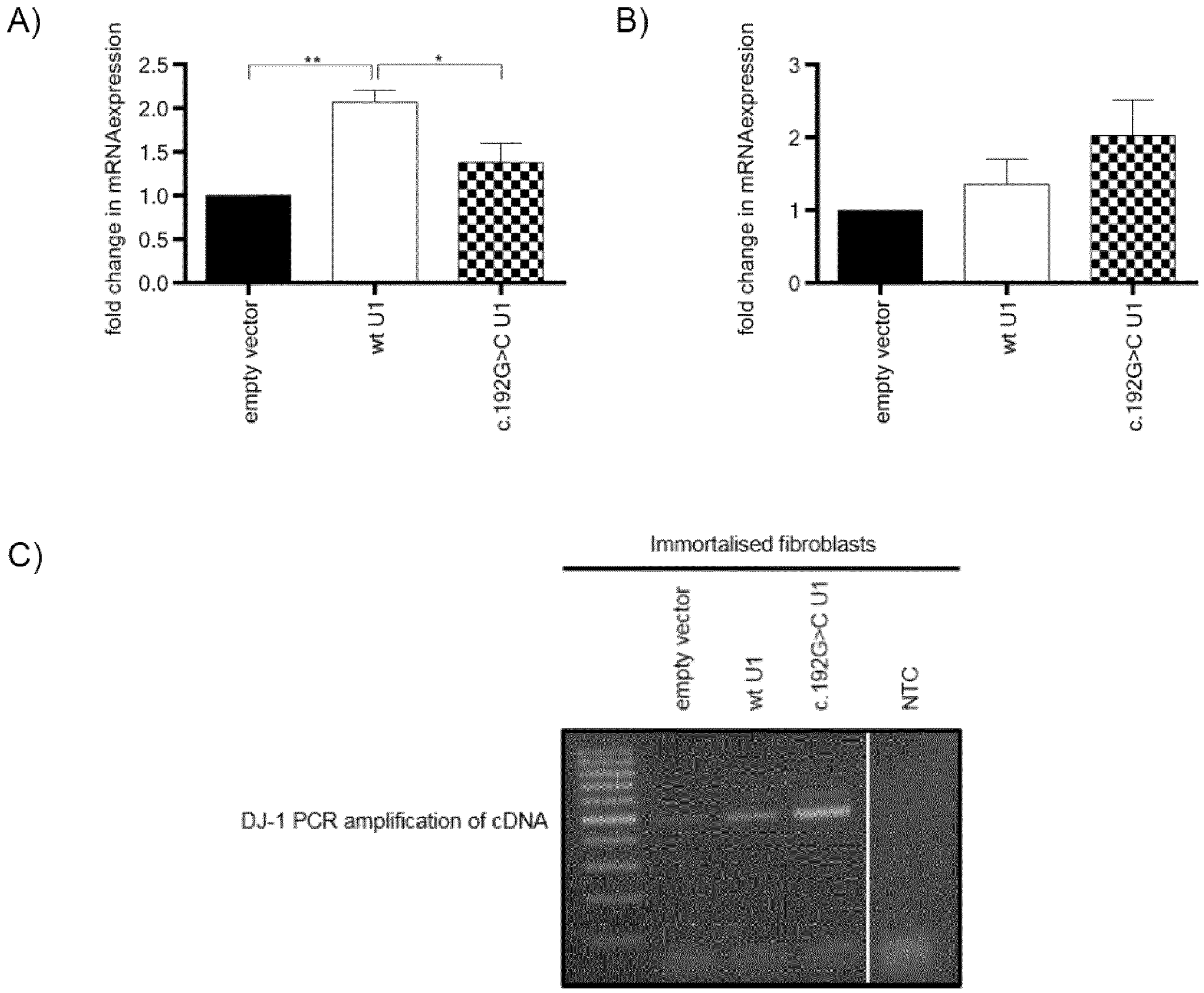
A)
fold change in mRNAexpression
2.5
2.0
1.5
1.0
0.5
0.0
**
*
empty vector
wt U1
c.192G>C U1
B)
fold change in mRNAexpression
3
2
1
0
empty vector
wt U1
c.192G>C U1
C)
Immortalised fibroblasts
empty vector
wt U1
c.192G>C U1
NTC
DJ-1 PCR amplification of cDNA

Figure 16 A

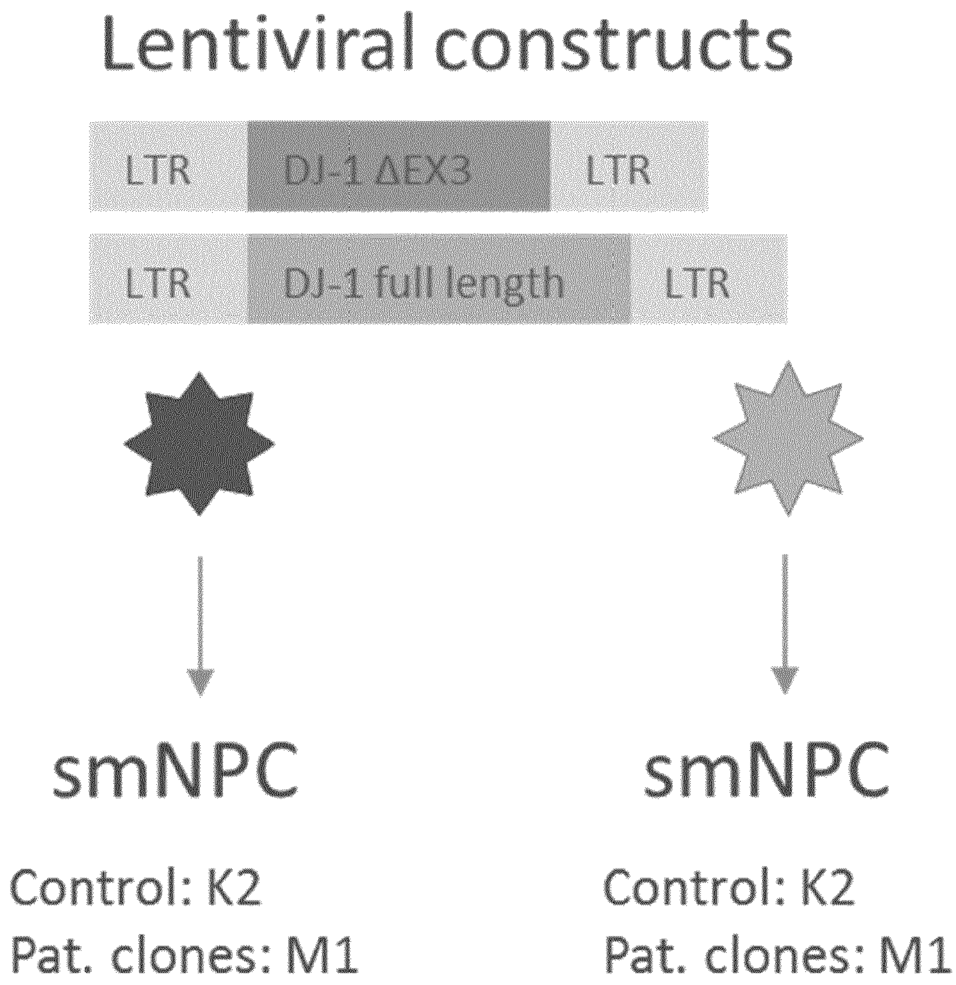
Lentiviral constructs
LTR
DJ-1 ΔEX3
LTR
LTR
DJ-1 full length
LTR
smNPC
Control: K2
Pat. clones: M1
smNPC
Control: K2
Pat. clones: M1

**Figure 16 B**

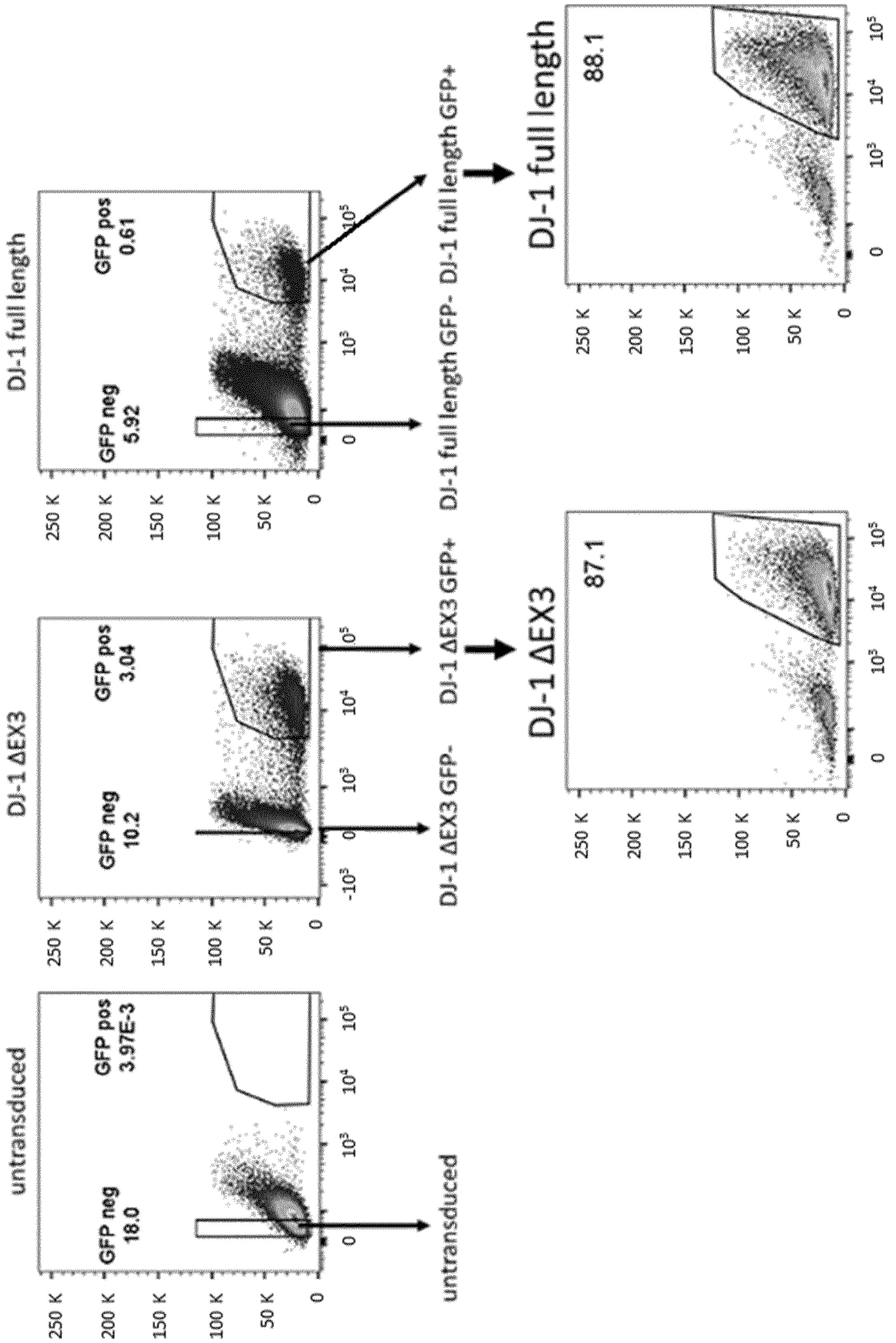
untransduced
GFP neg
18.0
GFP pos
3.97E-3
untransduced
DJ-1 ΔEX3
GFP neg
10.2
GFP pos
3.04
DJ-1 ΔEX3 GFP-
DJ-1 ΔEX3 GFP+
DJ-1 full length
GFP neg
5.92
GFP pos
0.61
DJ-1 full length GFP-
DJ-1 full length GFP+
DJ-1 ΔEX3
87.1
DJ-1 full length
88.1

**Figure 16 C**

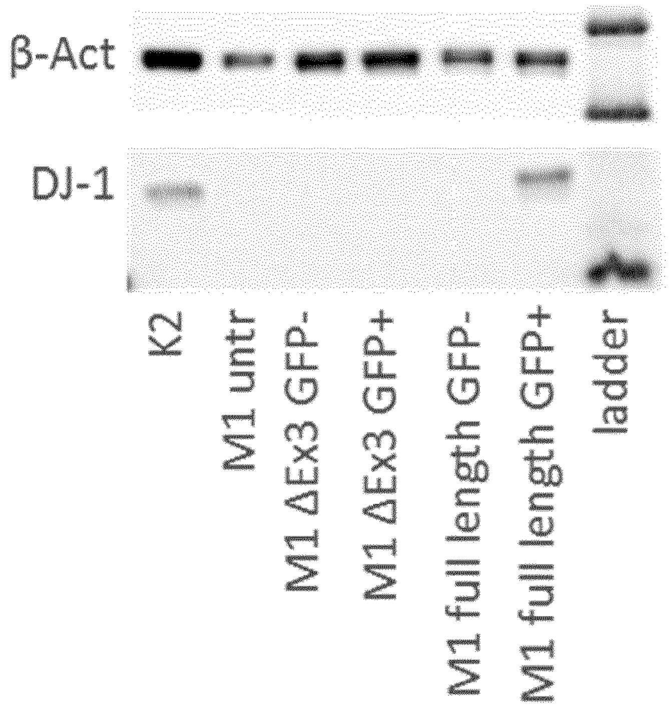
β-Act
DJ-1
K2
M1 untr
M1 ΔEx3 GFP-
M1 ΔEx3 GFP+
M1 full length GFP-
M1 full length GFP+
ladder

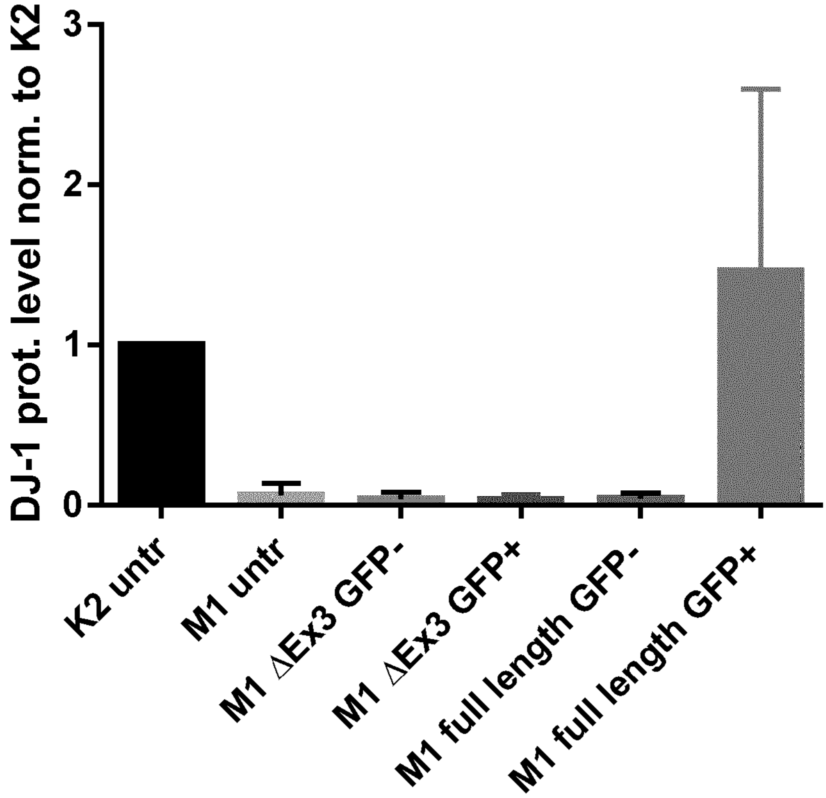
DJ-1 prot. level norm. to K2
3
2
1
0
K2 untr
M1 untr
M1 ΔEx3 GFP-
M1 ΔEx3 GFP+
M1 full length GFP-
M1 full length GFP+

Figure 17 A

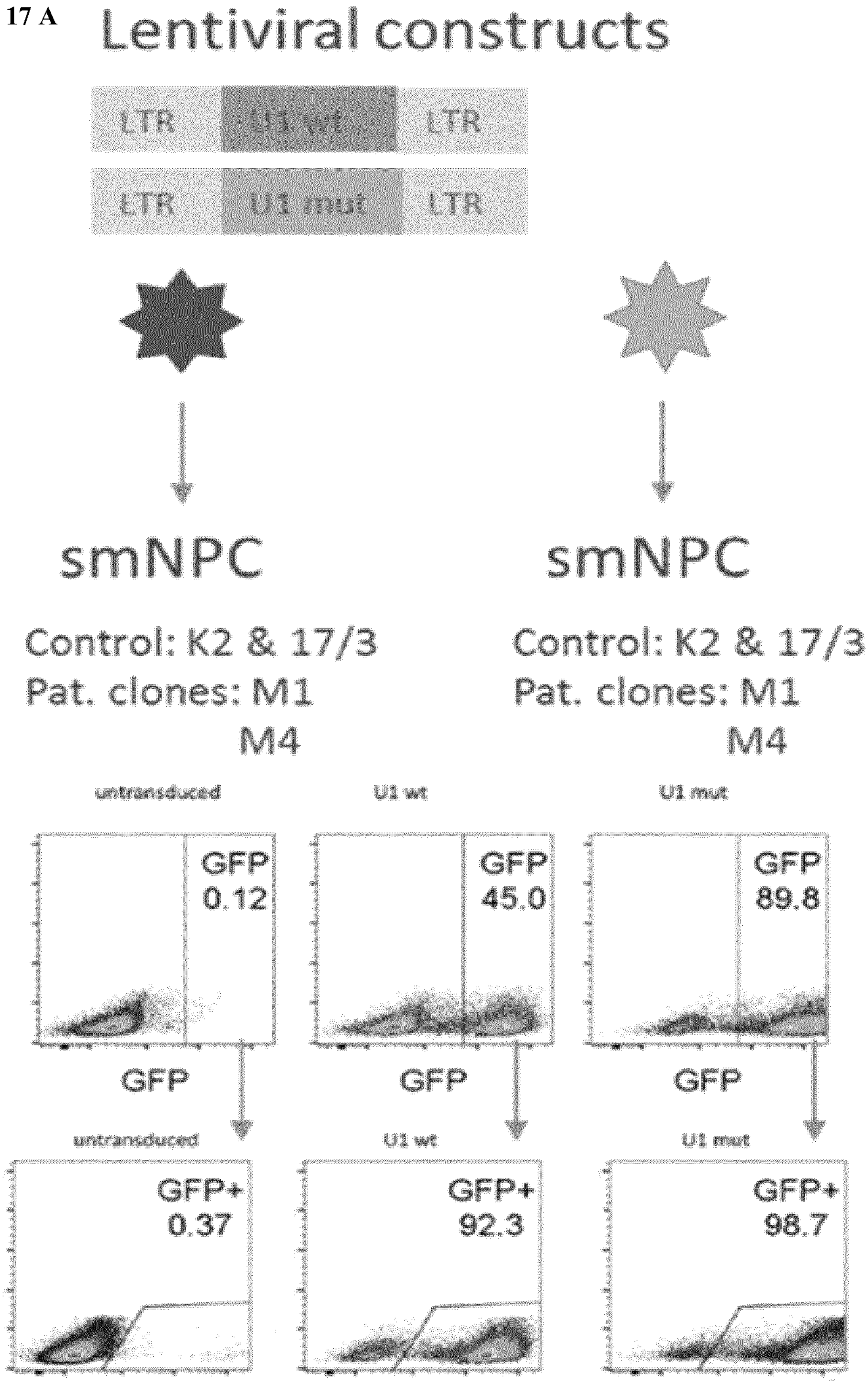
Lentiviral constructs
LTR
U1 wt
LTR
LTR
U1 mut
LTR
smNPC
smNPC
Control: K2 & 17/3
Pat. clones: M1
M4
Control: K2 & 17/3
Pat. clones: M1
M4
untransduced
U1 wt
U1 mut
GFP
0.12
GFP
45.0
GFP
89.8
GFP
GFP
GFP
untransduced
U1 wt
U1 mut
GFP+
0.37
GFP+
92.3
GFP+
98.7

**Figure 17 B + C**

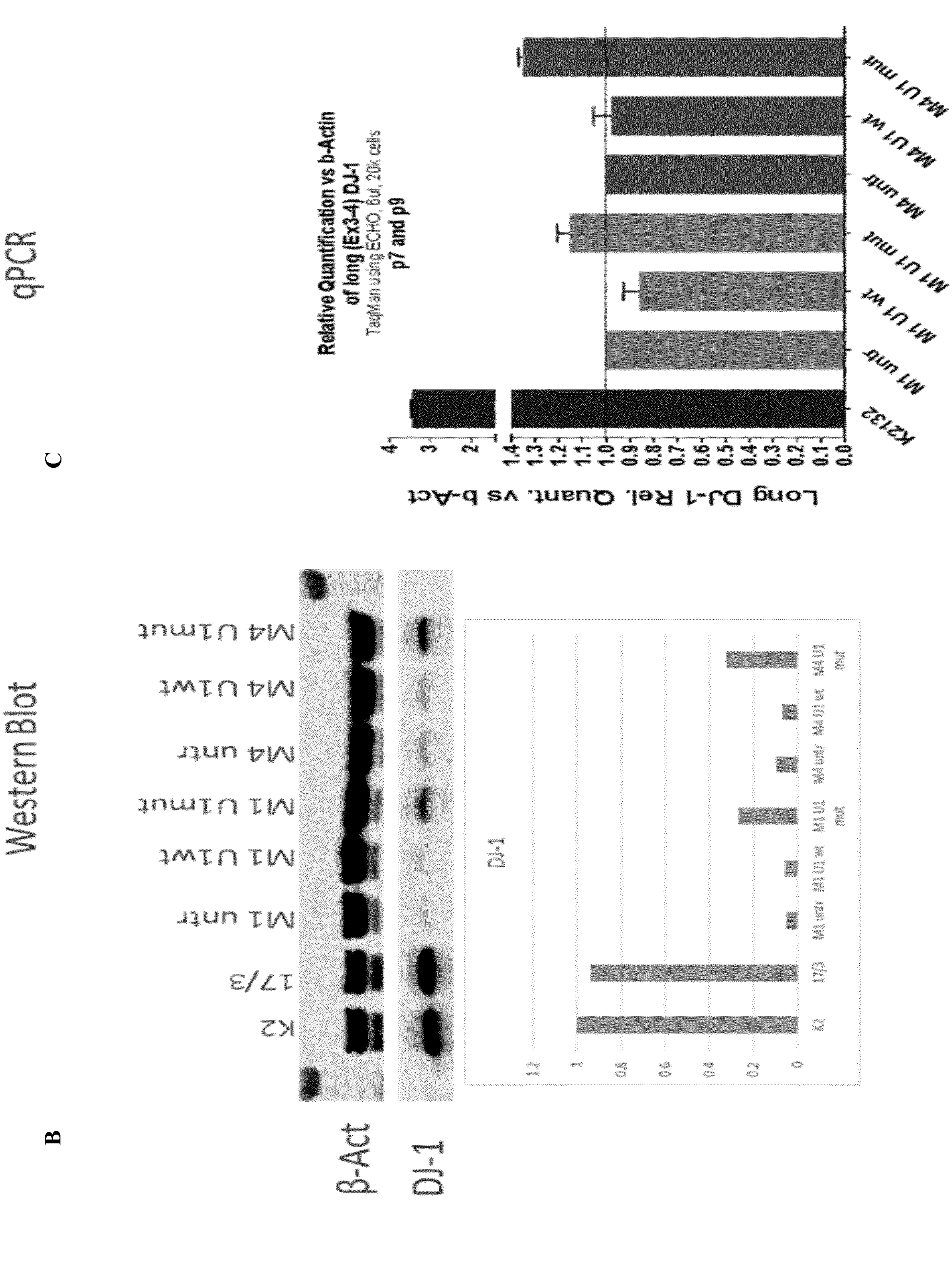
B
Western Blot
β-Act
DJ-1
K2
17/3
M1 untr
M1 U1wt
M1 U1mut
M4 untr
M4 U1wt
M4 U1mut
DJ-1
C
qPCR
Relative Quantification vs b-Actin
of long (Ex3-4) DJ-1
TaqMan using ECHO, 6ul, 20k cells
p7 and p9
Long DJ-1 Rel. Quant. vs b-Act
K2132
M1 untr
M1 U1 wt
M1 U1 mut
M4 untr
M4 U1 wt
M4 U1 mut

**Figure 18 A + B**

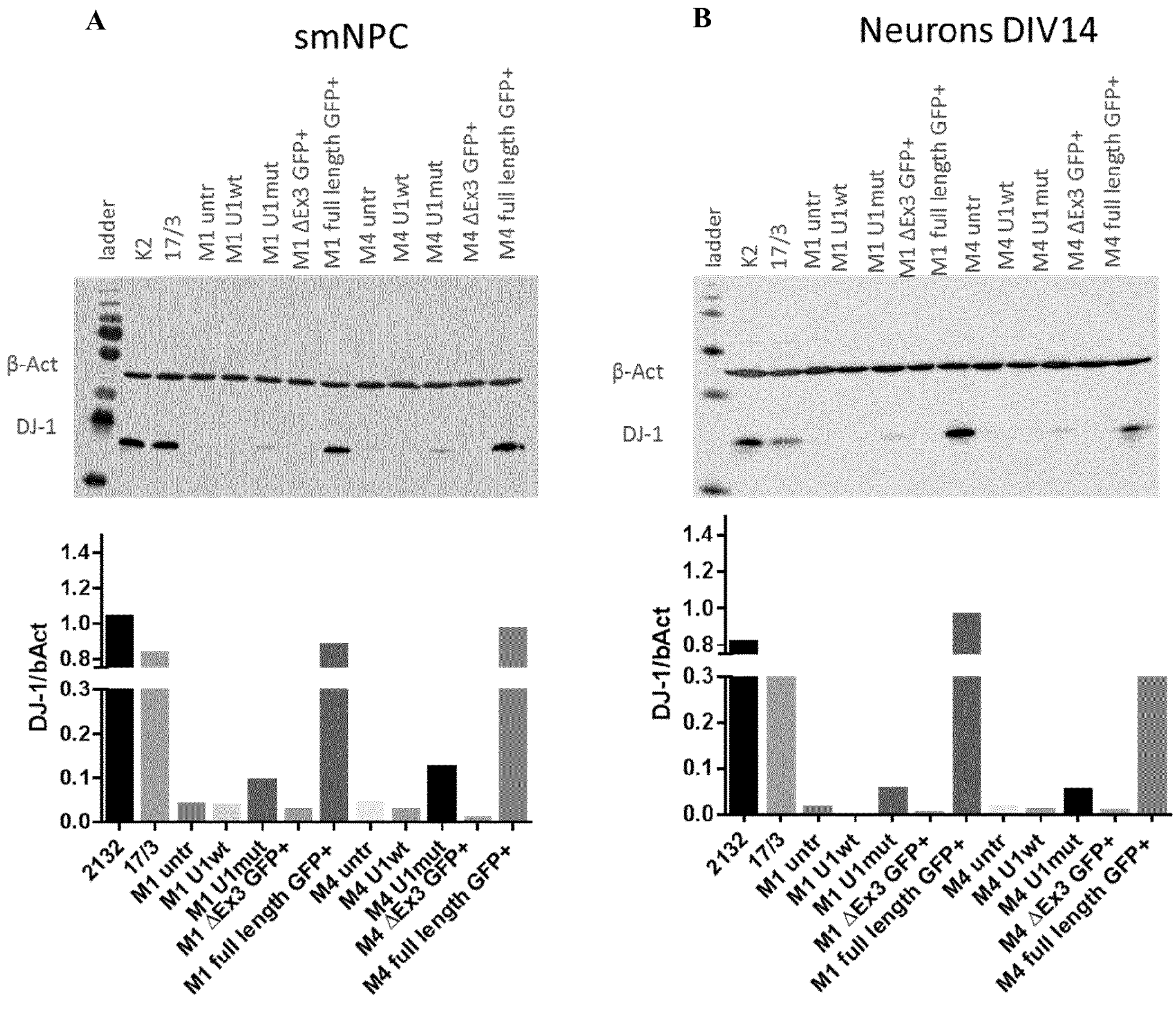
A
smNPC
ladder
K2
17/3
M1 untr
M1 U1wt
M1 U1mut
M1 ΔEx3 GFP+
M1 full length GFP+
M4 untr
M4 U1wt
M4 U1mut
M4 ΔEx3 GFP+
M4 full length GFP+
β-Act
DJ-1
DJ-1/bAct
2132
17/3
M1 untr
M1 U1wt
M1 U1mut
M1 ΔEx3 GFP+
M1 full length GFP+
M4 untr
M4 U1wt
M4 U1mut
M4 ΔEx3 GFP+
M4 full length GFP+
B
Neurons DIV14
ladder
K2
17/3
M1 untr
M1 U1wt
M1 U1mut
M1 ΔEx3 GFP+
M1 full length GFP+
M4 untr
M4 U1wt
M4 U1mut
M4 ΔEx3 GFP+
M4 full length GFP+
β-Act
DJ-1
DJ-1/bAct
2132
17/3
M1 untr
M1 U1wt
M1 U1mut
M1 ΔEx3 GFP+
M1 full length GFP+
M4 untr
M4 U1wt
M4 U1mut
M4 ΔEx3 GFP+
M4 full length GFP+

**Figure 18 C**

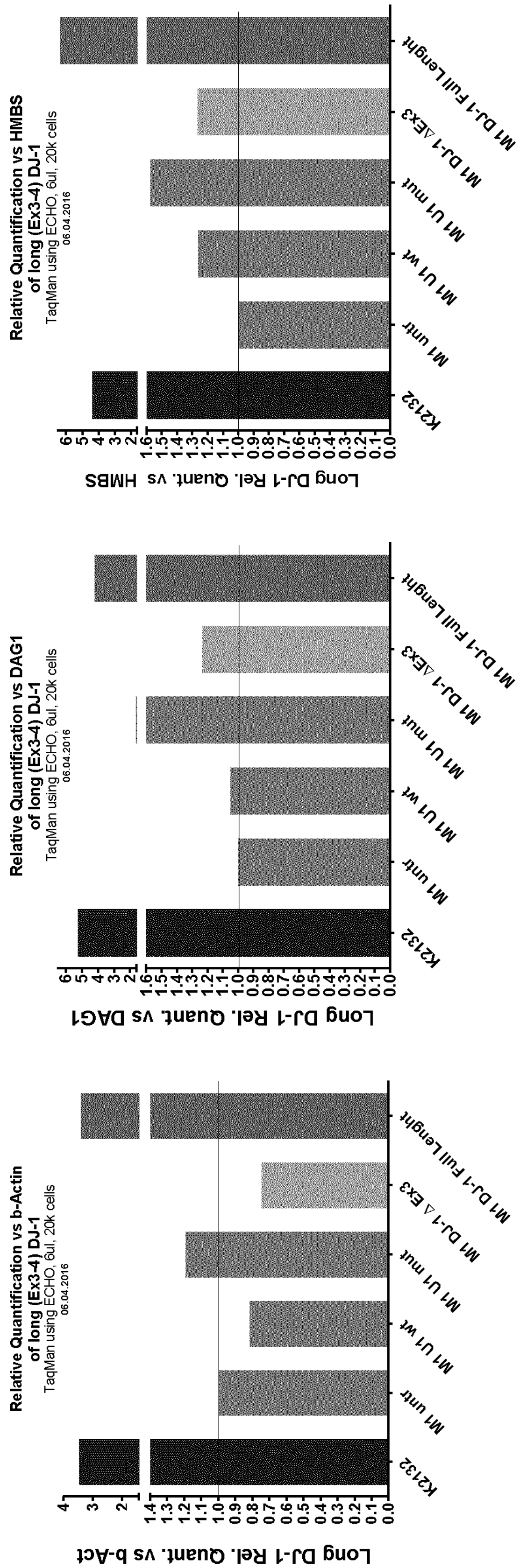
Relative Quantification vs HMBS
of long (Ex3-4) DJ-1
TaqMan using ECHO, 6ul, 20k cells
06.04.2016
Long DJ-1 Rel. Quant. vs HMBS
K2132
M1 untr
M1 U1 wt
M1 U1 mut
M1 DJ-1 △Ex3
M1 DJ-1 Full Lenght
Relative Quantification vs DAG1
of long (Ex3-4) DJ-1
TaqMan using ECHO, 6ul, 20k cells
06.04.2016
Long DJ-1 Rel. Quant. vs DAG1
Relative Quantification vs b-Actin
of long (Ex3-4) DJ-1
TaqMan using ECHO, 6ul, 20k cells
06.04.2016
Long DJ-1 Rel. Quant. vs b-Act

Figure 19

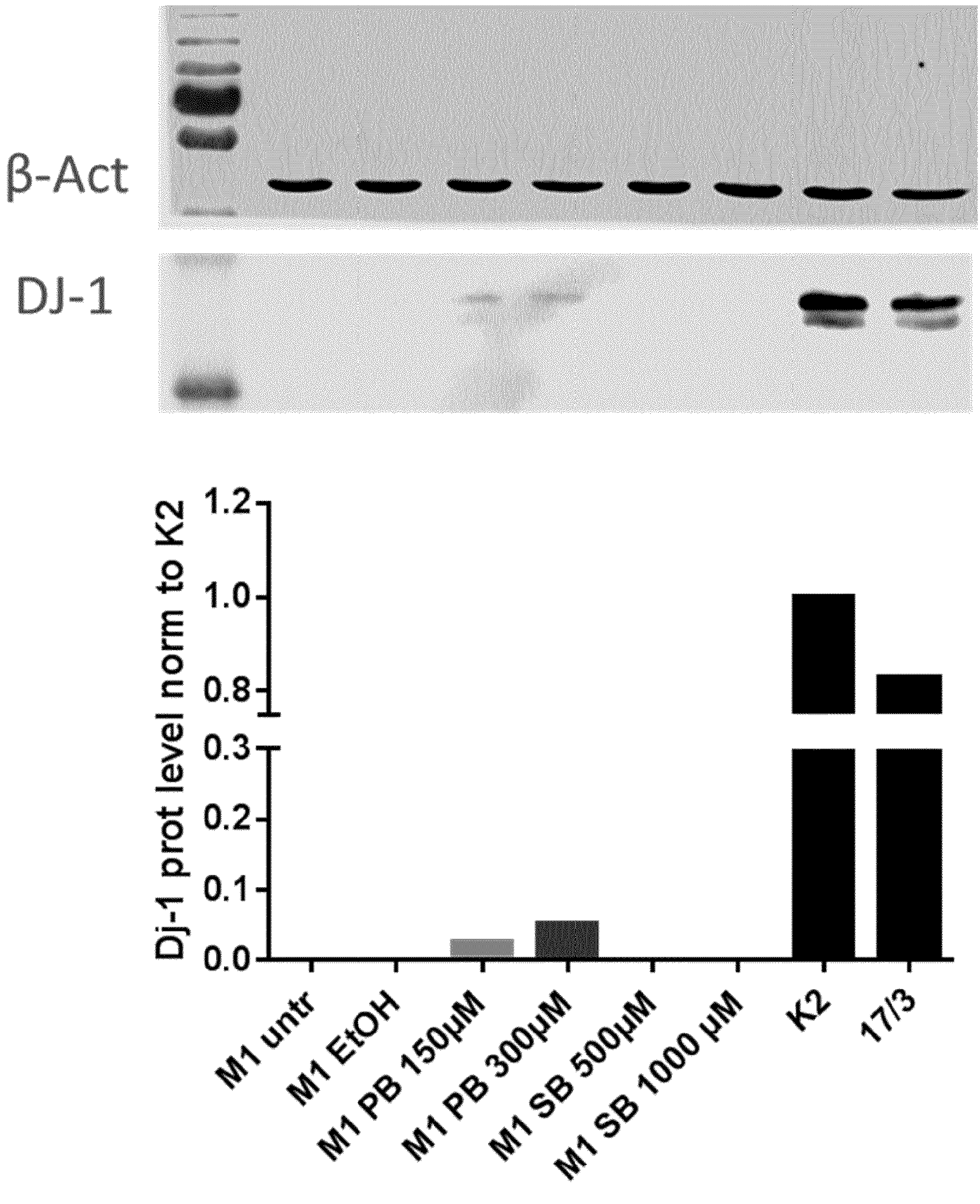
β-Act
DJ-1
Dj-1 prot level norm to K2
1.2
1.0
0.8
0.3
0.2
0.1
0.0
M1 untr
M1 EtOH
M1 PB 150µM
M1 PB 300µM
M1 SB 500µM
M1 SB 1000 µM
K2
17/3

Figure 20

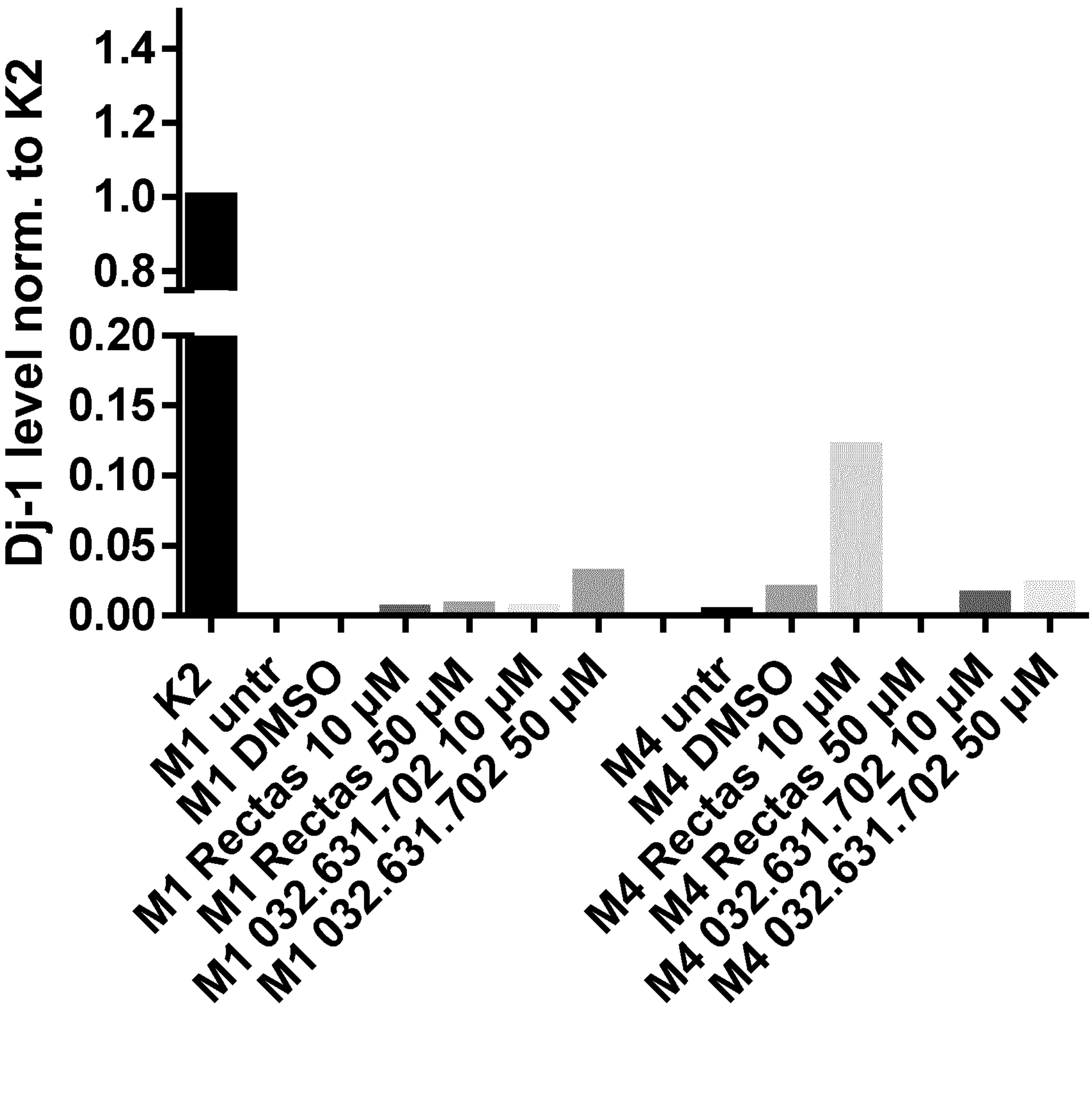
Dj-1 level norm. to K2
1.4
1.2
1.0
0.8
0.20
0.15
0.10
0.05
0.00
K2
M1 untr
M1 DMSO
M1 Rectas 10 µM
M1 Rectas 50 µM
M1 032.631.702 10 µM
M1 032.631.702 50 µM
M4 untr
M4 DMSO
M4 Rectas 10 µM
M4 Rectas 50 µM
M4 032.631.702 10 µM
M4 032.631.702 50 µM

**Figure 21**

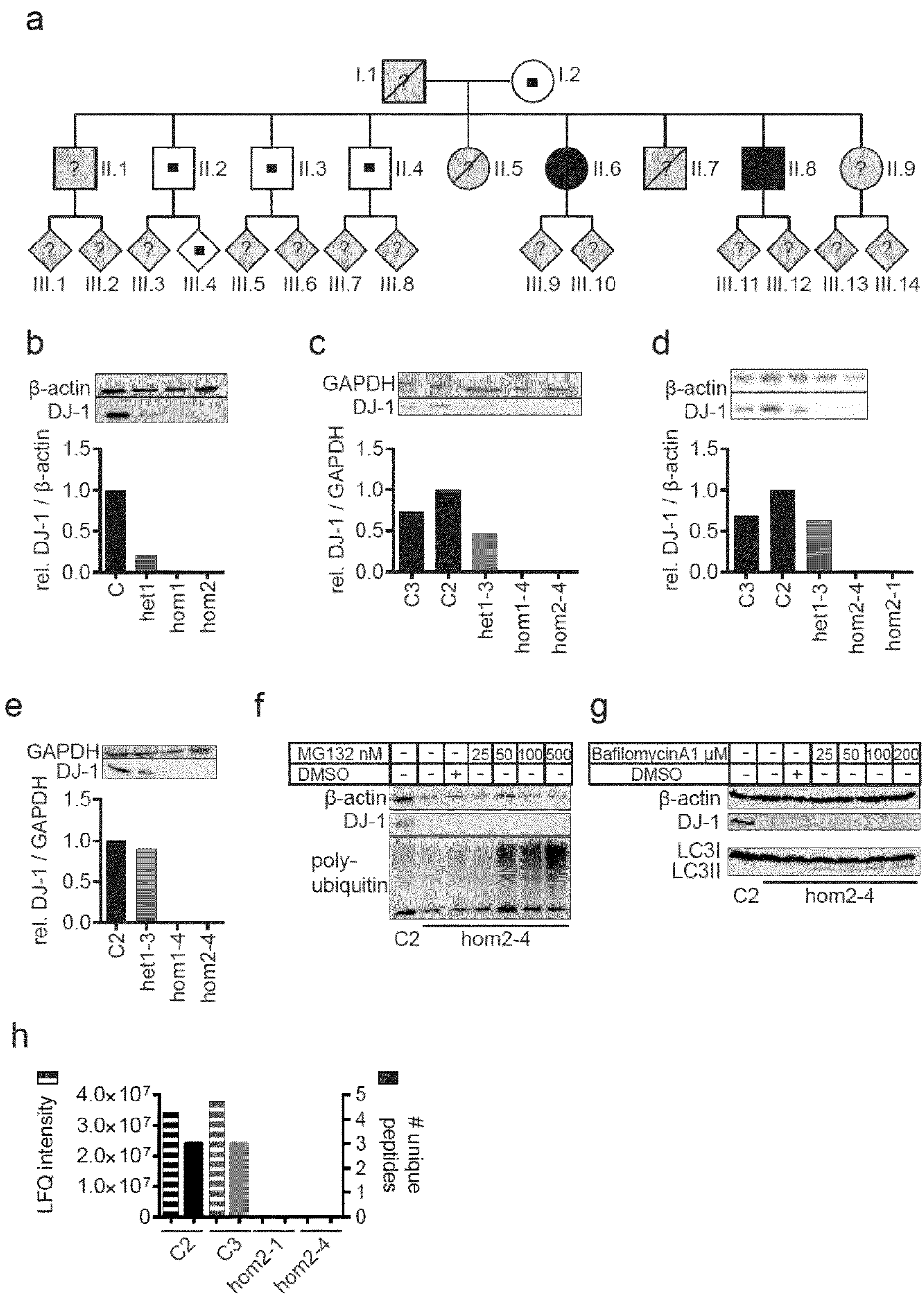

a
I.1
I.2
II.1
II.2
II.3
II.4
II.5
II.6
II.7
II.8
II.9
III.1 III.2 III.3 III.4 III.5 III.6 III.7 III.8 III.9 III.10 III.11 III.12 III.13 III.14
b
β-actin
DJ-1
rel. DJ-1 / β-actin
C het1 hom1 hom2
c
GAPDH
DJ-1
rel. DJ-1 / GAPDH
C3 C2 het1-3 hom1-4 hom2-4
d
β-actin
DJ-1
rel. DJ-1 / β-actin
C3 C2 het1-3 hom2-4 hom2-1
e
GAPDH
DJ-1
rel. DJ-1 / GAPDH
C2 het1-3 hom1-4 hom2-4
f
MG132 nM - - - 25 50 100 500
DMSO - - + - - - -
β-actin
DJ-1
poly-ubiquitin
C2 hom2-4
g
BafilomycinA1 μM - - - 25 50 100 200
DMSO - - + - - - -
β-actin
DJ-1
LC3I
LC3II
C2 hom2-4
h
LFQ intensity
# unique peptides
4.0x10⁷
3.0x10⁷
2.0x10⁷
1.0x10⁷
C2 C3 hom2-1 hom2-4

**Figure 22**

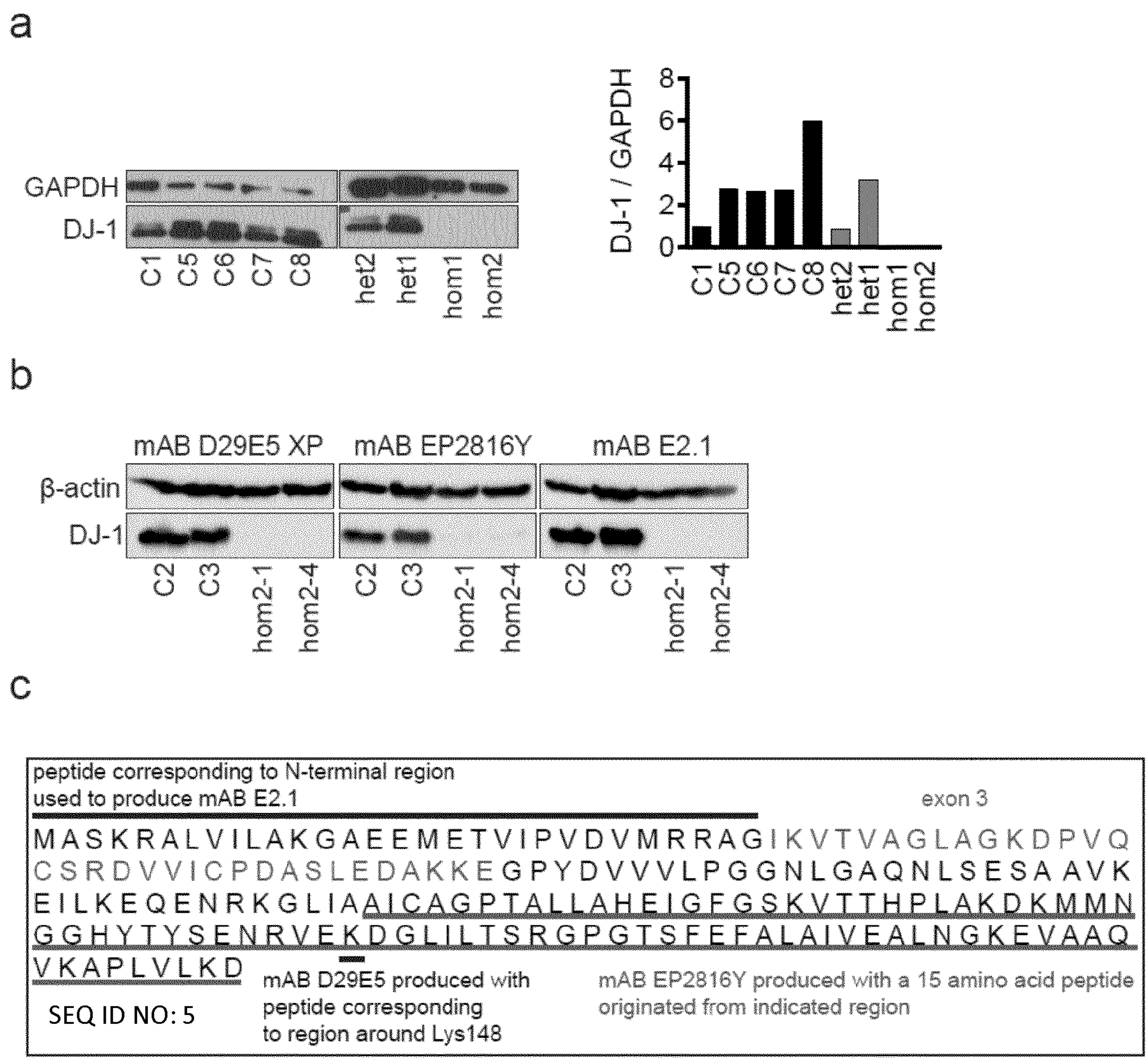
a
GAPDH
DJ-1
C1 C5 C6 C7 C8 het2 het1 hom1 hom2
DJ-1 / GAPDH
0 2 4 6 8
C1 C5 C6 C7 C8 het2 het1 hom1 hom2
b
mAB D29E5 XP
mAB EP2816Y
mAB E2.1
β-actin
DJ-1
C2 C3 hom2-1 hom2-4 C2 C3 hom2-1 hom2-4 C2 C3 hom2-1 hom2-4
c
peptide corresponding to N-terminal region
used to produce mAB E2.1
exon 3
MASKRALVILAKGAEEMETVIPVDVMRRAGIKVTVAGLAGKDPVQ
CSRDVVICPDASLEDAKKEGPYDVVVLPGGNLGAQNLSESAAVK
EILKEQENRKGLIAAICAGPTALLAHEIGFGSKVTTHPLAKDKMMN
GGHYTYSENRVEKDGLILTSRGPGTSFEFALAIVEALNGKEVAAQ
VKAPLVLKD
SEQ ID NO: 5
mAB D29E5 produced with
peptide corresponding
to region around Lys148
mAB EP2816Y produced with a 15 amino acid peptide
originated from indicated region

Figure 23

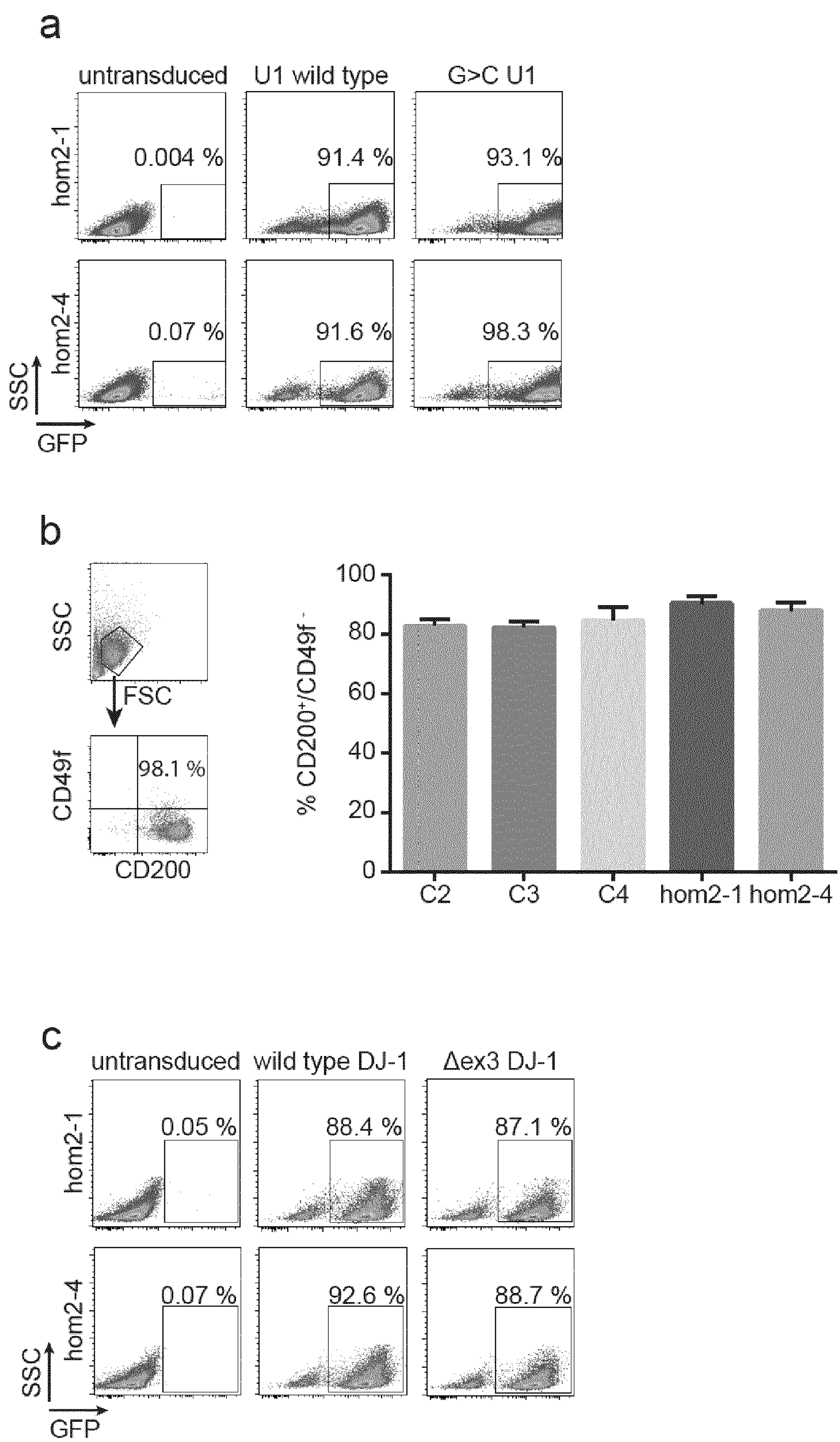
a
untransduced
U1 wild type
G>C U1
hom2-1
0.004 %
91.4 %
93.1 %
hom2-4
0.07 %
91.6 %
98.3 %
SSC
GFP
b
SSC
FSC
CD49f
98.1 %
CD200
% CD200+/CD49f -
100
80
60
40
20
0
C2
C3
C4
hom2-1
hom2-4
c
untransduced
wild type DJ-1
Δex3 DJ-1
hom2-1
0.05 %
88.4 %
87.1 %
hom2-4
0.07 %
92.6 %
88.7 %
SSC
GFP

Figure 24

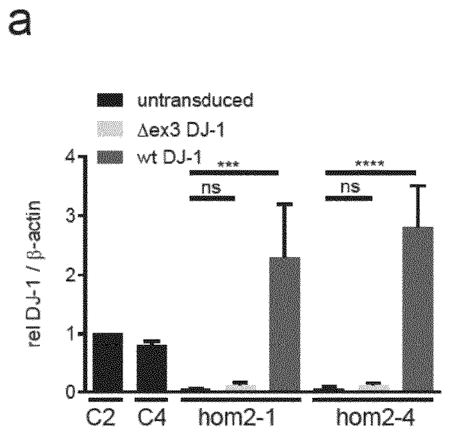


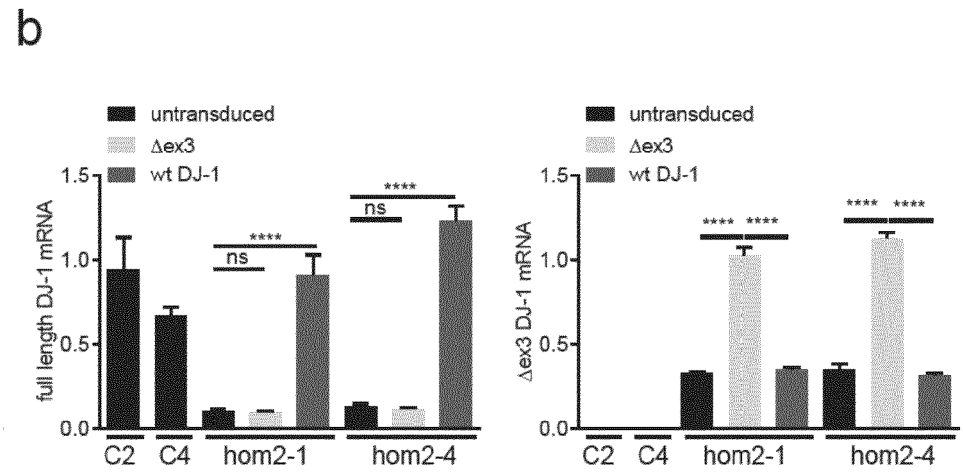


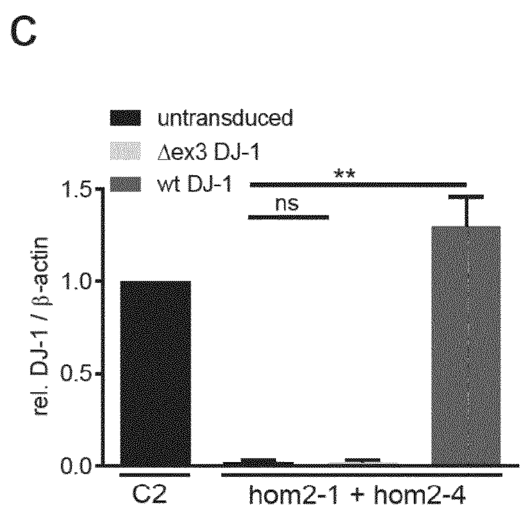


Figure 24 (continued)

SEQ ID NO: 12

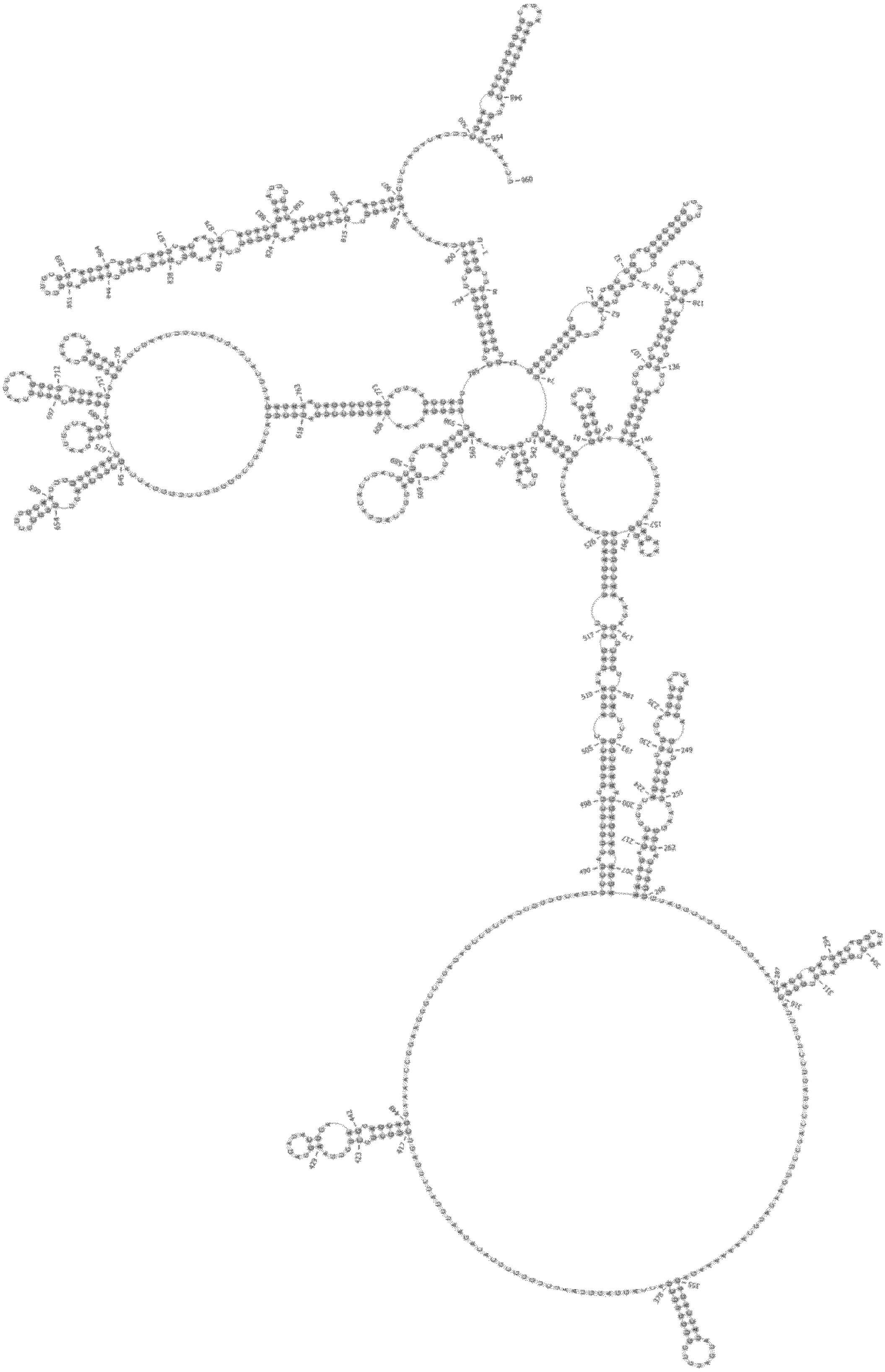

d

**Figure 24 (continued)**

SEQ ID NO: 13

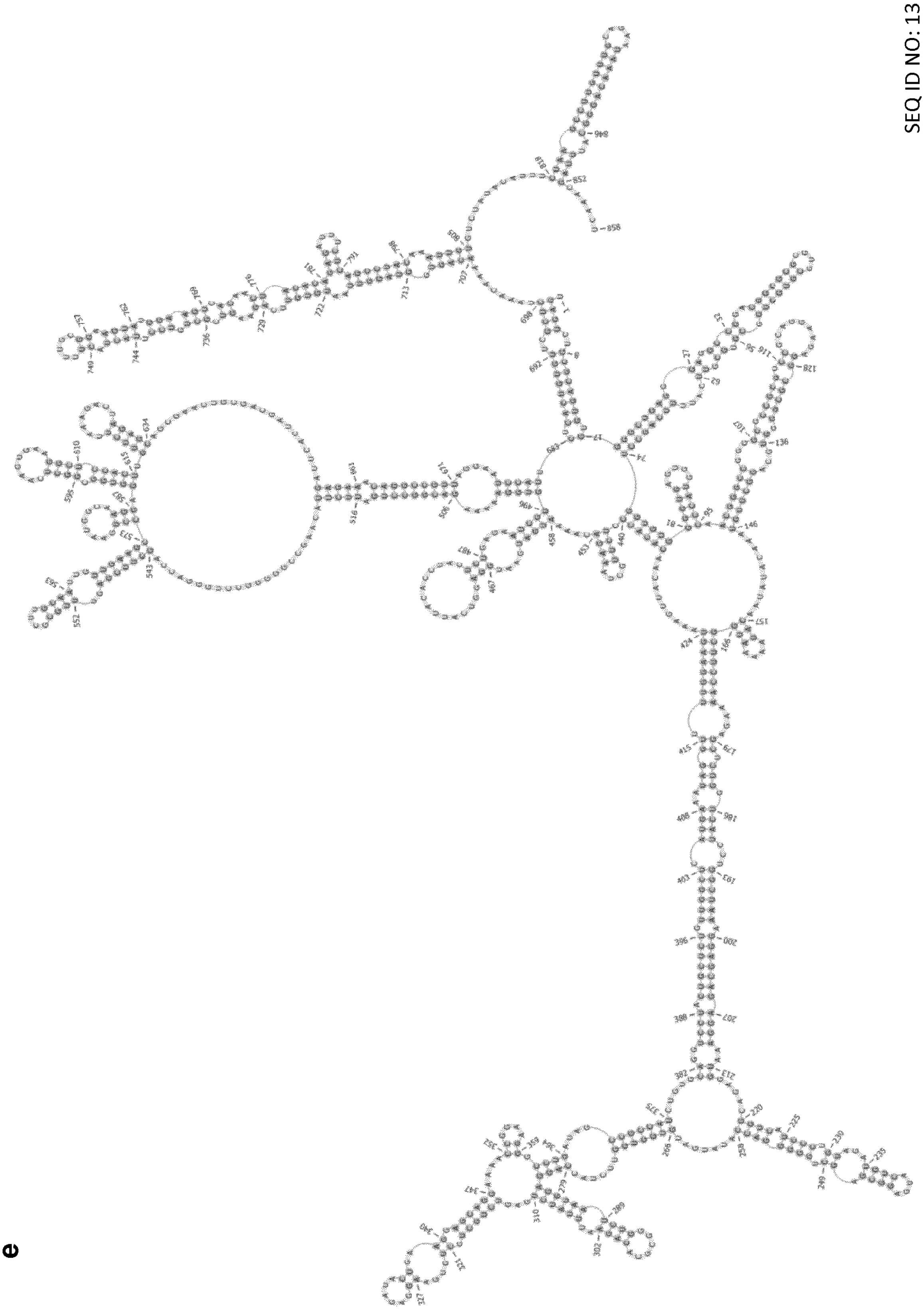

e

**Figure 24 (continued)**

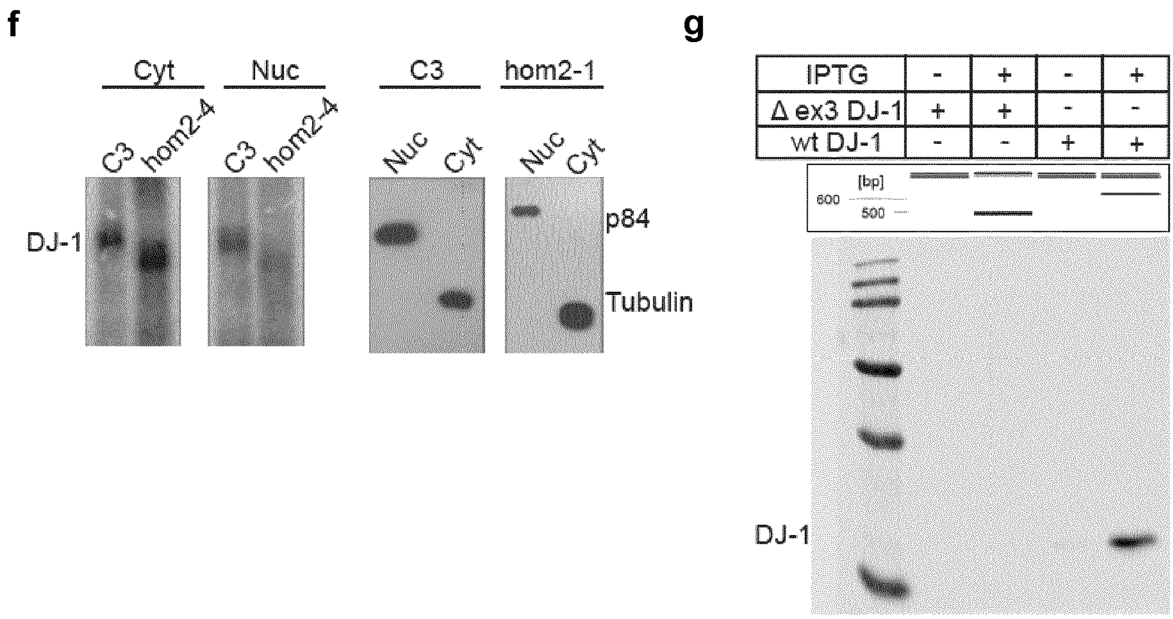


**Figure 25**

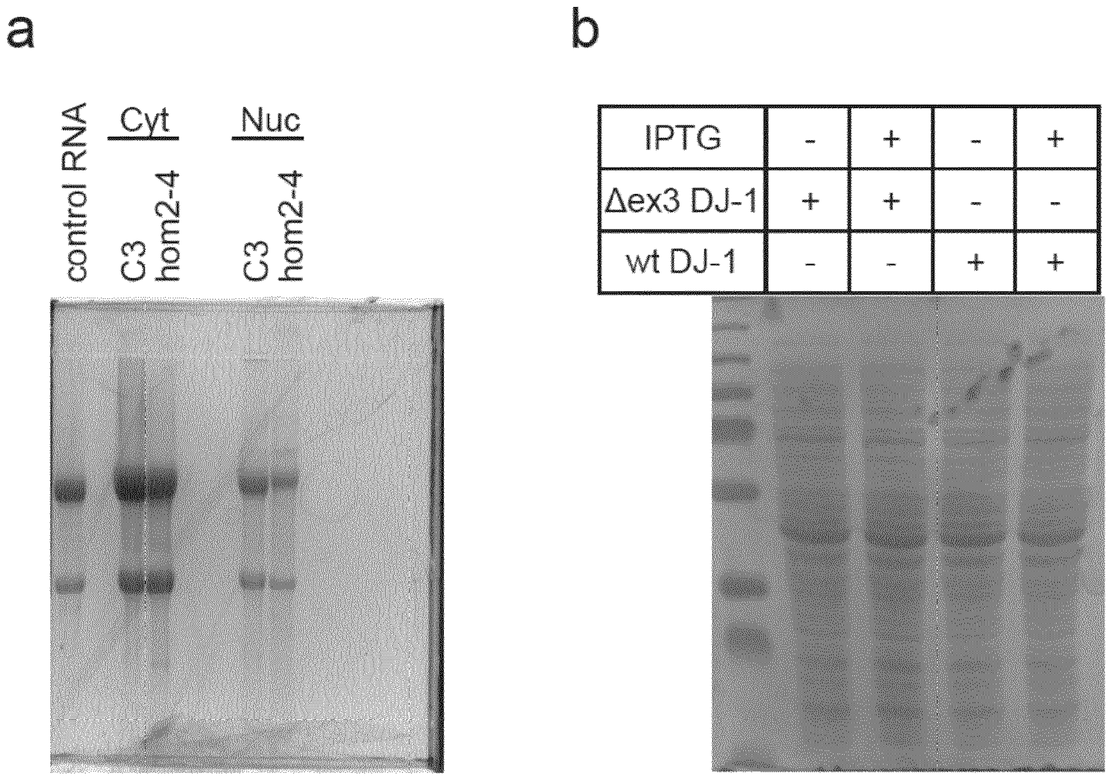

**Figure 26**

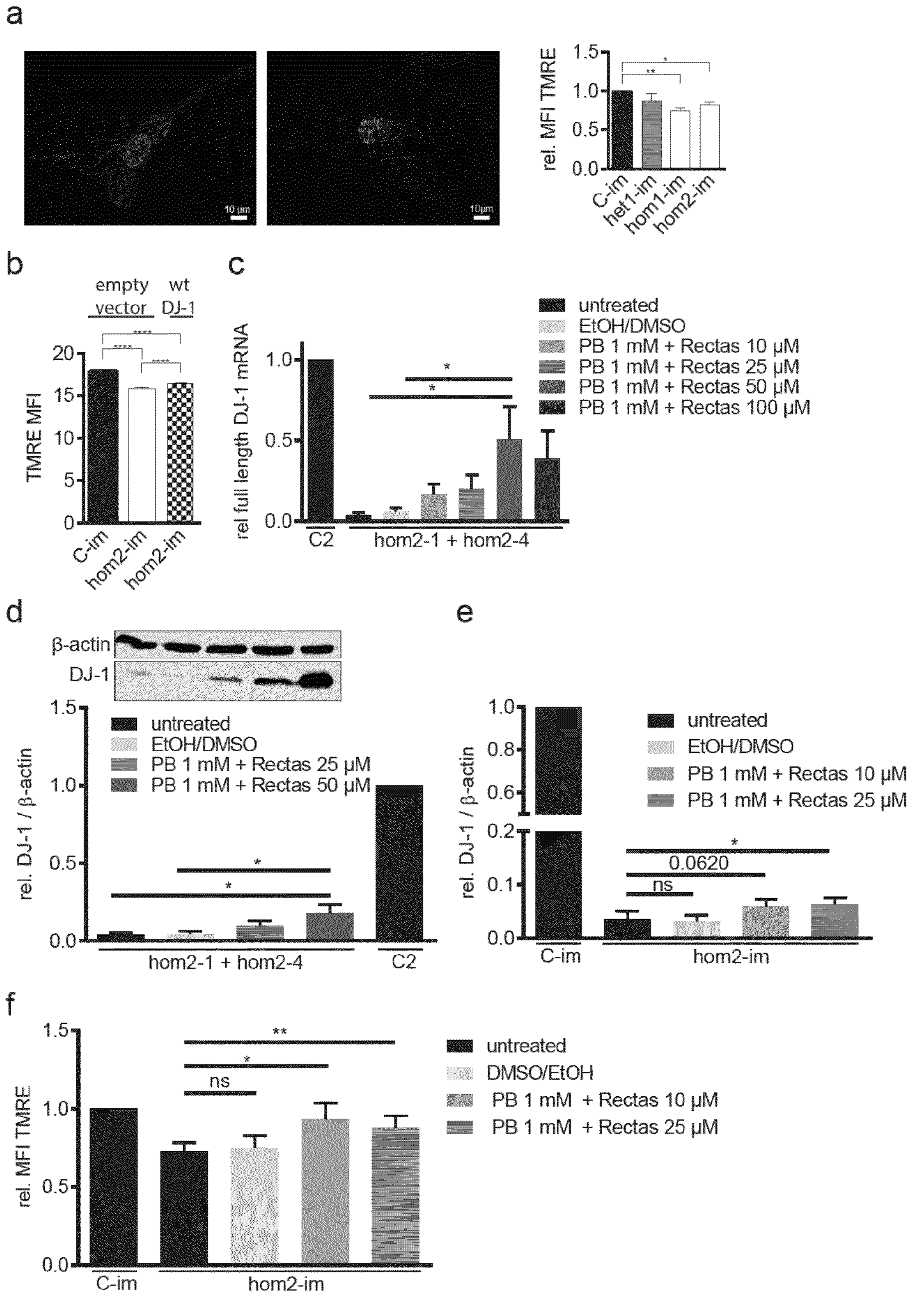
a
10 µm
10µm
rel. MFI TMRE
1.5
1.0
0.5
0.0
*
**
C-im
het1-im
hom1-im
hom2-im
b
empty vector
wt DJ-1
****
****
****
TMRE MFI
20
15
10
5
0
C-im
hom2-im
hom2-im
c
untreated
EtOH/DMSO
PB 1 mM + Rectas 10 µM
PB 1 mM + Rectas 25 µM
PB 1 mM + Rectas 50 µM
PB 1 mM + Rectas 100 µM
rel full length DJ-1 mRNA
1.0
0.5
0.0
*
*
C2
hom2-1 + hom2-4
d
β-actin
DJ-1
1.5
1.0
0.5
0.0
rel. DJ-1 / β-actin
untreated
EtOH/DMSO
PB 1 mM + Rectas 25 µM
PB 1 mM + Rectas 50 µM
*
*
hom2-1 + hom2-4
C2
e
1.0
0.8
0.6
0.2
0.1
0.0
rel. DJ-1 / β-actin
untreated
EtOH/DMSO
PB 1 mM + Rectas 10 µM
PB 1 mM + Rectas 25 µM
*
0.0620
ns
C-im
hom2-im
f
1.5
1.0
0.5
0.0
rel. MFI TMRE
**
*
ns
untreated
DMSO/EtOH
PB 1 mM + Rectas 10 µM
PB 1 mM + Rectas 25 µM
C-im
hom2-im

Figure 27

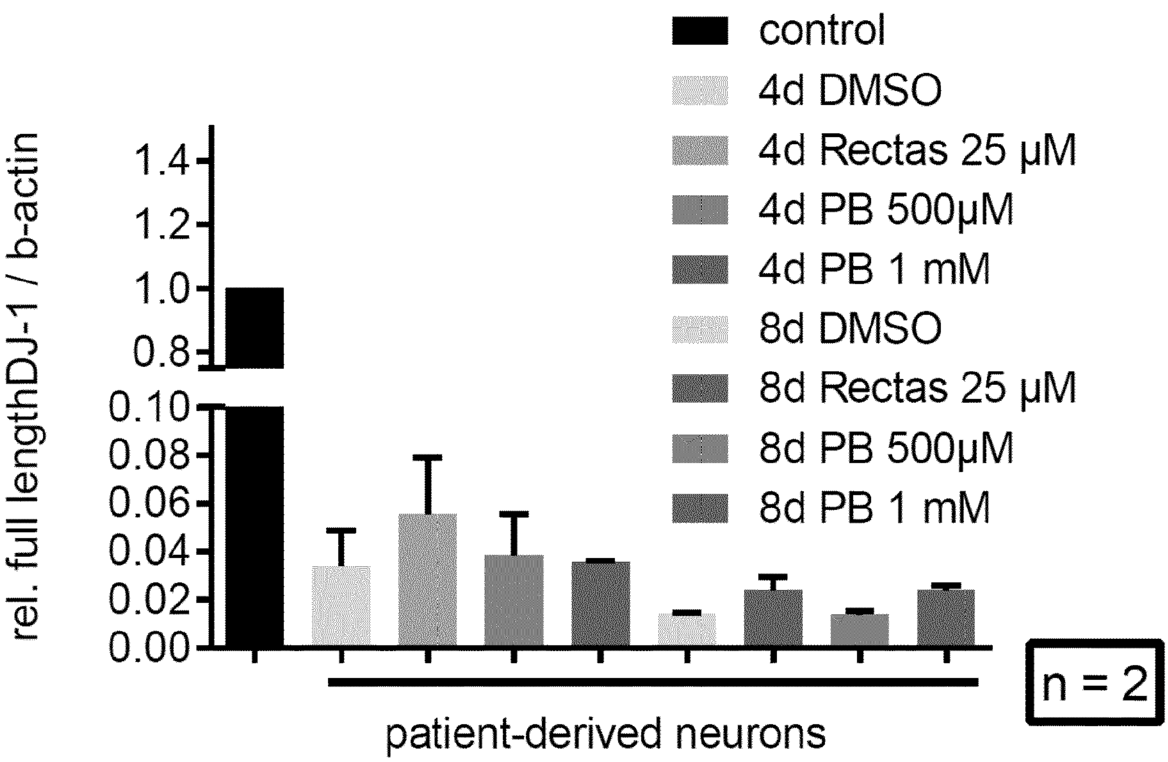
control
4d DMSO
4d Rectas 25 µM
4d PB 500µM
4d PB 1 mM
8d DMSO
8d Rectas 25 µM
8d PB 500µM
8d PB 1 mM
rel. full lengthDJ-1 / b-actin
1.4
1.2
1.0
0.8
0.10
0.08
0.06
0.04
0.02
0.00
patient-derived neurons
n = 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HERING et al.** *Human Mutation,* 2004, vol. 24, 321-329
- **MALGIERI ; ELIEZER.** *Protein Science,* 2007, vol. 17, 855-868
- **ONO et al.** *Parkinsonism and Related Disorders,* 2009, vol. 15, 649-654
- **HERTZ et al.** *Cell,* 2013, vol. 154, 737-747
- **COGNATA et al.** *Neurogenetics,* 2015, vol. 16, 245-263
- Remington's Pharmaceutical Sciences. Maack Publishing Co, 2012
- **BABENDURE, J. R.** Control of mammalian translation by mRNA structure near caps. *RNA,* 2006, vol. 12, 851-861
- **DOMA, M. K. ; PARKER, R.** Endonucleolytic cleavage of eukaryotic mRNAs with stalls in translation elongation. *Nature,* 2006, vol. 440, 561-564
- **AXELROD, F. B. et al.** Kinetin improves IKBKAP mRNA splicing in patients with familial dysautonomia. *Pediatr. Res.,* 2011, vol. 70, 480-3
- **SHETTY, R. S. et al.** Specific correction of a splice defect in brain by nutritional supplementation. *Hum. Mol. Genet.,* 2011, vol. 20, 4093-101
- **SAMARANCH, L. et al.** PINK1-linked parkinsonism is associated with Lewy body pathology. *Brain,* 2010, vol. 133, 1128-42
- **NISSIM-RAFINIA, M. ; KEREM, B.** Splicing regulation as a potential genetic modifier. *Trends Genet,* 2002, vol. 18, 123-7
- Parkinson Progression Marker Initiative. The Parkinson Progression Marker Initiative (PPMI). *Prog. Neurobiol.,* 2011, vol. 95, 629-35
- **SOEMEDI, R. et al.** Pathogenic variants that alter protein code often disrupt splicing. *Nat. Genet.,* 2017
- **TAN, A. ; ABECASIS, G. R. ; KANG, H. M.** Unified representation of genetic variants. *Bioinformatics,* 2015, vol. 31, 2202-2204
- **LI, H. et al.** The Sequence Alignment/Map format and SAMtools. *Bioinformatics,* 2009, vol. 25, 2078-2079
- **WANG, K. ; LI, M. ; HAKONARSON, H.** ANNOVAR: functional annotation of genetic variants from high-throughput sequencing data. *Nucleic Acids Res.,* 2010, vol. 38, e164-e164
- **LIU, X. ; WU, C. ; LI, C. ; BOERWINKLE, E.** dbNSFP v3.0: A One-Stop Database of Functional Predictions and Annotations for Human Nonsynonymous and Splice-Site SNVs. *Hum. Mutat.,* 2016, vol. 37, 235-241
- The 1000 Genomes Project Consortium. A global reference for human genetic variation. *Nature,* 2015, vol. 526, 68-74
- **YEO, G ; BURGE, C. B.** Maximum entropy modeling of short sequence motifs with applications to RNA splicing signals. *J. Comput. Biol. J. Comput. Mol. Cell Biol.,* 2004, vol. 11, 377-394
- **JIAN, X. ; BOERWINKLE, E. ; LIU, X.** In silico prediction of splice-altering single nucleotide variants in the human genome. *Nucleic Acids Res.,* 2014, vol. 42, 13534-13544
- **LIU, X. ; WU, C. ; LI, C ; BOERWINKLE, E.** dbNSFP v3.0: A One-Stop Database of Functional Predictions and Annotations for Human Nonsynonymous and Splice-Site SNVs. *Hum. Mutat.,* 2016, vol. 37, 235-241
- **SHIRLEY, M. D. ; MA, Z. ; PEDERSEN, B. S. ; WHEELAN, S. J.** Efficient 'pythonic' access to FASTA files using pyfaidx. *PeerJ PrePrints,* 2015
- **ZHAN, X. ; HU, Y. ; LI, B. ; ABECASIS, G. R. ; LIU, D. J.** RVTESTS: an efficient and comprehensive tool for rare variant association analysis using sequence data. *Bioinformatics,* 2016, vol. 32, 1423-1426